# EUROPEAN PATENT APPLICATION

(11) **EP 4 741 412 A1**
(43) Date of publication of application: **13.05.2026**
(21) Application number: 24836064.6
(22) Date of filing: 03.07.2024
(51) Int. Cl.: C07K 7/06, G01N 27/62, G01N 33/574

(54) **GLYCOPEPTIDE, METHOD FOR ANALYZING GLYCOPEPTIDE, AND METHOD FOR ANALYZING CANCER**

(30) Priority: 05.07.2023 JP 2023110408
(71) Applicant: ENU Pharma, Inc., Sapporo-shi Hokkaido 001-0021 (JP)
(72) Inventor: NISHIMURA, Shin-Ichiro, Sapporo-shi, Hokkaido 060-0808 (JP); YOKOI, Yasuhiro, Sapporo-shi, Hokkaido 060-0808 (JP)
(74) Representative: Godemeyer Blum Lenze Patentanwälte Partnerschaft mbB - werkpatent
(86) International application number: PCT/JP2024/024051
(87) International publication number: WO 2025/009551

(57) **Abstract**

It is an object of the present invention to provide a material for systematically searching for and identifying epitopes specific to various diseases including cancer. In addition, it is another object of the present invention to establish a method for analyzing diseases using such epitopes specific to various diseases including cancer. The present invention relates to a glycopeptide, in which one glycan consisting of 6 or less sugars binds to each of one or two or more threonines in a peptide consisting of the amino acid sequence TTPPTTATPIR, and the glycan is an O-linked glycan. Moreover, the present invention relates to a method for analyzing a glycopeptide, comprising analyzing whether or not an epitope glycopeptide is contained in a sample containing a glycopeptide obtained by protease digestion of the serum or the plasma, wherein the glycopeptide is a peptide consisting of the amino acid sequence TTPPTTATPIR, in which one glycan consisting of 6 or less sugars binds to each of one or two or more threonines, and the glycan is an O-linked glycan.

## Description

### Technical Field

The present invention relates to a glycopeptide, a method for analyzing a glycopeptide, and a method for analyzing cancer.

### Background Art

Fibronectin is a glycoprotein essential for cancer proliferation and growth, invasion, and further, formation of the cancer microenvironment, and such fibronectin has been known to be the most important extracellular matrix component. In addition, fibronectin has also been widely known as one of the most important ligand molecules for various integrin heterodimers that are highly expressed on the surface of many cancer cells (e.g., Non-Patent Document 1). For this reason, cancer therapeutic drugs are being developed based on the molecular mechanism of inhibiting disease-specific binding between fibronectin (a glycoprotein molecule), and integrin that is highly expressed on the surface of cancer cells (e.g., Non-Patent Document 2).

It has been known that, in fibronectin in cancer patients and fetuses, three domains, namely, IIICS (type III homology connective segment domain), ED-A (extra domain A) and ED-B (extra domain B), which are not found in adult fibronectin (i.e., the expression levels thereof are low in adults) are generated by alternative splicing (e.g., Non-Patent Document 3). Research and development are actively underway regarding the discovery of drugs targeting fibronectin isomers containing such domains consisting of regions significantly expressed in cancer, in particular, drug delivery systems (DDS) for cancer therapy (e.g., Non-Patent Document 4). Moreover, in Non-Patent Documents 5 and 6, cancer diagnostic drugs using antibodies targeting each of ED-A and ED-B as biomarkers (targets) are studied. At present, clinical trials of antibody conjugates, to which cytokines such as IL-2 have bound, are continuously ongoing, but no practical applications have been achieved yet.

Non-Patent Document 7 discloses that the mouse monoclonal antibody FDC-6, which was established by isolation and immunization of fibronectin generated from human hepatocellular carcinoma cells (HUH-7), reacts with fibronectin derived from cancer patients and fetuses, but does not react with fibronectin from adults. Such fibronectin is defined as oncofetal fibronectin (onfFN). FDC-6 is a mouse antibody that binds to the glycopeptide epitopes ²¹¹⁴Val-Thr (GalNAcα)-His-Pro-Gly-Tyr²¹¹⁹ and ²¹¹⁴Val-Thr (Galβ1,3GalNAcα)-His-Pro-Gly-Tyr²¹¹⁹ in the IIICS domain of onfFN (Non-Patent Documents 8 and 9). It has been reported that OnfFNs containing these glycosylation regions are deeply involved in the TGF-β-induced "epithelial-mesenchymal transition (EMT)" process in cancer progression and metastasis (Non-Patent Document 10). However, the significance and specific function of these glycosylation regions in cancer biology, as well as the potential for developing novel cancer diagnostic and therapeutic methods based on FDC-6 or this epitope, still remain unknown.

### Prior Art Documents

### Non-Patent Documents

Non-Patent Document 1: R. O. Hynes, Fibronectin, Springer, New York, 1990
Non-Patent Document 2: H. Kessler, et al., A comprehensive evaluation of the activity and selectivity profile of ligands for RGD-binding integrins. Sci. Rep. 2017, 7, 39805
Non-Patent Document 3: J. E. Schwarzbauer, Alternative splicing of fibronectin: Three variants, three functions. BioEssays 1991, 13, 527-533
Non-Patent Document 4: D. P. Reinhardt, et al., Fibronectin-targeted drug delivery in cancer. Adv. Drug Deliv. Rev. 2016, 97, 101-110
Non-Patent Document 5: D. Neri, et al., The extra-domain A of fibronectin is a vascular marker of solid tumors and metastasis. Cancer Res. 2007, 67, 10948-10957
Non-Patent Document 6: L. J. Dubois, et al., Human fibronectin extra domain B as a biomarker for targeted therapy in cancer. Mol. Oncol. 2020, 14, 1555-1568
Non-Patent Document 7: Hakomori, S.-i. et al., The oncofetal domain of fibronectin defined by monoclonal antibody FDC-6: Its presence in fibronectins from fetal and tumor tissues and its absence in those from normal adult tissues and plasma. Pro. Natl. Acad. Sci. USA 1985, 82, 6517-6521
Non-Patent Document 8: Hakomori, S.-i. et al., The oncofetal structure of human fibronectin defined by monoclonal antibody FDC-6: Unique structural requirement for the antigenic specificity proved by a glycosylhexapeptide. J. Biol. Chem 1988, 263, 3314-3322
Non-Patent Document 9: Hakomori, S.-i. et al., An alpha N-acetylgalactosaminylation at the threonine residue of a defined peptide sequence creates the oncofetal peptide epitope in human fibronectin. ibid, 1989, 264, 10472-10476
Non-Patent Document 10: Hakomori, S.-i. et al., Involvement of O-glycosylation defining oncofetal fibronectin in epithelial-mesenchymal-transition process. Pro. Natl. Acad. Sci. USA 2011, 108, 17690-17695

### Summary of Invention

### Objects to be Solved by the Invention

As mentioned above, various studies having, as a target for cancer diagnostic and therapeutic drugs, fibronectin, a multifunctional, high-molecular-weight glycoprotein that is the most important as an extracellular matrix, have been actively conducted for over 40 years. However, neither pharmaceutical products nor diagnostic technologies targeting this molecule have been practically used to date.

Thus, in order to solve the problems of the prior art techniques, the present inventors have conducted extensive studies on fibronectin regarding epitopes specific to various diseases including cancer (cancer-specific structural changes caused by post-translational glycosylation: dynamic epitopes).

It is an object of the present invention to provide a material for systematically searching for and identifying epitopes specific to various diseases including cancer. In addition, it is another object of the present invention to identify dynamic epitopes specific to various diseases including cancer, and to establish a method for analyzing diseases using these dynamic epitopes.

### Means for Solving the Objects

Examples of specific aspects of the present invention will be described below.
[1] A glycopeptide, in which one glycan consisting of 6 or less sugars binds to each of one or two or more threonines in a peptide consisting of the amino acid sequence TTPPTTATPIR, and the glycan is an O-linked glycan.
[2] The glycopeptide according to [1], wherein the glycan is selected from the group consisting of the following (1) to (4):
   (1) GalNAcα1,
   (2) Galβ1,3GalNAcα1,
   (3) Neu5Acα2,6GalNAcα1, and
   (4) Neu5Acα2,3Galβ1,3GalNAcα1.
[3] The glycopeptide according to [1] or [2], wherein the binding position(s) of the glycan is one, two or three sites selected from the group consisting of the threonine at position 2, the threonine at position 5, and the threonine at position 6 from the N-terminal side.
[4] The glycopeptide according to any one of [1] to [3], wherein the binding position(s) of the glycan is the threonine at position 2, the threonine at position 5, the threonine at position 6, the threonines at positions 2 and 5, the threonines at positions 2 and 6, the threonines at positions 5 and 6, or the threonines at positions 2, 5 and 6, from the N-terminal side.
[5] The glycopeptide according to any one of [1] to [4], wherein the glycans binding to each of the two or more threonines are homologous or heterologous.
[6] The glycopeptide according to any one of [2] to [5], wherein the glycopeptide having the glycan (2) is a glycopeptide represented by any of the following structural formulae: 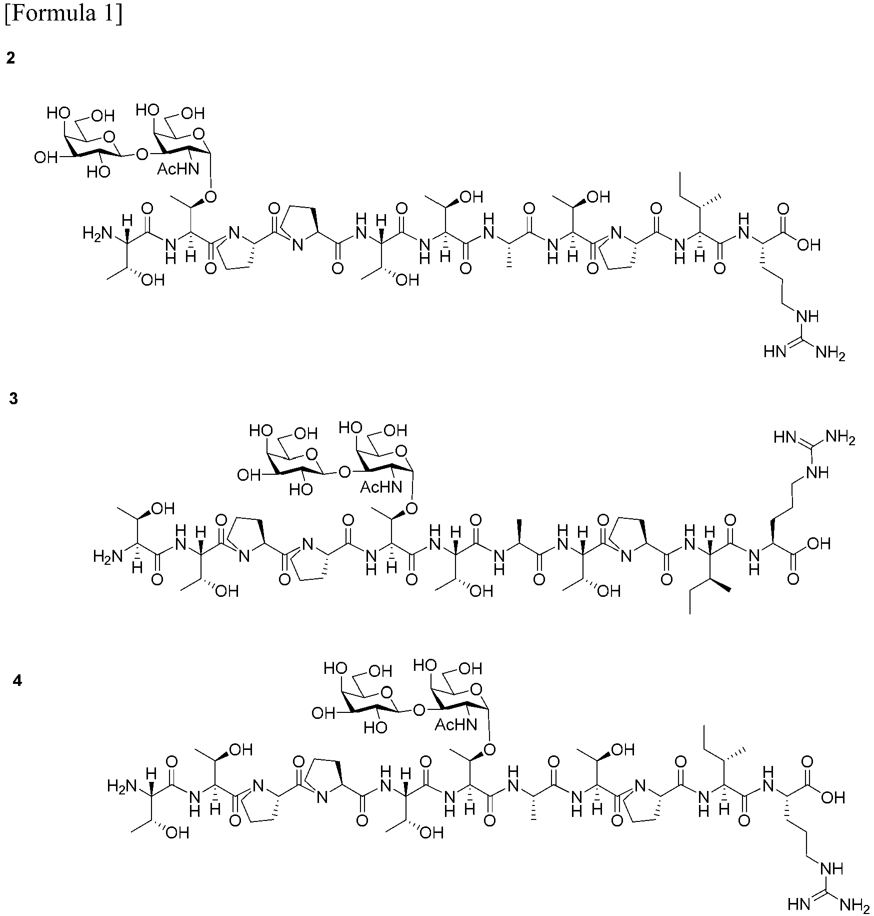 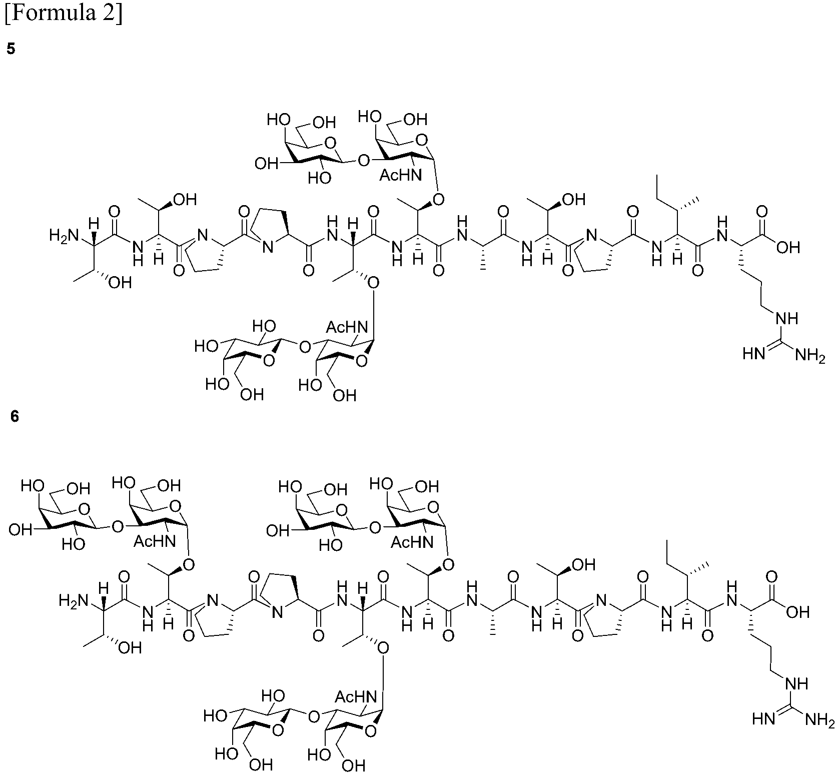
[7] The glycopeptide according to any one of [2] to [6], wherein the glycopeptide having the glycan (4) is a glycopeptide represented by any of the following structural formulae: 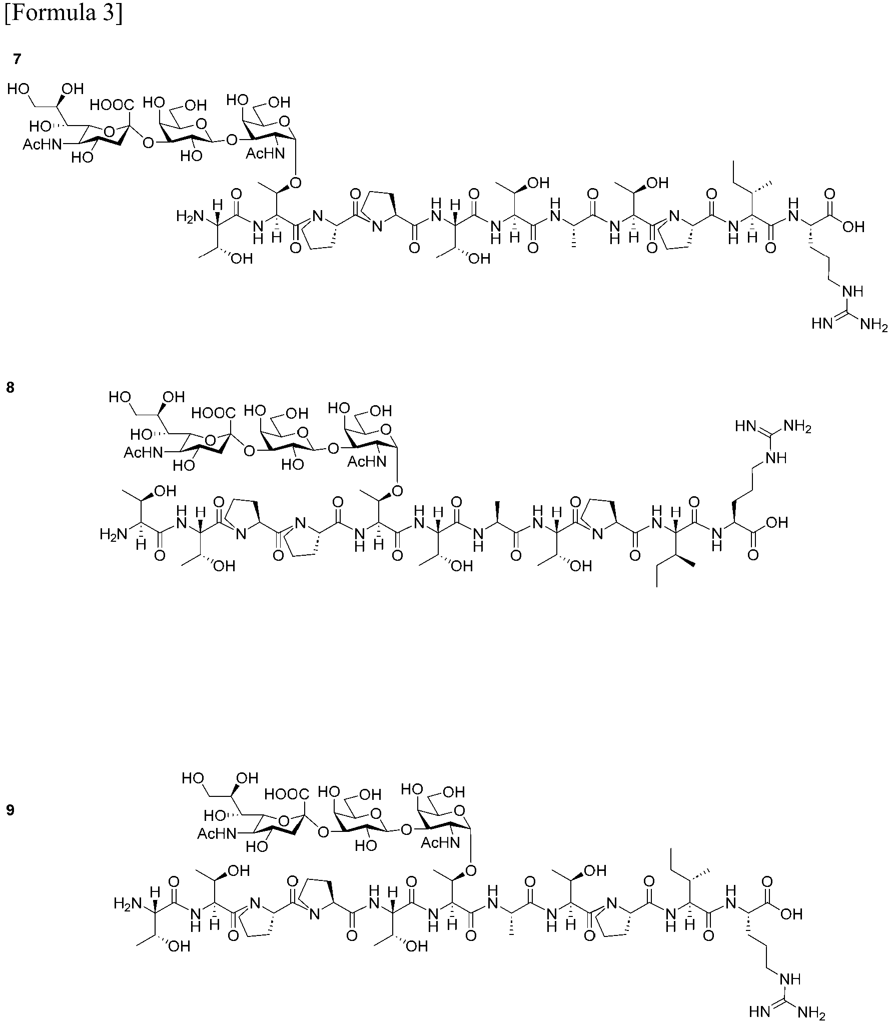 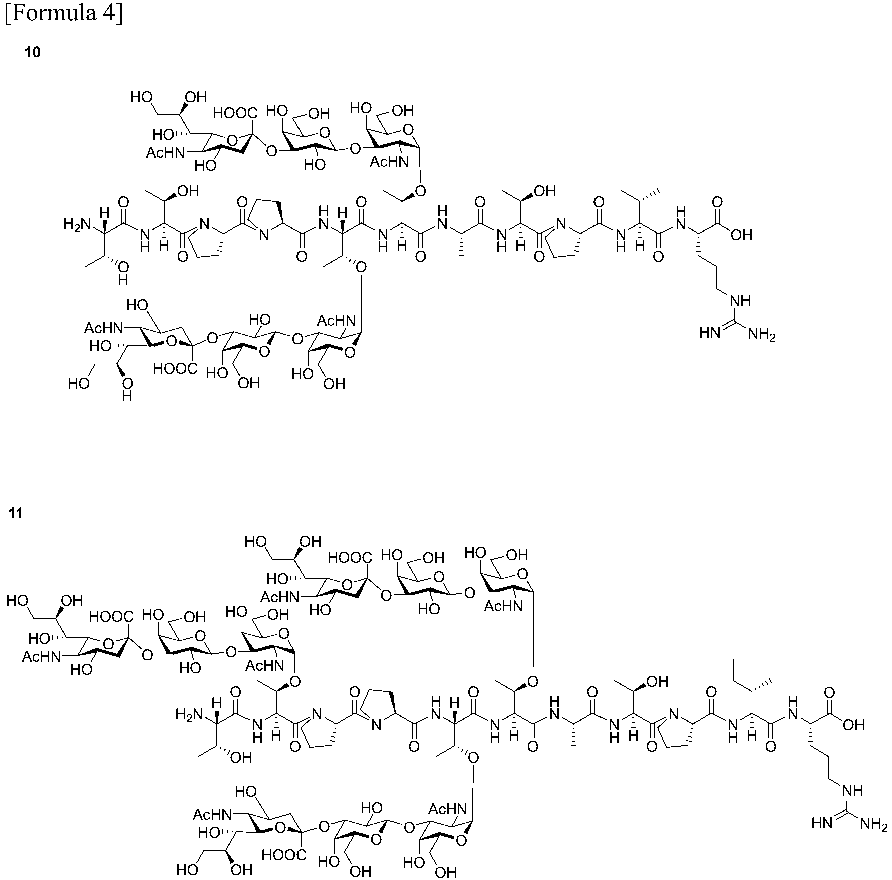
[8] The glycopeptide according to any one of [2] to [7], wherein the glycopeptide having the glycan (1) is a glycopeptide represented by any of the following structural formulae: 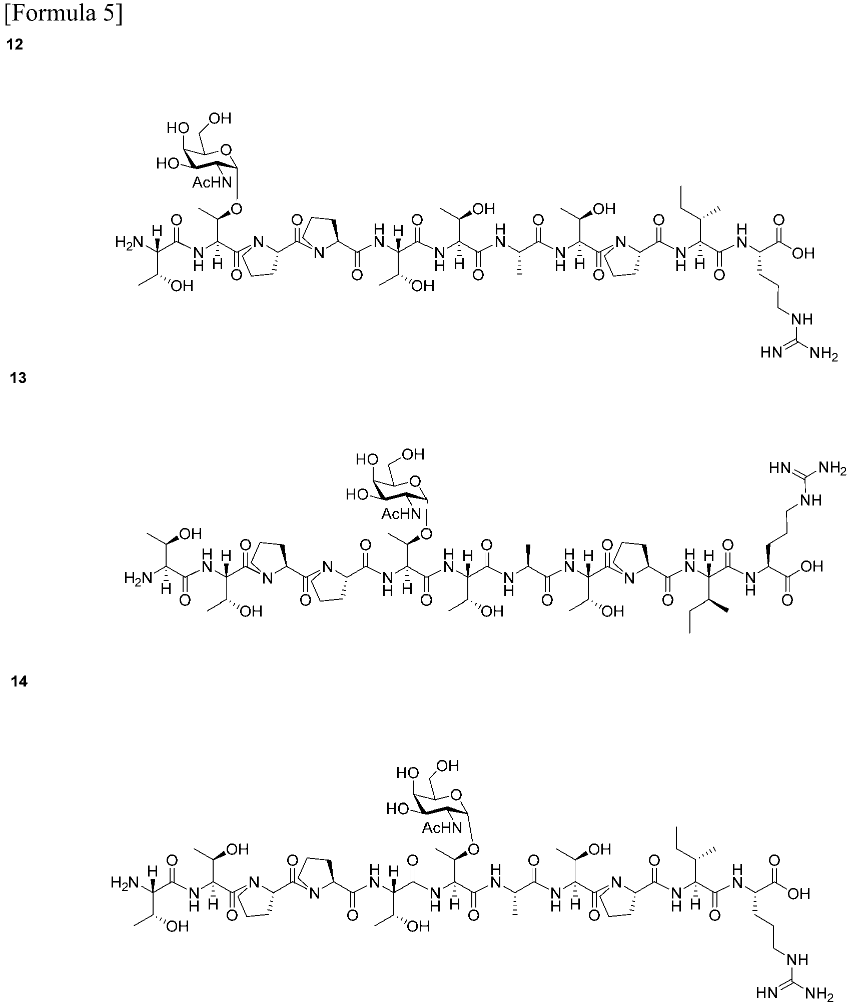 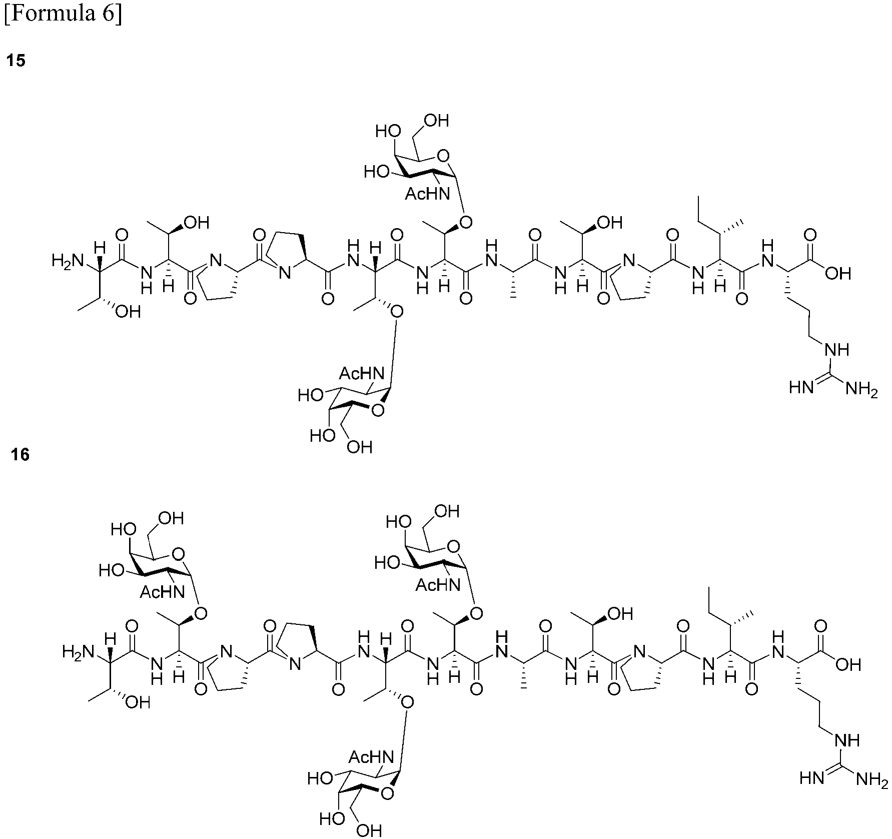
[9] The glycopeptide according to any one of [2] to [8], wherein the glycopeptide having the glycan (3) is a glycopeptide represented by any of the following structural formulae: 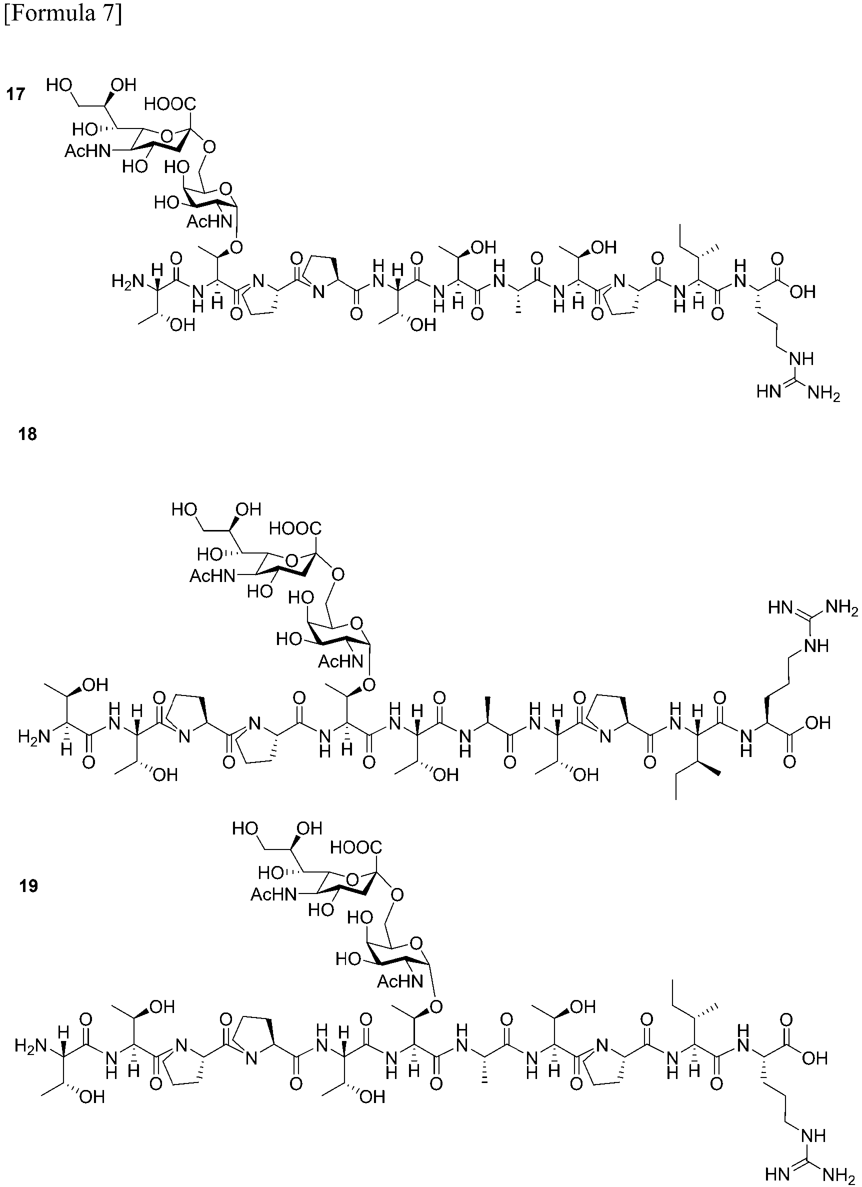 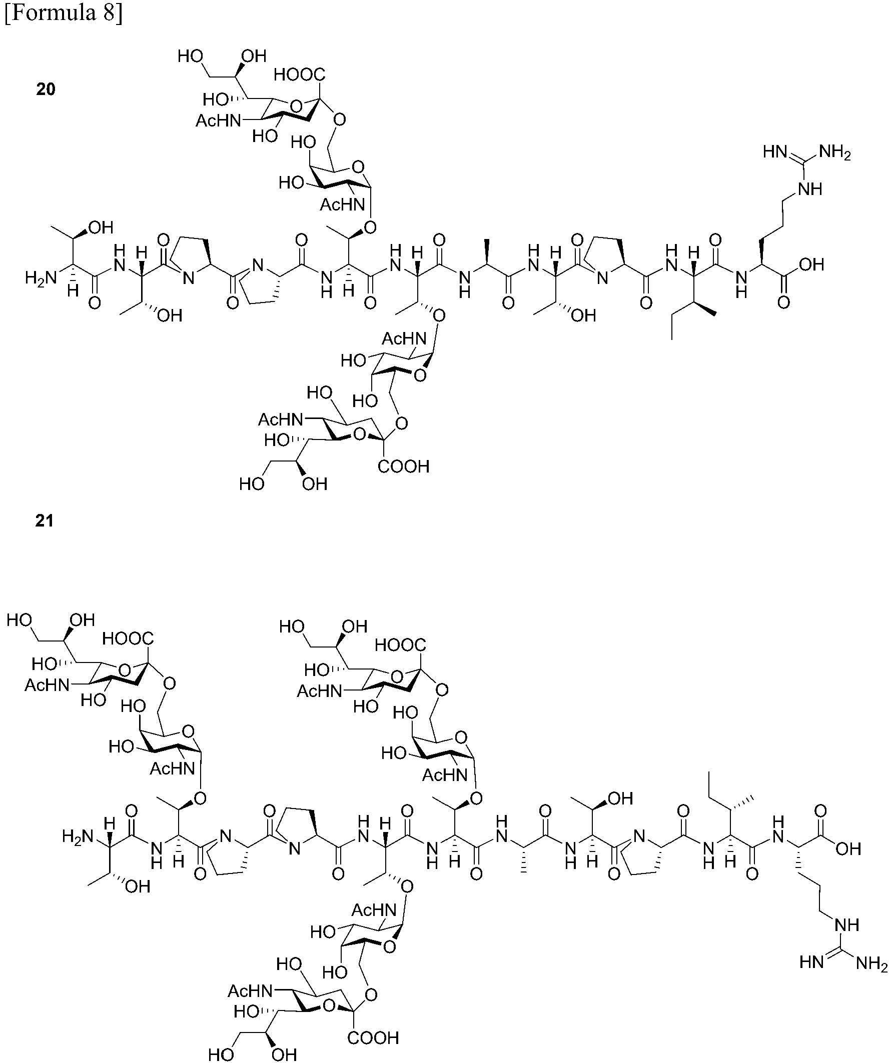
[10] The glycopeptide according to any one of [1] to [9], which is used as a tumor marker.
[11] The glycopeptide according to any one of [1] to [9], which is used as an immunogen for antibody production.
[12] A method for analyzing a glycopeptide, comprising:
   analyzing whether or not a glycopeptide is contained in a sample containing a glycopeptide obtained by protease digestion of the serum or the plasma, wherein
   the glycopeptide is a peptide consisting of the amino acid sequence TTPPTTATPIR, in which one glycan consisting of 6 or less sugars binds to each of one or two or more threonines, and the glycan is an O-linked glycan.
[13] The method for analyzing a glycopeptide according to [12], comprising analyzing whether or not Glycopeptide 15 and/or Glycopeptide 20 represented by the following structural formulae are contained in the sample: 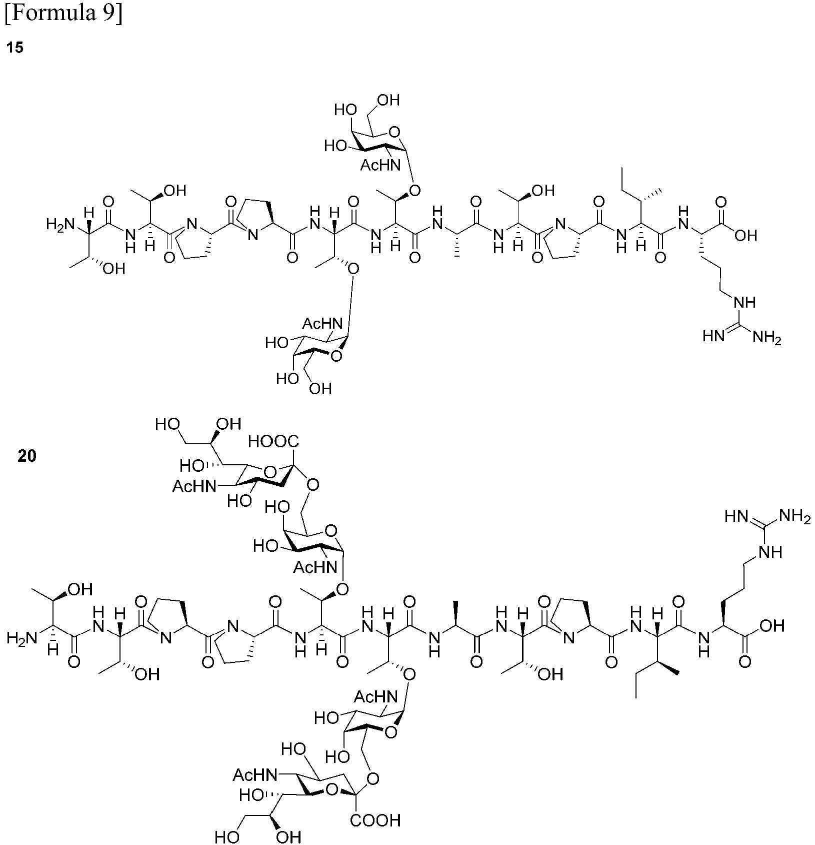
[14] The method for analyzing a glycopeptide according to [12] or [13], comprising further analyzing whether or not one type, or two or more types selected from the group consisting of Glycopeptide 5, Glycopeptide 9, Glycopeptide 10 and Glycopeptide 16 represented by the following structural formulae are contained in the sample: 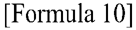 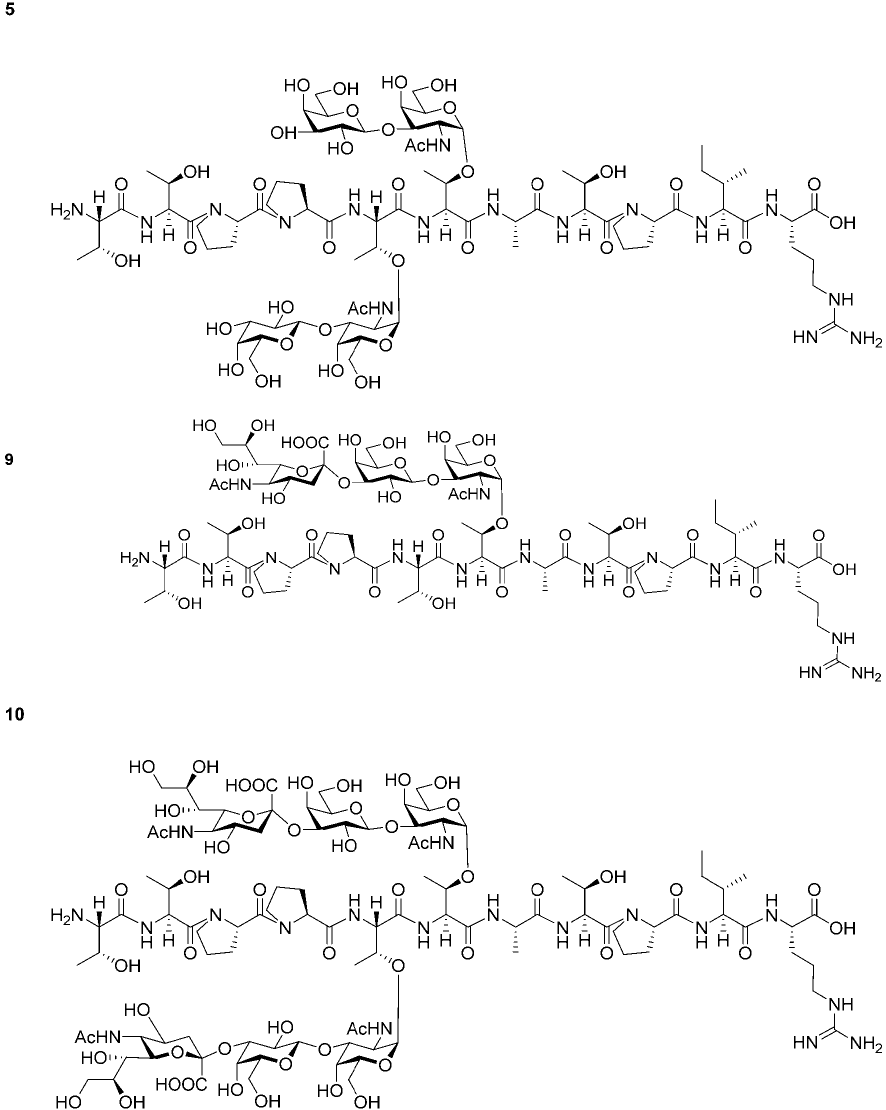 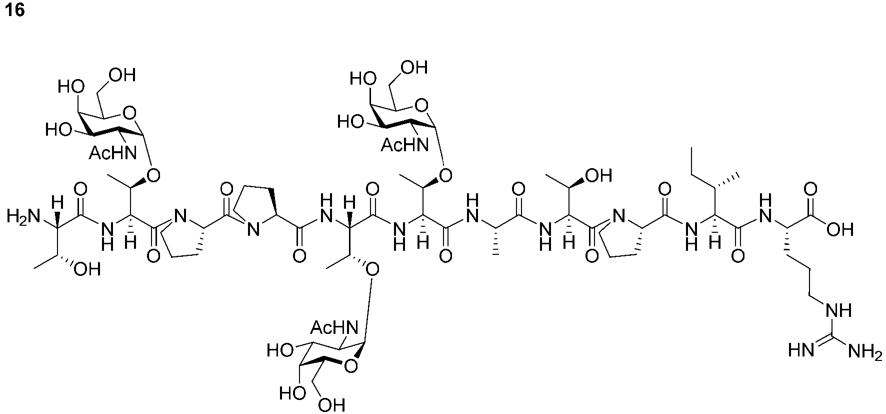
[15] A method for analyzing a glycopeptide, comprising analyzing whether or not one type, or two or more types selected from the group consisting of Glycopeptide 5, Glycopeptide 9, Glycopeptide 10 and Glycopeptide 16 represented by the following structural formulae are contained in a sample containing a glycopeptide obtained by protease digestion of the serum or the plasma: 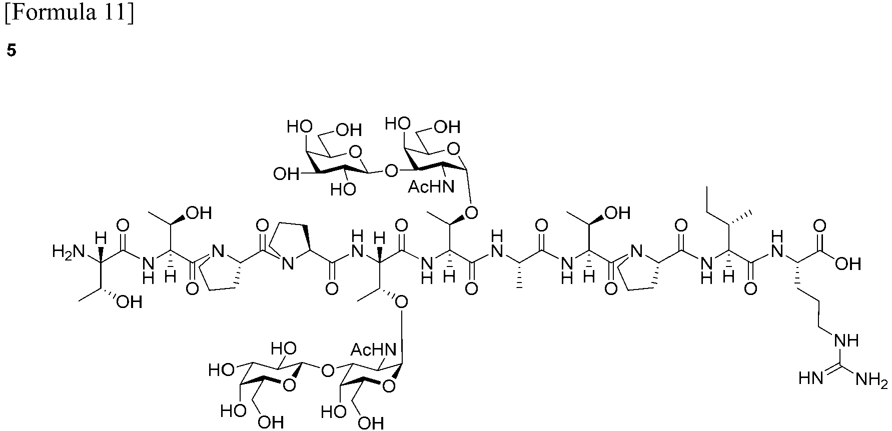 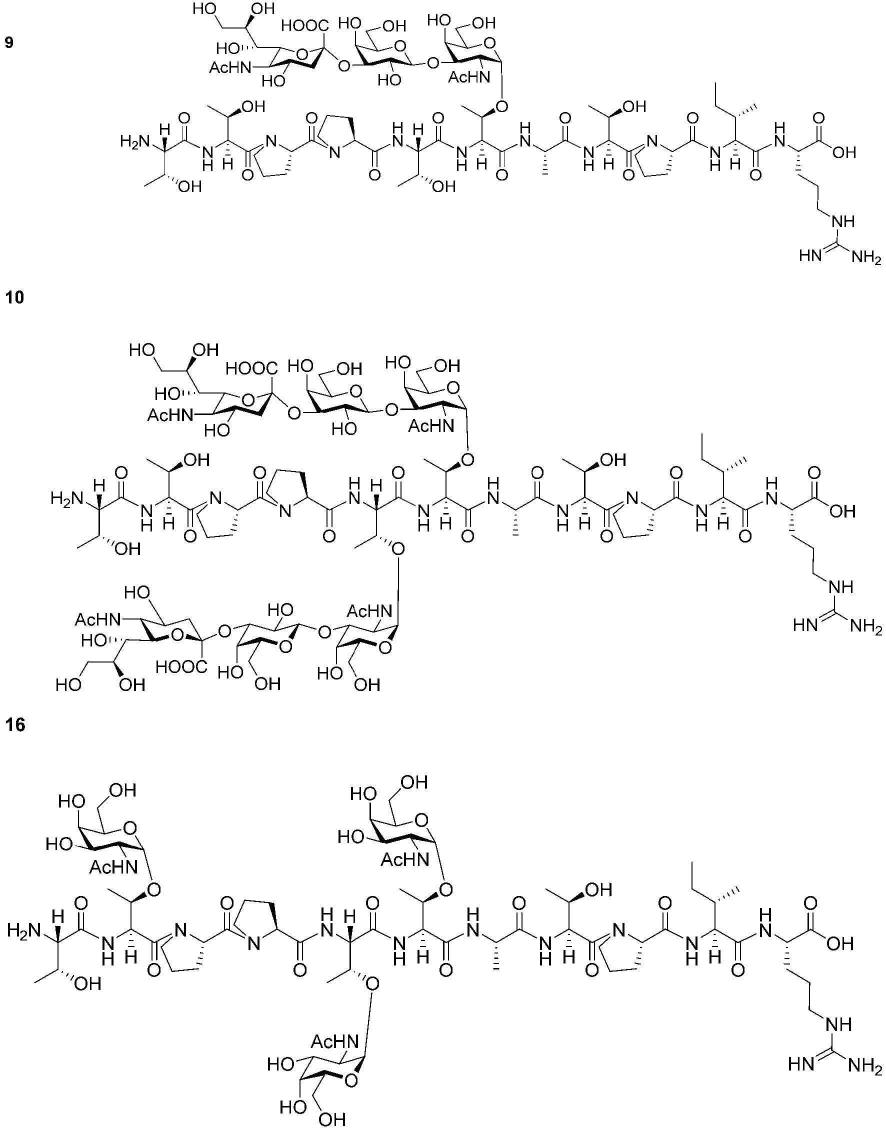
[16] The method for analyzing a glycopeptide according to any one of [12] to [15], wherein the analyzing comprises performing the mass spectrometry of the glycopeptide.
[17] The method for analyzing a glycopeptide according to any one of [12] to [16], wherein the analyzing comprises performing the mass spectrometry of the glycopeptide according to tandem mass spectrometry.
[18] The method for analyzing a glycopeptide according to any one of [12] to [17], which is used in detection of digestive system cancer and/or detection of the stage of digestive system cancer progression.
[19] The method for analyzing a glycopeptide according to [18], wherein the digestive system cancer is liver cancer or pancreatic cancer.
[20] The method for analyzing a glycopeptide according to any one of [12] to [19], wherein the sample is obtained by removing a portion of a serum protein or a plasma protein.
[21] A method for analyzing cancer, including the method for analyzing a glycopeptide according to any one of [12] to [20].

### Advantageous Effects of Invention

According to the present invention, there can be provided a novel glycopeptide for systematically searching for and identifying epitopes specific to various diseases including cancer. By using this glycopeptide, a disease-specific dynamic epitope can be identified, and a method for analyzing diseases using this dynamic epitope can be established. By identifying such a disease-specific dynamic epitope, the development of a novel therapeutic antibody drug is also expected.

### Brief Description of Drawings

[Figure 1] Figure 1 shows the results of mass spectrometry of Peptide 1 by MALDI-TOF/MS.
[Figure 2] Figure 2 shows the results of mass spectrometry of Glycopeptide 2 by MALDI-TOF/MS.
[Figure 3] Figure 3 shows the results of mass spectrometry of Glycopeptide 3 by MALDI-TOF/MS.
[Figure 4] Figure 4 shows the results of mass spectrometry of Glycopeptide 4 by MALDI-TOF/MS.
[Figure 5] Figure 5 shows the results of mass spectrometry of Glycopeptide 5 by MALDI-TOF/MS.
[Figure 6] Figure 6 shows the results of mass spectrometry of Glycopeptide 6 by MALDI-TOF/MS.
[Figure 7] Figure 7 shows the results of mass spectrometry of Glycopeptide 7 by MALDI-TOF/MS.
[Figure 8] Figure 8 shows the results of mass spectrometry of Glycopeptide 8 by MALDI-TOF/MS.
[Figure 9] Figure 9 shows the results of mass spectrometry of Glycopeptide 9 by MALDI-TOF/MS.
[Figure 10] Figure 10 shows the results of mass spectrometry of Glycopeptide 10 by MALDI-TOF/MS.
[Figure 11] Figure 11 shows the results of mass spectrometry of Glycopeptide 11 by MALDI-TOF/MS.
[Figure 12] Figure 12 shows the results of mass spectrometry of Glycopeptide 12 by MALDI-TOF/MS.
[Figure 13] Figure 13 shows the results of mass spectrometry of Glycopeptide 13 by MALDI-TOF/MS.
[Figure 14] Figure 14 shows the results of mass spectrometry of Glycopeptide 14 by MALDI-TOF/MS.
[Figure 15] Figure 15 shows the results of mass spectrometry of Glycopeptide 15 by MALDI-TOF/MS.
[Figure 16] Figure 16 shows the results of mass spectrometry of Glycopeptide 16 by MALDI-TOF/MS.
[Figure 17] Figure 17 shows the results of mass spectrometry of Glycopeptide 17 by MALDI-TOF/MS.
[Figure 18] Figure 18 shows the results of mass spectrometry of Glycopeptide 18 by MALDI-TOF/MS.
[Figure 19] Figure 19 shows the results of mass spectrometry of Glycopeptide 19 by MALDI-TOF/MS.
[Figure 20] Figure 20 shows the results of mass spectrometry of Glycopeptide 20 by MALDI-TOF/MS.
[Figure 21] Figure 21 shows the results of mass spectrometry of Glycopeptide 21 by MALDI-TOF/MS.
[Figure 22] (a) shows the spectrum of Glycopeptide (16) at an ER mode. (b) shows the results of observation of fragment ions generated by CID for optimization of Q3.
[Figure 23] Figure 23 is a schematic view explaining the method of quadrupole mass spectrometry.
[Figure 24] Figure 24 shows the calibration curve of Peptide 1.
[Figure 25] Figure 25 shows the calibration curve of Glycopeptide 2.
[Figure 26] Figure 26 shows the calibration curve of Glycopeptide 3.
[Figure 27] Figure 27 shows the calibration curve of Glycopeptide 4.
[Figure 28] Figure 28 shows the calibration curve of Glycopeptide 5.
[Figure 29] Figure 29 shows the calibration curve of Glycopeptide 6.
[Figure 30] Figure 30 shows the calibration curve of Glycopeptide 7.
[Figure 31] Figure 31 shows the calibration curve of Glycopeptide 8.
[Figure 32] Figure 32 shows the calibration curve of Glycopeptide 9.
[Figure 33] Figure 33 shows the calibration curve of Glycopeptide 10.
[Figure 34] Figure 34 shows the calibration curve of Glycopeptide 11.
[Figure 35] Figure 35 shows the calibration curve of Glycopeptide 12.
[Figure 36] Figure 36 shows the calibration curve of Glycopeptide 13.
[Figure 37] Figure 37 shows the calibration curve of Glycopeptide 14.
[Figure 38] Figure 38 shows the calibration curve of Glycopeptide 15.
[Figure 39] Figure 39 shows the calibration curve of Glycopeptide 16.
[Figure 40] Figure 40 shows the calibration curve of Glycopeptide 17.
[Figure 41] Figure 41 shows the calibration curve of Glycopeptide 18.
[Figure 42] Figure 42 shows the calibration curve of Glycopeptide 19.
[Figure 43] Figure 43 shows the calibration curve of Glycopeptide 20.
[Figure 44] Figure 44 shows the calibration curve of Glycopeptide 21.
[Figure 45] Figure 45 shows the results of the blood concentration (nM) of Peptide 1 in individual specimens from healthy subjects and liver cancer patients, displayed as a box plot.
[Figure 46] Figure 46 shows the results of the blood concentration (nM) of Glycopeptide 2 in individual specimens from healthy subjects and liver cancer patients, displayed as a box plot.
[Figure 47] Figure 47 shows the results of the blood concentration (nM) of Glycopeptide 3 in individual specimens from healthy subjects and liver cancer patients, displayed as a box plot.
[Figure 48] Figure 48 shows the results of the blood concentration (nM) of Glycopeptide 4 in individual specimens from healthy subjects and liver cancer patients, displayed as a box plot.
[Figure 49] Figure 49 shows the results of the blood concentration (nM) of Glycopeptide 5 in individual specimens from healthy subjects and liver cancer patients, displayed as a box plot.
[Figure 50] Figure 50 shows the results of the blood concentration (nM) of Glycopeptide 6 in individual specimens from healthy subjects and liver cancer patients, displayed as a box plot.
[Figure 51] Figure 51 shows the results of the blood concentration (nM) of Glycopeptide 7 in individual specimens from healthy subjects and liver cancer patients, displayed as a box plot.
[Figure 52] Figure 52 shows the results of the blood concentration (nM) of Glycopeptide 8 in individual specimens from healthy subjects and liver cancer patients, displayed as a box plot.
[Figure 53] Figure 53 shows the results of the blood concentration (nM) of Glycopeptide 9 in individual specimens from healthy subjects and liver cancer patients, displayed as a box plot.
[Figure 54] Figure 54 shows the results of the blood concentration (nM) of Glycopeptide 10 in individual specimens from healthy subjects and liver cancer patients, displayed as a box plot.
[Figure 55] Figure 55 shows the results of the blood concentration (nM) of Glycopeptide 11 in individual specimens from healthy subjects and liver cancer patients, displayed as a box plot.
[Figure 56] Figure 56 shows the results of the blood concentration (nM) of Glycopeptide 12 in individual specimens from healthy subjects and liver cancer patients, displayed as a box plot.
[Figure 57] Figure 57 shows the results of the blood concentration (nM) of Glycopeptide 13 in individual specimens from healthy subjects and liver cancer patients, displayed as a box plot.
[Figure 58] Figure 58 shows the results of the blood concentration (nM) of Glycopeptide 14 in individual specimens from healthy subjects and liver cancer patients, displayed as a box plot.
[Figure 59] Figure 59 shows the results of the blood concentration (nM) of Glycopeptide 15 in individual specimens from healthy subjects and liver cancer patients, displayed as a box plot.
[Figure 60] Figure 60 shows the results of the blood concentration (nM) of Glycopeptide 16 in individual specimens from healthy subjects and liver cancer patients, displayed as a box plot.
[Figure 61] Figure 61 shows the results of the blood concentration (nM) of Glycopeptide 17 in individual specimens from healthy subjects and liver cancer patients, displayed as a box plot.
[Figure 62] Figure 62 shows the results of the blood concentration (nM) of Glycopeptide 18 in individual specimens from healthy subjects and liver cancer patients, displayed as a box plot.
[Figure 63] Figure 63 shows the results of the blood concentration (nM) of Glycopeptide 19 in individual specimens from healthy subjects and liver cancer patients, displayed as a box plot.
[Figure 64] Figure 64 shows the results of the blood concentration (nM) of Glycopeptide 20 in individual specimens from healthy subjects and liver cancer patients, displayed as a box plot.
[Figure 65] Figure 65 shows the results of the blood concentration (nM) of Glycopeptide 21 in individual specimens from healthy subjects and liver cancer patients, displayed as a box plot.
[Figure 66] Figure 66 shows the results of a statistical significance test for Glycopeptides 5, 9, 15, 16, and 20, which show a significant correlation with the progression (stage) of liver cancer.
[Figure 67] Figure 67 is a view explaining a structural change generated by post-translational modification in peptide region.
[Figure 68] Figure 68 shows the results of the blood concentration (nM) of Glycopeptide 5 in individual stage 4 specimens from healthy subjects, liver cancer patients and pancreatic cancer patients, displayed as a box plot.
[Figure 69] Figure 69 shows the results of the blood concentration (nM) of Glycopeptide 7 in individual stage 4 specimens from healthy subjects, liver cancer patients and pancreatic cancer patients, displayed as a box plot.
[Figure 70] Figure 70 shows the results of the blood concentration (nM) of Glycopeptide 8 in individual stage 4 specimens from healthy subjects, liver cancer patients and pancreatic cancer patients, displayed as a box plot.
[Figure 71] Figure 71 shows the results of the blood concentration (nM) of Glycopeptide 9 in individual stage 4 specimens from healthy subjects, liver cancer patients and pancreatic cancer patients, displayed as a box plot.
[Figure 72] Figure 72 shows the results of the blood concentration (nM) of Glycopeptide 10 in individual stage 4 specimens from healthy subjects, liver cancer patients and pancreatic cancer patients, displayed as a box plot.
[Figure 73] Figure 73 shows the results of the blood concentration (nM) of Glycopeptide **11** in individual stage 4 specimens from healthy subjects, liver cancer patients and pancreatic cancer patients, displayed as a box plot.
[Figure 74] Figure 74 shows the results of the blood concentration (nM) of Glycopeptide 15 in individual stage 4 specimens from healthy subjects, liver cancer patients and pancreatic cancer patients, displayed as a box plot.
[Figure 75] Figure 75 shows the results of the blood concentration (nM) of Glycopeptide 16 in individual stage 4 specimens from healthy subjects, liver cancer patients and pancreatic cancer patients, displayed as a box plot.
[Figure 76] Figure 76 shows the results of the blood concentration (nM) of Glycopeptide 20 in individual stage 4 specimens from healthy subjects, liver cancer patients and pancreatic cancer patients, displayed as a box plot.

### Embodiments of Carrying out the Invention

The present invention will be described in detail below. The explanation given below may be described based on some typical embodiments or specific examples. However, the present invention should not be limited to such embodiments. It is to be noted that, in the present description, the numerical range expressed with the wording "a number to another number" means the range that falls between the former number indicating the lower limit value of the range and the latter number indicating the upper limit value thereof.

### (Glycopeptide)

The present embodiment relates to a glycopeptide, in which one glycan consisting of 6 or less sugars binds to each of one or two or more threonines in a peptide consisting of the amino acid sequence TTPPTTATPIR (SEQ ID NO: 1: Thr-Thr-Pro-Pro-Thr-Thr-Ala-Thr-Pro-Ile-Arg), and the glycan is an O-linked glycan. The O-linked glycan is a glycan that binds to the hydroxyl group of threonine via an O-glycoside bond. In the amino acid sequence, T represents threonine (Thr), P represents proline (Pro), A represents alanine (Ala), I represents isoleucine (Ile), and R represents arginine (Arg). In addition, in the amino acid sequence shown in the present description, the left end indicates the N-terminus, and the right end indicates the C-terminus.

It is to be noted that the "glycopeptide" of the present embodiment also includes glycopeptide derivatives. Examples of the glycopeptide derivatives may include those with cysteine introduced into the C-terminus of the aforementioned glycopeptide, and those with levulinic acid introduced into the N-terminus thereof.

The glycopeptide of the present embodiment is a synthetic glycopeptide that can be produced, for example, by *in vitro* synthesis, and the present glycopeptide is a novel substance (novel compound).

In the present description, the "glycan" refers to a compound that can be formed by linking one or more sugar units (monosaccharides and/or their derivatives). When two or more sugar units are linked together, the individual sugar units are allowed to bind to one another by dehydration condensation via a glycosidic bond. It is to be noted that the sugars that constitute the O-linked glycan may be monosaccharides, or that the O-linked glycan may consist of 1, 2, 3, 4, 5, or 6 sugars, and the O-linked glycan preferably consists of 1, 2, or 3 sugars, and more preferably consists of 1 or 2 sugars.

Glycan preferably has N-acetylgalactosamine (GalNAc) as a constituent sugar. When the glycan consists of a single sugar, it preferably consists of N-acetylgalactosamine (GalNAc), and it is particularly preferably the glycan represented by the following (1).

(1) GalNAcα1

When the glycan consists of two or more sugars, it preferably has, as a constituent sugar, N-acetylgalactosamine (GalNAc), galactose (Gal), or N-acetylneuraminic acid (Neu5Ac), and the glycan is preferably selected from the group consisting of the following (2) to (4). In the glycans of the following formulas (2) to (4), GalNAcα1 binds to threonine.

(2) Galβ1,3GalNAcα1

(3) Neu5Acα2,6GalNAcα1

(4) Neu5Acα2,3Galβ1,3GalNAcα1

It is to be noted that the glycans represented by the above (1) to (4) are four O-glycan structures known to be prominently observed in cancer cells. Among the above-described glycans, GalNAcα1 is sometimes called Tn antigen, Galβ1,3GalNAcα1 is sometime called T antigen, Neu5Acα2,6GalNAcα1 is sometimes called sialyl T antigen (STn antigen), and Neu5Acα2,3Galβ1,3GalNAcα1 is sometimes called sialyl T antigen (ST antigen).

When the glycan consists of three or more sugars, it preferably has, as constituent sugar(s), one type, or two or more types selected from the group consisting of N-acetylgalactosamine (GalNAc), galactose (Gal), N-acetylneuraminic acid (Neu5Ac), fucose (Fuc), and N-acetylglucosamine (GlcNAc). Examples of such glycan may include disialyl T antigen (Neu5Acα2,3Galβ1,3[Neu5Acα2,6]GalNAcα1) and sialyl Lewis x/a glycan (Neu5Acα2,3Galβ1,4[Fucα1,3]GlcNAc or Neu5Acα2,3Galβ1,3[Fucα1,4]GlcNAc).

In the glycopeptide of the present embodiment, glycan binds to each of one or two or more threonines in the above-described peptide having a predetermined sequence. In this case, glycan binds to each of the following threonines. It is to be noted that, in the present description, for example, a state in which a glycan binding to the threonine at position 1 from the N-terminal side is designated T1, a state in which a glycan binding to the threonine at position 2 from the N-terminal side is designated T2, a state in which a glycan binding to the threonine at position 5 from the N-terminal side is designated T5, a state in which a glycan binding to the threonine at position 6 from the N-terminal side is designated T6, and a state in which a glycan binding to the threonine at position 8 from the N-terminal side is designated T8.
(1) Threonine (T1) at position 1 from the N-terminal side
(2) Threonine (T2) at position 2 from the N-terminal side
(3) Threonine (T5) at position 5 from the N-terminal side
(4) Threonine (T6) at position 6 from the N-terminal side
(5) Threonine (T8) at position 8 from the N-terminal side

When glycan binds to each of two threonines in the above-described peptide having a predetermined sequence, the combination of threonines to which glycan binds is, for example, as follows. In the present description, for example, when glycan binds to both of the threonine at position 1 from the N-terminal side and the threonine at position 2 from the N-terminal side, it is designated T1/T2.
(6) T1/T2, T1/T5, T1/T6, T1/T8
(7) T2/T5, T2/T6, T2/T8
(8) T5/T6, T5/T8
(9) T6/T8

When glycan binds to each of three threonines in the above-described peptide having a predetermined sequence, the combination of threonines to which glycan binds is, for example, as follows. In the present description, for example, when glycan binds to all of the threonine at position 1 from the N-terminal side, the threonine at position 2 from the N-terminal side, and the threonine at position 5 from the N-terminal side, it is designated T1/T2/T5.
(10) T1/T2/T5, T1/T2/T6, T1/T2/T8, T1/T5/T6, T1/T5/T8, T1/T6/T8,
(11) T2/T5/T6, T2/T5/T8, T2/T6/T8, T5/T6/T8

When glycan binds to each of four threonines in the above-described peptide having a predetermined sequence, the combination of threonines to which glycan binds is, for example, as follows.
(12) T1/T2/T5/T6, T1/T2/T5/T8, T2/T5/T6/T8

When glycan binds to each of five threonines in the above-described peptide having a predetermined sequence, the combination of threonines to which glycan binds is, for example, as follows.
(13) T1/T2/T5/T6/T8

Among others, the binding position of glycan is preferably 1 site, or 2 or 3 sites selected from the group consisting of threonine (T2) at position 2 from the N-terminal side, threonine (T5) at position 5 from the N-terminal side, and threonine (T6) at position 6 from the N-terminal side. More specifically, the binding position of glycan is preferably threonine (T2) at position 2 from the N-terminal side, threonine (T5) at position 5 from the N-terminal side, threonine (T6) at position 6 from the N-terminal side, threonines (T2/T5) at positions 2 and 5 from the N-terminal side, threonines (T2/T6) at positions 2 and 6 from the N-terminal side, threonines (T5/T6) at positions 5 and 6 from the N-terminal side, or threonines (T2/T5/T6) at positions 2, 5 and 6 from the N-terminal side; and the binding position of glycan is more preferably threonine (T2) at position 2 from the N-terminal side, threonine (T5) at position 5 from the N-terminal side, threonine (T6) at position 6 from the N-terminal side, threonines (T5/T6) at positions 5 and 6 from the N-terminal side, or threonines (T2/T5/T6) at positions 2, 5 and 6 from the N-terminal side.

When glycan binds to each of two or more threonines, the glycans binding to individual threonines may be either homologous or heterologous, and the glycans are preferably homologous. In particular, homologous glycans preferably bind to threonines (T5/T6) at positions 5 and 6.

The glycopeptide of the present embodiment is preferably represented, for example, by any of the following structural formulae. For example, the glycopeptide having the aforementioned (1) glycan (GalNAcα1) is preferably a glycopeptide represented by any of the following structural formulae.

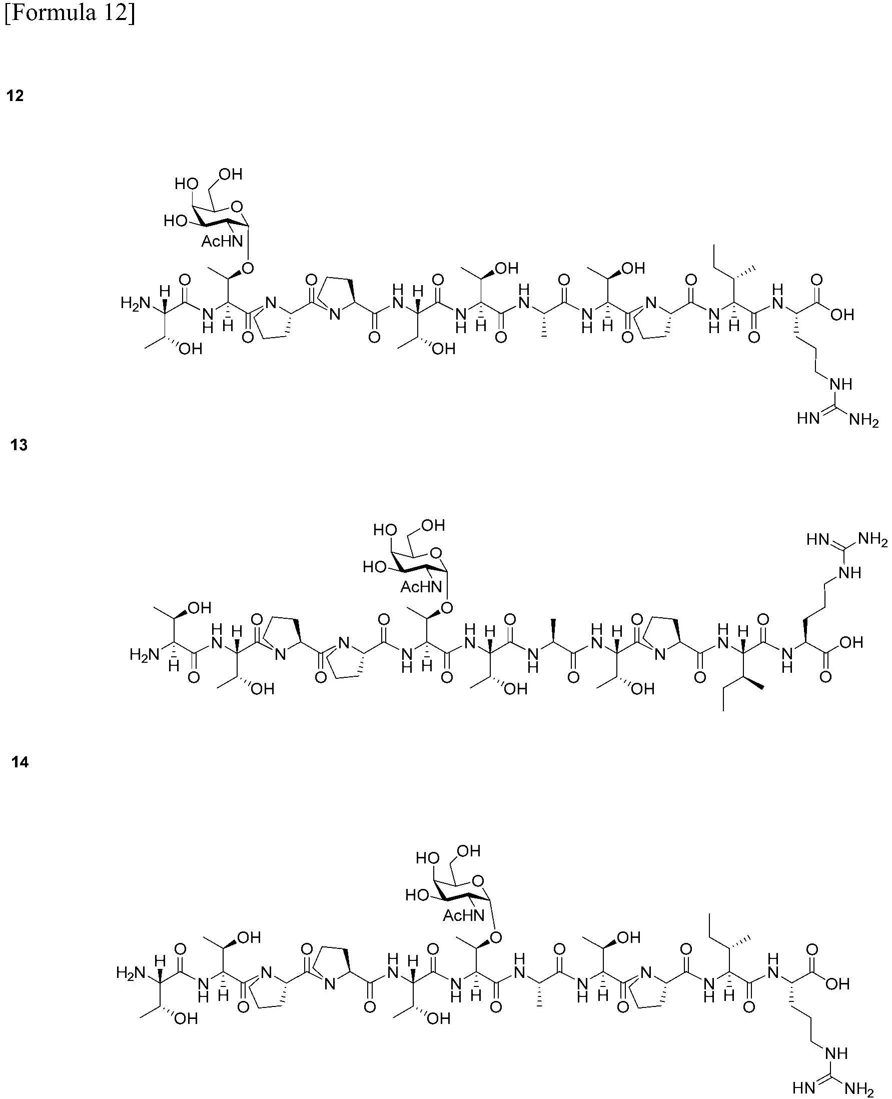

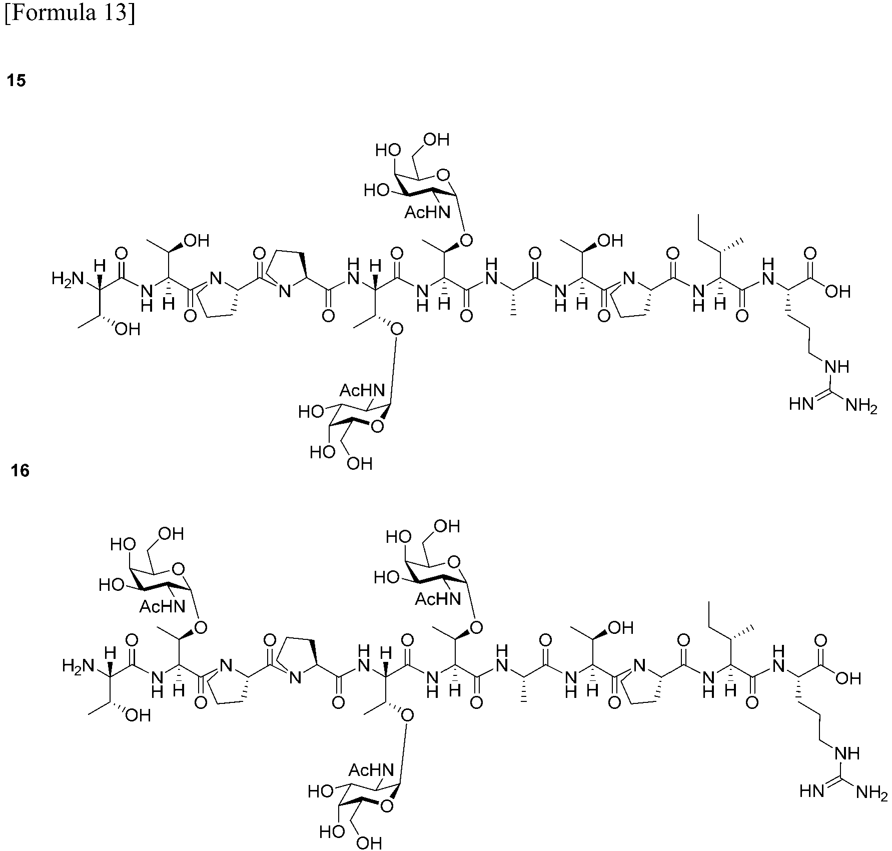

The glycopeptide having the aforementioned (2) glycan (Galβ1,3GalNAcα1) is preferably a glycopeptide represented by any of the following structural formulae.

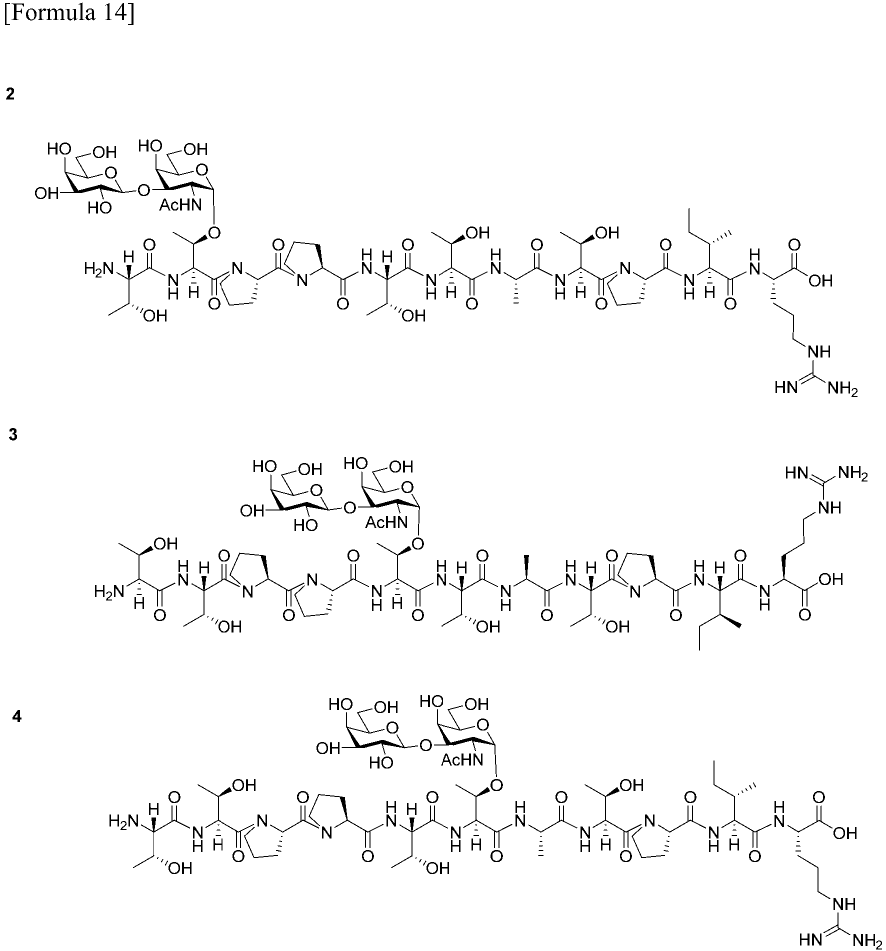

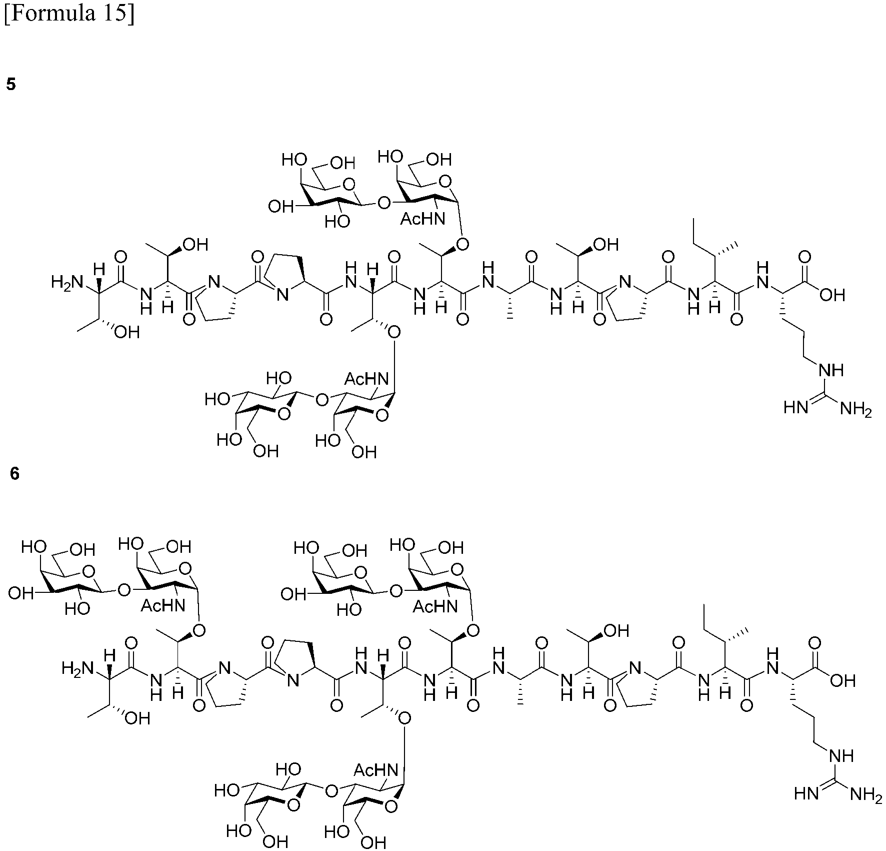

The glycopeptide having the aforementioned (3) glycan (Neu5Acα2,6GalNAcα1) is preferably a glycopeptide represented by any of the following structural formulae.

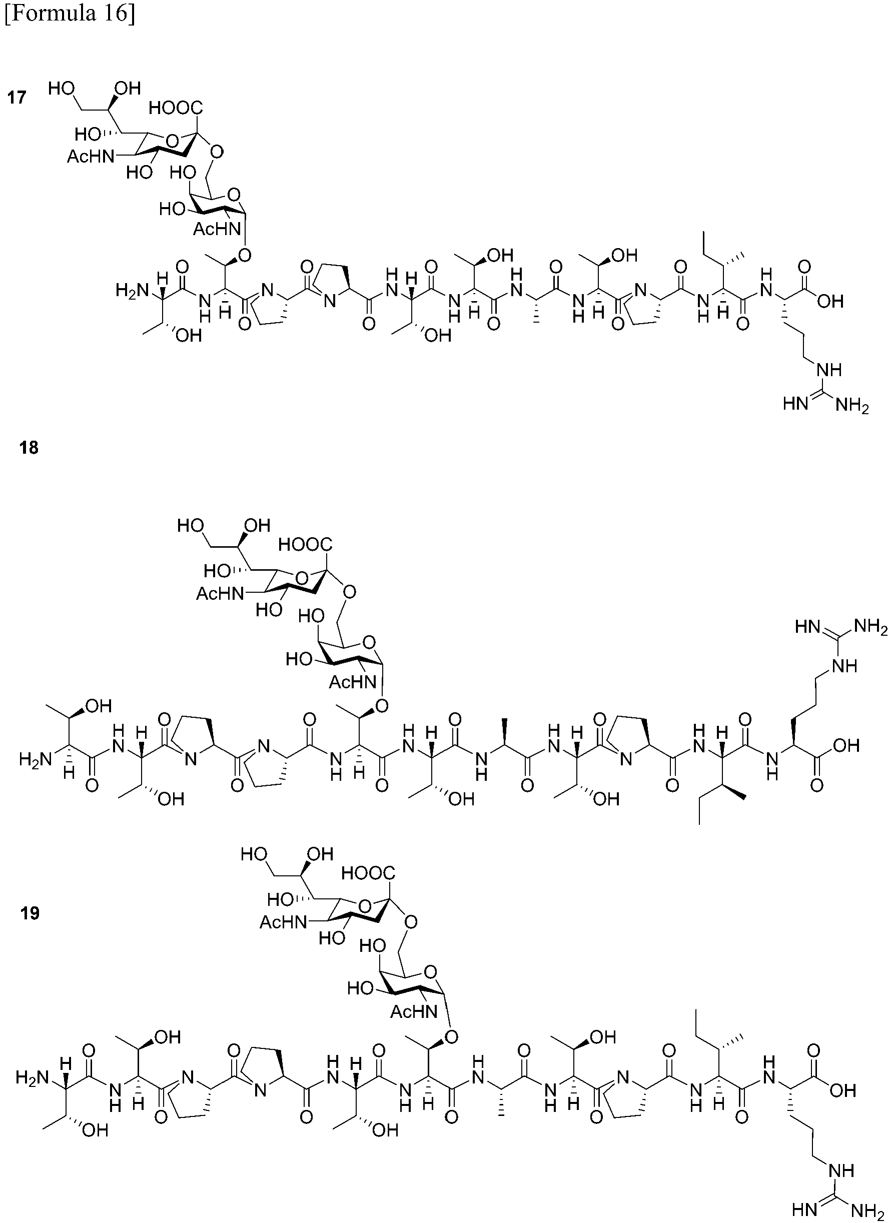

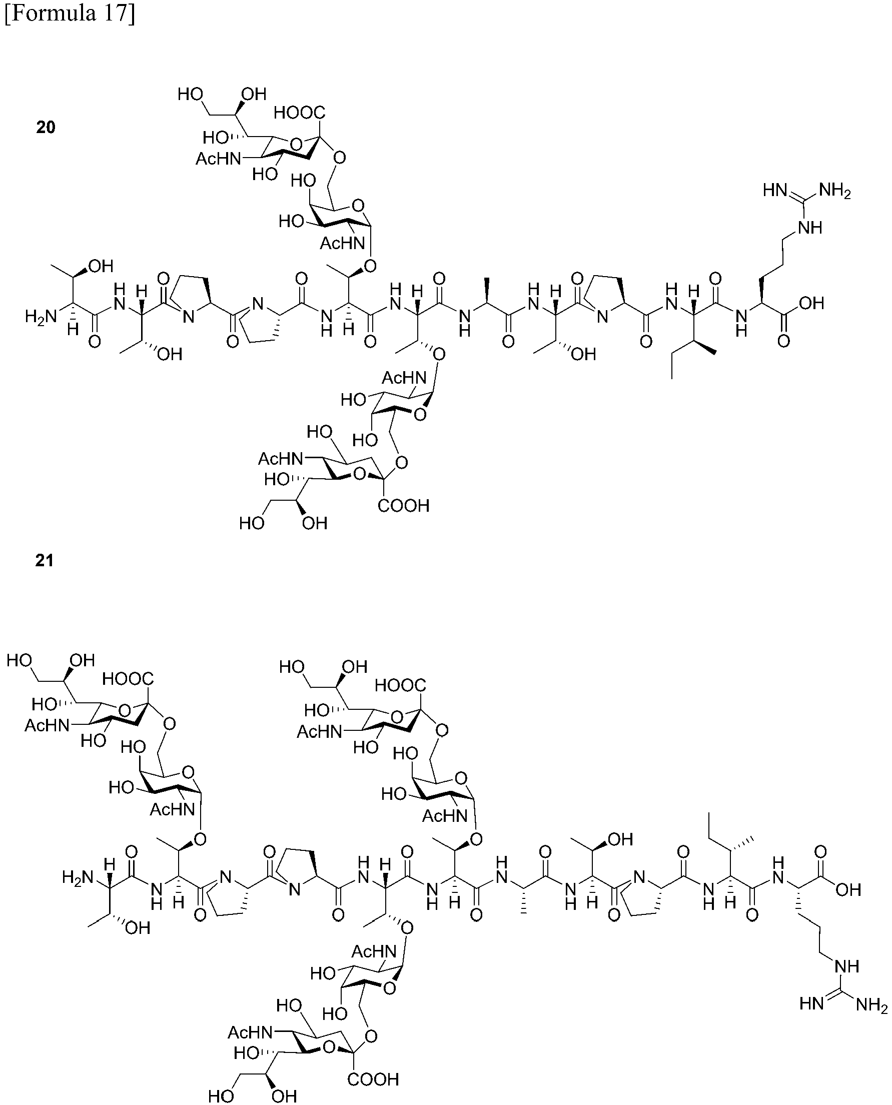

The glycopeptide having the aforementioned (4) glycan (Neu5Acα2,3Galβ1,3GalNAcα1) is preferably a glycopeptide represented by any of the following structural formulae.

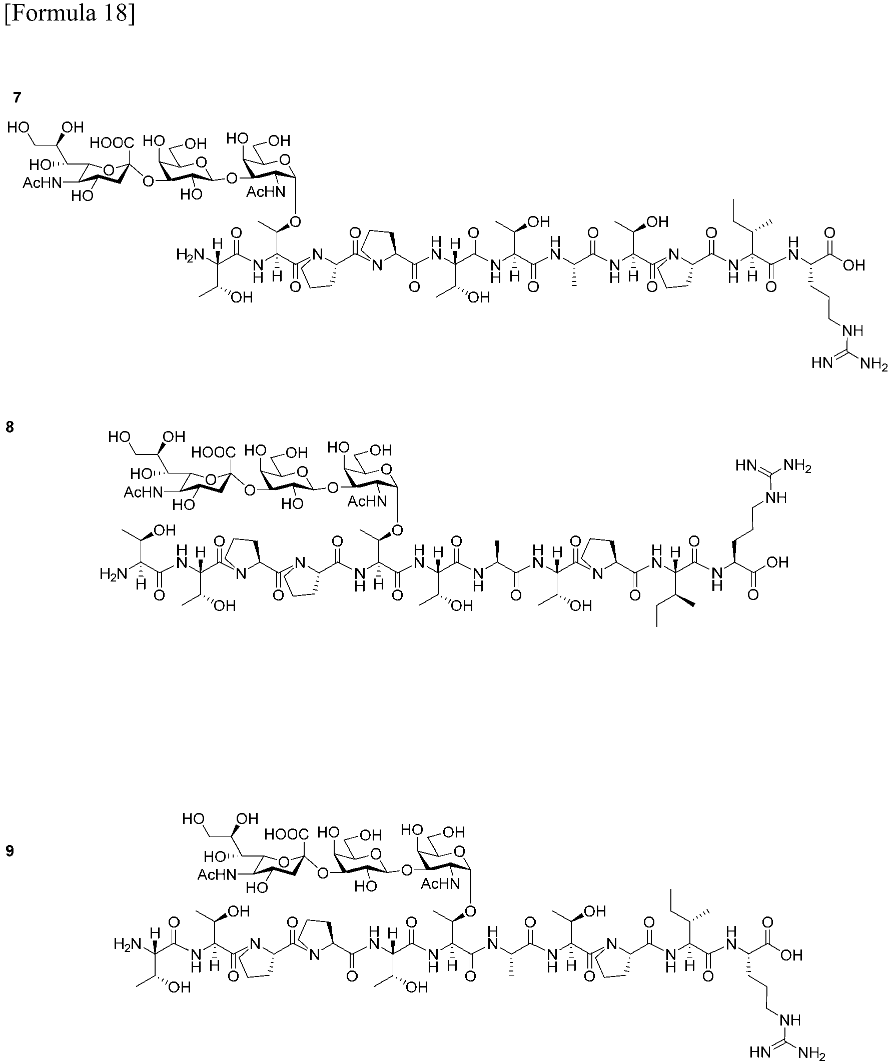

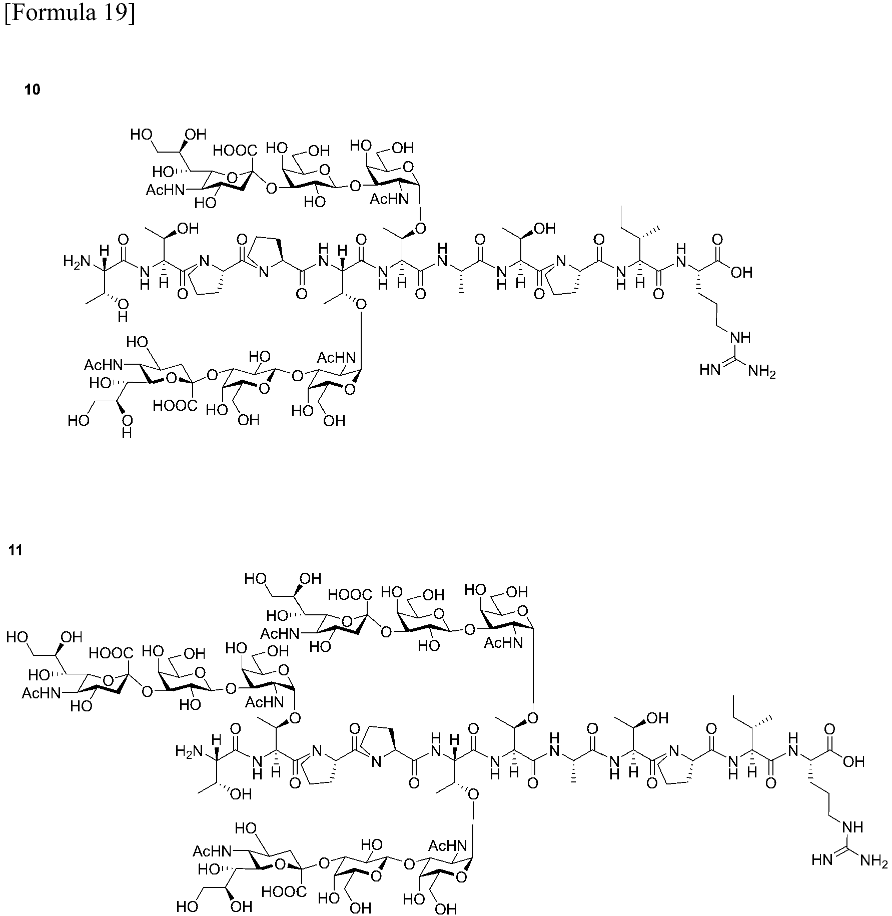

In the present description, the aforementioned Glycopeptides 2 to 21 are also referred to as Compounds 2 to 21 at times. In addition, a peptide having no glycans is referred to as Peptide 1 (Compound 1).

It is to be noted that, in the glycopeptide of the present embodiment, glycan may bind to one, two, or more threonines in the peptide, and that glycan may also bind to amino acids other than threonine. On the other hand, in the glycopeptide of the present embodiment, amino acids other than threonine may be blocked from being introduced with glycans.

The amino acid sequence of the above-mentioned synthetic glycopeptide is identical to the amino acid sequence of the peptide region ²¹⁵¹Thr-Thr-Pro-Pro-Thr-Thr-Ala-Thr-Pro-Ile-Arg²¹⁶¹, which contains consecutive threonines present in the IIICS domain (type III homology connective segment domain) of onfFN (oncofetal fibronectin). The aforementioned O-linked glycan that can be possessed by ²¹⁵¹Thr-Thr-Pro-Pro-Thr-Thr-Ala-Thr-Pro-Ile-Arg²¹⁶¹ in the IIICS domain of onfFN is allowed to bind to the synthetic glycopeptide, so that it becomes possible to track the structural changes of glycans *in vivo.*

In the present embodiment, by using such a synthetic glycopeptide, disease-specific "dynamic epitopes" can be systematically searched and identified. For example, if the glycan structure of a glycopeptide changes due to cell canceration, a glycopeptide having glycan before and after the structural change can be identified and further quantified, so that cancer detection and diagnosis can be carried out. Moreover, if the glycan structure of a glycopeptide changes depending on the stage of cancer progression, a glycopeptide having glycan before and after the structural change can be identified and further quantified, so that the stage of cancer can be specified. Accordingly, the glycopeptide of the present embodiment can be used as a tumor marker (e.g., a biomarker for cancer diagnosis).

It is to be noted that the glycopeptide of the present embodiment cannot only be used to cancer diagnosis, but it can also be used to detect and diagnose diseases in which the glycan structure changes due to post-translational modification in the peptide region ²¹⁵¹Thr-Thr-Pro-Pro-Thr-Thr-Ala-Thr-Pro-Ile-Arg²¹⁶¹ in the IIICS domain of onfFN.

The glycopeptide of the embodiment can also be used as an immunogen or vaccine for antibody production. By using the glycopeptide of the present embodiment, it is also possible to develop a novel therapeutic antibody drug. Since an immunogen for antibody production can be produced by synthesis in the present embodiment, the cost and time required for immunogen production can be significantly suppressed, and thus, it is also possible to efficiently develop antibody drugs against antigen epitopes having clear structures.

Thus, the present inventors have succeeded in synthesizing novel glycopeptides for systematically searching for and identifying epitopes specific to various diseases, including cancer. Furthermore, as described later, by using these synthetic glycopeptides, the present inventors have identified disease-specific dynamic epitopes and have established a method for analyzing diseases using these dynamic epitopes. In particular, these "dynamic epitopes" newly emerge due to specific post-translational glycosylation observed at the spatiotemporal level of a protein during cancer progression (i.e., positions modified and combinations thereof, and differences in the glycan structures, are defined by the stage of disease progression and/or the type of cancer), and these "dynamic epitopes" serve as novel diagnostic biomarkers and at the same time, become target molecules of novel promising therapeutic antibodies. If novel cancer-specific epitopes present in molecules generated by cancer cells can be identified, they will accelerate the development of early cancer detection and diagnosis methods using various advanced instrumental analysis methods, as well as the research and development of novel therapeutic antibody drugs.

### (Glycopeptide-containing material)

The present embodiment may relate to a solution containing the aforementioned glycopeptide. For example, the present embodiment may be a glycopeptide solution containing a predetermined concentration of the aforementioned glycopeptide, wherein the glycopeptide solution may be used to create a calibration curve for cancer analysis, as described later. The glycopeptide solution for creating a calibration curve may contain optional components such as an antioxidant, a preservative and a coloring agent, within the range that does not inhibit the creation of the calibration curve.

In addition, the present embodiment may also relate to a solid body (e.g., a granular or powdery body) containing the aforementioned glycopeptide. The solid body may be obtained, for example, by freeze-drying a solution containing the glycopeptide.

### (Method for producing glycopeptide)

The present embodiment relates to a method for producing the aforementioned glycopeptide. The method for producing the glycopeptide comprises synthesizing a glycopeptide according to solid-phase peptide synthesis. According to the solid-phase peptide synthesis, for example, when TTPPTTATPIR (Thr-Thr-Pro-Pro-Thr-Thr-Thr-Ala-Thr-Pro-Ile-Arg) is synthesized as Peptide 1, the solid-phase synthesis is performed using Fmoc amino acid derivatives as amino acids that constitute Peptide 1, followed by deprotection of the Fmoc groups. The peptide of interest can be synthesized by repeating elongation of the Fmoc amino acids and de-Fmocation.

When a glycopeptide is synthesized, the solid-phase synthesis is performed using Fmoc-threonine having a predetermined glycan. As mentioned above, the solid-phase synthesis using an Fmoc amino acid derivatives allows the introduction of Fmoc-threonine having a predetermined glycan into a predetermined site, thereby synthesizing the glycopeptide of interest.

Examples of the Fmoc-threonine having a predetermined glycan may include the following:

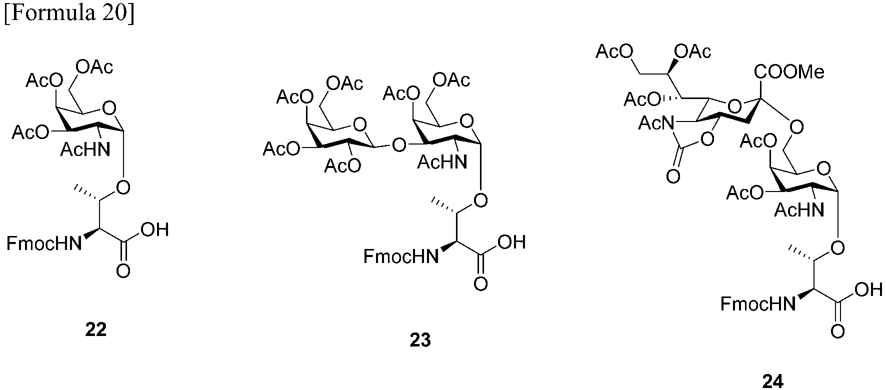

A glycopeptide having an ST antigen (Neu5Acα2,3Galβ1,3GalNAcα1) can be derived from a glycopeptide having a T antigen (Galβ1,3GalNAcα1) (T antigen) by an enzymatic transglycosylation reaction. In the transglycosylation reaction, CMP-sialic acid used as a glycosyl donor is allowed to act on sialyltransferase, so that the sialic acid is allowed to bind to the Gal that is present on the non-reducing terminal side of the glycan, so that the glycan can be elongated. Examples of the sialyltransferase may include α2,8-sialyltransferase and α-2,3-sialyltransferase, with α-2,3-sialyltransferase being preferable. As such sialyltransferase, commercially available products can also be used, and the commercially available sialyltransferase may be, for example, the recombinant α-2,3-sialyltransferase derived from Pasteurella multocida, manufactured by Sigma-Aldrich. The reaction temperature for the transglycosylation reaction is, for example, preferably 15°C to 60°C, and the reaction time is preferably approximately 10 minutes to 100 hours.

For glycopeptides containing the ST antigen (Neu5Acα2,3Galβ1,3GalNAcα1), it is preferable to deprotect the acetyl groups in the glycan region after termination of the solid-phase synthesis. For example, the acetyl groups may be deprotected by dissolving the glycopeptide obtained by freeze-drying in methanol, and 1 N sodium hydroxide aqueous solution is then added thereto, followed by shaking the mixture at room temperature.

It is to be noted that the glycopeptide having the ST antigen (Neu5Acα2,3Galβ1,3GalNAcα1) can also be obtained by performing solid-phase synthesis using Fmoc-threonine. For example, Fmoc-Thr (Neu5Aca2,3Galβ1,3GalNAcα1)-OH can be used as such Fmoc-threonine. In this case, synthesis can be performed with reference to S. Dziadek, et al., Chem. Eur. J. 2004, 10, 4150-4162, etc.

The glycopeptide synthesized by the above-described solid-phase synthesis may be purified, as needed. For example, a glycopeptide purified by reversed-phase high-performance liquid chromatography can also be obtained.

### (Method for analyzing glycopeptide)

The present embodiment relates to an analysis method for examining whether or not the aforementioned glycopeptide is contained in the serum or the plasma. Specifically, the method for analyzing a glycopeptide of the present embodiment comprises analyzing whether or not a glycopeptide is contained in a sample containing a glycopeptide obtained by protease digestion of the serum or the plasma. The concerned glycopeptide is the aforementioned glycopeptide, and it is a peptide consisting of the amino acid sequence TTPPTTATPIR, in which one glycan consisting of 6 or less sugars binds to each of one or two or more threonines, and the glycan is an O-linked glycan. In addition, the protease used in the method for analyzing a glycopeptide of the present embodiment may be, for example, chymotrypsin or trypsin, and of these, trypsin is preferable.

By digesting the serum or plasma with protease, a peptide consisting of the amino acid sequence TTPPTTATPIR is obtained from the IIICS domain of onfFN contained in the serum or plasma. In the peptide consisting of the above-described amino acid sequence, one glycan consisting of 6 or less sugars (O-linked glycan) binds to each of one or two or more threonines. The present embodiment relates to a method for analyzing the type and amount of a glycopeptide contained in the protease digest of the serum or plasma. In particular, the present embodiment preferably relates to a method for analyzing the type and amount of a glycopeptide contained in the trypsin digest of the serum.

The analysis method of the present embodiment preferably comprises analyzing whether or not one type, or two or more types selected from the group consisting of Glycopeptide 5, Glycopeptide 8, Glycopeptide 9, Glycopeptide 10, Glycopeptide 15, Glycopeptide 16 and Glycopeptide 20, in the sample containing a glycopeptide obtained by protease digestion of the serum or the plasma. The analysis method of the present embodiment particularly preferably comprises performing the mass spectrometry on each of Glycopeptide 8, Glycopeptide 9, Glycopeptide 10, Glycopeptide 15 and Glycopeptide 20 in the sample (the protease digest of the serum or the plasma) containing a glycopeptide obtained by protease digestion of the serum or the plasma. It is to be noted that the structures of Glycopeptide 8, Glycopeptide 9, Glycopeptide 10, Glycopeptide 15 and Glycopeptide 20 are the following:

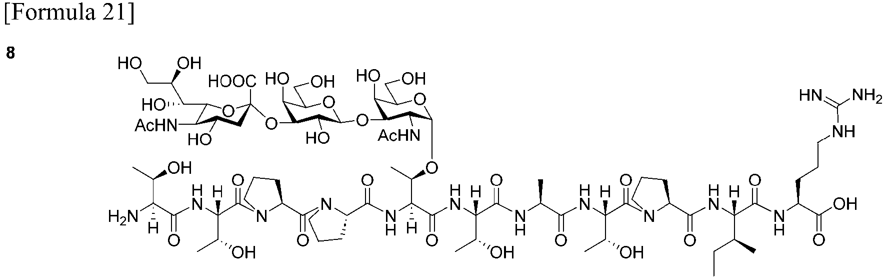

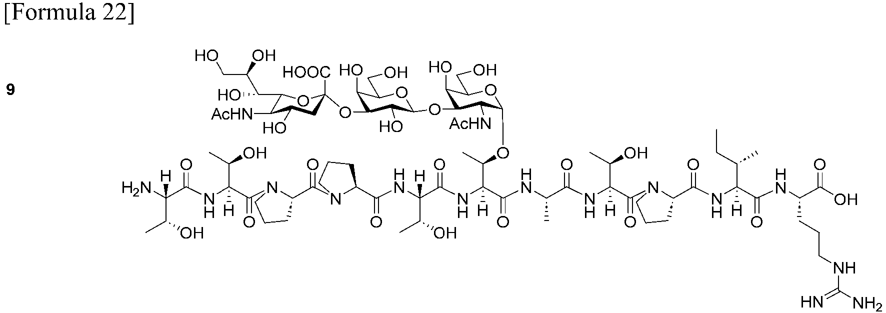

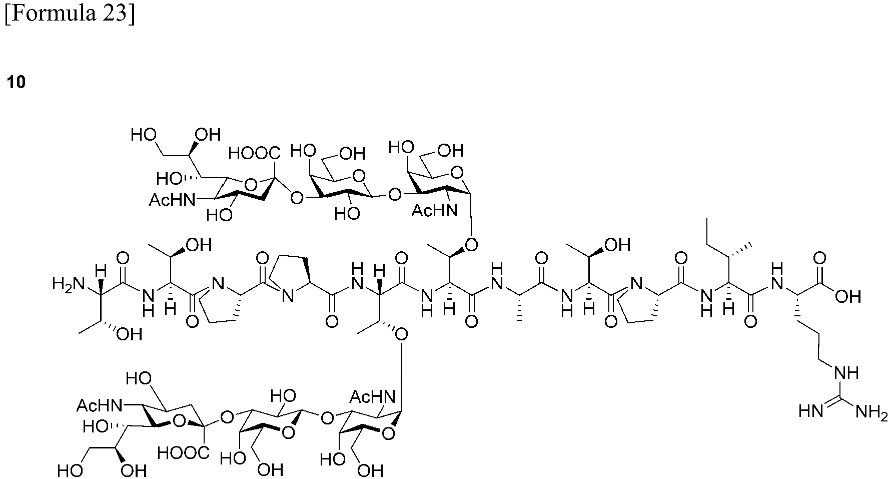

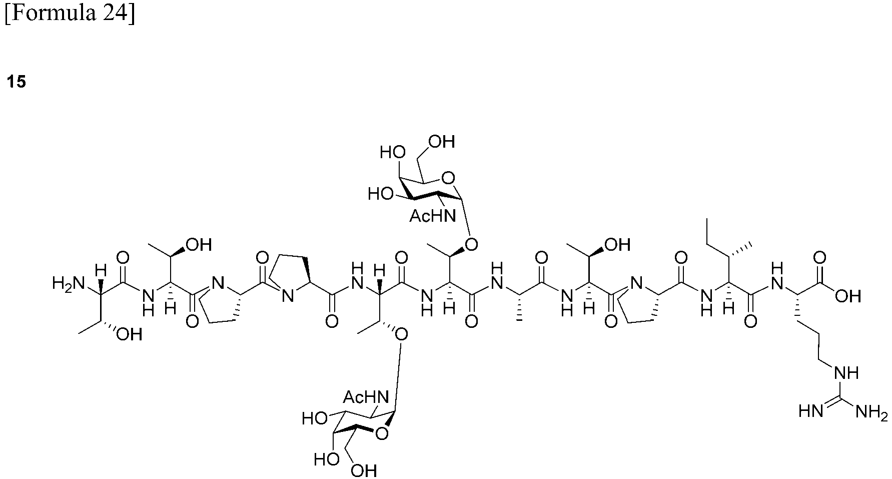

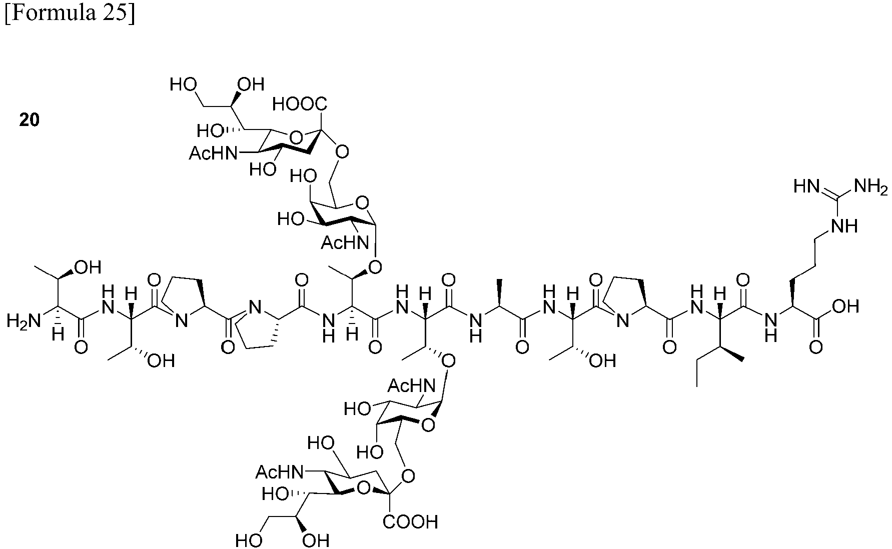

Moreover, in the case of liver cancer patients, the analysis method of the present embodiment preferably comprises further analyzing whether or not one type, or two or more types selected from the group consisting of Glycopeptide 5, Glycopeptide 9, Glycopeptide 10, Glycopeptide 15, Glycopeptide 16 and Glycopeptide 20, are contained in the sample (the protease digest of the serum or the plasma) containing a glycopeptide obtained by protease digestion of the serum or the plasma, and the analysis method of the present embodiment more preferably comprises further analyzing whether or not one or two types selected from the group consisting of Glycopeptide 15 and Glycopeptide 20, are contained in the sample. The analysis method of the present embodiment preferably comprises performing mass spectrometry on each of Glycopeptide 5, Glycopeptide 9, Glycopeptide 10, Glycopeptide 15, Glycopeptide 16 and Glycopeptide 20 in the sample (the protease digest of the serum or the plasma) containing a glycopeptide obtained by protease digestion of the serum or the plasma, and the analysis method of the present embodiment particularly preferably comprises performing mass spectrometry on each of Glycopeptide 15 and Glycopeptide 20.

Furthermore, in the case of pancreatic cancer patients, the analysis method of the present embodiment preferably comprises analyzing whether or not one type, or two or more types selected from the group consisting of Glycopeptide 5, Glycopeptide 8, Glycopeptide 9, Glycopeptide 10, Glycopeptide 11 and Glycopeptide 16, are contained in the sample (the trypsin digest of the serum or the plasma) containing a glycopeptide obtained by trypsin digestion of the serum or the plasma, and the analysis method of the present embodiment more preferably comprises analyzing whether or not one type, or two or more types selected from the group consisting of Glycopeptide 8, Glycopeptide 9 and Glycopeptide 10, are contained in the sample. The analysis method of the present embodiment preferably comprises performing mass spectrometry on each of Glycopeptide 5, Glycopeptide 8, Glycopeptide 9, Glycopeptide 10, Glycopeptide 11 and Glycopeptide 16 in the sample (the protease digest of the serum or the plasma) containing a glycopeptide obtained by protease digestion of the serum or the plasma, and the analysis method of the present embodiment particularly preferably comprises performing mass spectrometry on each of Glycopeptide 8, Glycopeptide 9 and Glycopeptide 10.

In the present embodiment, it is preferable to remove a portion of the serum protein or plasma protein from the serum or plasma before subjecting the serum or plasma to protease digestion. Specifically, it is preferable to remove the top 14 major serum or plasma proteins with high blood concentrations. The removal of serum or plasma proteins can be performed using known methods, and for example, the multiple-affinity removal system ("MARS," Agilent Technologies spin columns) can be used. By removing a portion of the serum protein or plasma protein, protease digestion can be efficiently carried out, and the accuracy of mass spectrometry of the target glycopeptide can be enhanced.

When the mass spectrometry of a glycopeptide is performed, it is preferable to adopt tandem mass spectrometry (selective reaction monitoring method: SRM) as an analysis method. By using the tandem mass spectrometry (selective reaction monitoring method: SRM), quantitative analysis of a glycopeptide can be performed quickly and accurately. Herein, SRM (selective reaction monitoring) parameters of glycopeptides can be optimized according to the following procedures, using a system consisting of an LC-MS/MS (4000 QTRAP triple quadrupole mass spectrometer (AB Sciex Pte. Ltd.)) and an Eksigent NanoLC Expert^{™} 400 (AB Sciex Pte. Ltd.).

Specifically,
(a) parent ion selection (Q1) in the first quadrupole,
(b) optimization (Q2) of collision-induced dissociation (CID) in the second quadrupole, and
(c) evaluation and selection (Q3) of fragment ions specific to individual glycopeptides in the third quadrupole,
are carried out, and the SRM parameters for each glycopeptide are determined (e.g., S.-I. Nishimura, et al., Selective reaction monitoring approach using structure-defined synthetic glycopeptides for validating glycopeptide biomarkers pre-determined by bottom-up glycoproteomics. RSC Adv 2022, 12, 21385-21393).

Herein, the quadrupole mass spectrometer adjusts the radio frequency (RF) voltage and the direct current (DC) voltage, so that four parallel rod-shaped electrodes function as mass filters that selectively pass only ions in a specific mass region. Therefore, once the target ion Q1 and the fragment ion Q3 are determined, the voltages may be set so that only these ions pass through each filter. At that time, four parameters, namely, delustering potential (DP), entrance potential (EP), collision energy (CE), and collision cell exit potential (CXP), need to be optimized. DP is the voltage used to guide all ions generated by the ion generator to Q0, EP is the voltage used to guide the ions from Q0 to Q1, CE is the voltage used to fragment target ions, and CXP is the voltage used to guide the generated fragment ions into Q3. These voltages are adjusted and optimized to maximize detection sensitivity. Of the four parameters, CE has the greatest impact on the sensitivity of the parent ions and the fragment ions.

As mentioned above, LC-coupled triple quadrupole tandem mass spectrometers are used in quantitative proteomics research according to multiple reaction monitoring (MRM), but there have been no known cases of successful quantitative analysis of glycopeptides using MRM/SRM. The method for analyzing a glycopeptide of the present embodiment is the world's first rapid and precise diagnostic technique using MRM/SRM, which can determine the stage of cancer progression by quantitatively analyzing specific glycopeptide fragments in the protease digest of blood proteins.

In the analysis method of the present embodiment, first, after the above-mentioned parameters have been determined for the above-mentioned synthetic Glycopeptides 2 to 21, mass spectrometry is performed and a calibration curve for each glycopeptide is prepared. Thereafter, mass spectrometry is performed on Glycopeptides 2 to 21 contained in samples obtained by protease digestion of the serum or plasma from liver cancer patients and healthy subjects, and the content (concentration) of each glycopeptide contained in the serum or plasma is calculated from the calibration curve obtained above. By performing this type of analysis, the present inventors have found that the concentrations of Glycopeptides 15 and 20 are significantly increased from stage 2 onwards in the liver cancer patients. On the other hand, the present inventors have also found that the concentrations of Glycopeptide 5, Glycopeptide 9, Glycopeptide 10 and Glycopeptide 16 are high in healthy subjects or stage 1 liver cancer patients, and that the concentrations are significantly decreased, as the stage progresses. More specifically, it has been found that the concentration of Glycopeptide 9 is high in healthy subjects, and that its concentration is significantly decreased as the cancer progresses after stage 1. Moreover, it has been found that the concentrations of Glycopeptide 5, Glycopeptide 10, and Glycopeptide 16 are high in stage 1 liver cancer patients, and that the concentrations are significantly decreased as the cancer progresses after stage 2. Based on these findings, whether or not Glycopeptide 15 and/or Glycopeptide 20 are contained in a sample containing a glycopeptide obtained by protease digestion of the serum or the plasma (the protease digest of the serum or the plasma) is analyzed, and mass spectrometry is further performed on Glycopeptide 15 and/or Glycopeptide 20. If high concentrations of Glycopeptides 15 and 20 are detected, it can be determined that liver cancer has progressed. On the other hand, when the concentrations of Glycopeptide 5, Glycopeptide 9, Glycopeptide 10, and/or Glycopeptide 16 are detected at low levels or these glycopeptides are not detected in a sample containing a glycopeptide obtained by protease digestion of the serum or the plasma (the protease digest of the serum or the plasma), it can be determined that liver cancer has progressed or that the patient may be affected with a disease including liver cancer. Furthermore, when the concentrations of Glycopeptides 15 and 20 are significantly increased and the concentrations of Glycopeptide 5, Glycopeptide 9, Glycopeptide 10, and Glycopeptide 16 are significantly decreased, it can be more accurately determined that liver cancer has progressed, and it can also be determined that the liver cancer is at least stage 1 or 2 or later. In this way, the analysis method of the present embodiment can identify glycopeptides whose content changes depending on the progression of a specific disease, and after identifying the glycopeptides involved in the disease, detecting changes in their content can be used to determine the presence or absence of a disease and its progression.

Furthermore, mass spectrometry was performed on Glycopeptides 2 to 21 contained in samples obtained by protease digestion of the serum or plasma from pancreatic cancer patients and healthy subjects. The content (concentration) of each glycopeptide contained in the serum or plasma is calculated from the calibration curve obtained above. As a result, it has been found that the concentrations of Glycopeptide 8, Glycopeptide 9, and Glycopeptide 10 have been significantly increased in the serum from stage 4 pancreatic cancer patients. On the other hand, the present inventors have found that, while the concentrations of Glycopeptide 5, Glycopeptide 11, and Glycopeptide 16 are high in healthy subjects, they are significantly decreased in stage 4 pancreatic cancer patients.

In particular, the analysis method of the present embodiment is useful as a method for analyzing cancer, and the present embodiment also relates to a method for analyzing cancer including the above-described method for analyzing a glycopeptide. The method for analyzing cancer of the present embodiment includes both detection of the presence or absence of cancer and determination of the stage of cancer progression.

The method for analyzing cancer of the present embodiment preferably comprises the following series of steps:
(1) performing mass spectrometry on synthetic glycopeptides and preparing a calibration curve for each glycopeptide;
(2) obtaining the target serum or plasma and removing a portion of the serum or plasma protein from the obtained serum or plasma;
(3) performing protease digestion on the serum or plasma, from which a portion of the serum or plasma protein has been removed, to prepare a measurement sample;
(4) performing mass spectrometry on the glycopeptide contained in the sample and calculating the concentration of the glycopeptide contained in the serum or plasma from the calibration curve; and
(5) determining the onset and progression of cancer based on the concentration of a glycopeptide, the content of which changes depending on the onset and progression of the cancer.

The present embodiment may also relate to a system for determining the onset and progression level of cancer, in which the above-described steps (1) to (5) are carried out.

The cancers to be analyzed are not particularly limited, and examples thereof may include liver cancer, stomach cancer, colon cancer, gallbladder cancer, esophageal cancer, pharyngeal cancer, pancreatic cancer, breast cancer, lung cancer, melanoma, uterine cancer, osteosarcoma, skin cancer, ovarian cancer, and kidney cancer. Of these, the cancers to be analyzed are preferably cancers of the digestive system, and the analysis method of the present embodiment is suitable for detection of digestive system cancer and/or detection of the stage of digestive system cancer progression. In particular, the cancers to be analyzed are preferably liver cancer and/or pancreatic cancer, with liver cancer being particularly preferable.

The present inventors have found that a glycan structure is changed by post-translational modification in the peptide region ²¹⁵¹Thr-Thr-Pro-Pro-Thr-Thr-Ala-Thr-Pro-Ile-Arg²¹⁶¹ including consecutive threonines which is present in the IIICS domain (type III homology connective segment domain) of onfFN (oncofetal fibronectin). In addition, the present inventors have discovered glycopeptides (Glycopeptides 15 and 20, and Glycopeptides 5, 9, 10, and 16) acting as specific "dynamic epitopes" that sensitively reflect "the stage of cancer progression" within the molecules of fibronectin, a glycoprotein having diverse functions regarding regulation of cancer cell adhesion, growth, invasion, and metastasis, among the aforementioned synthetic glycopeptides. Since it has been quantitatively proved that the blood concentrations of fibronectin containing the dynamic epitopes generated by such cancer-specific glycosylation drastically changes after a specific early stage in liver cancer patient, a novel technique capable of early detection of cancer and/or precise (quantitative) diagnosis of the cancer progression, which is used as a novel, unprecedent and ultrasensitive biomarker, has been realized. These glycopeptides are useful not only as immunogens for antibody production, but also as cancer vaccines.

Moreover, another embodiment relates to a method for analyzing a glycopeptide, comprising, in the case of liver cancer, analyzing whether or not one type, or two or more types selected from the group consisting of Glycopeptide 5, Glycopeptide 9, Glycopeptide 10, Glycopeptide 11 and Glycopeptide 16, are contained in a sample containing a glycopeptide obtained by protease digestion of the serum or the plasma (the protease digest of the serum or the plasma). Furthermore, another embodiment relates to a method for analyzing a glycopeptide, comprising, in the case of pancreatic cancer, analyzing whether or not one type, or two or more types selected from the group consisting of Glycopeptide 5, Glycopeptide 8, Glycopeptide 9, Glycopeptide 10, Glycopeptide 11 and Glycopeptide 16, are contained in a sample containing a glycopeptide obtained by protease digestion of the serum or the plasma (the protease digest of the serum or the plasma).

For example, according to the present analysis methods, it also becomes possible to confirm the absence of cancer or to evaluate the therapeutic effects of cancer, by confirming that a sample containing a glycopeptide obtained by protease digestion of the serum or the plasma (the protease digest of the serum or the plasma) contains one type, or two or more types selected from the group consisting of Glycopeptide 5, Glycopeptide 9, Glycopeptide 10, Glycopeptide 11 and Glycopeptide 16.

### Examples

The characteristics of the present invention will be more specifically described in the examples described below. In the following examples, the materials used, the amounts, ratios, the details of the treatments, the treatment procedures, etc. may be modified, as appropriate, unless they overstep the spirit and the scope of the invention. Accordingly, the invention should not be restrictively interpreted by the following specific examples.

In the Examples described below, threonine (T2) located at position 2 from the N-terminal side is also referred to as Thr2152; threonine (T5) located at position 5 from the N-terminal side is also referred to as Thr2155; and threonine (T6) located at position 6 from the N-terminal side is also referred to as Thr2156.

### (Examples)

### < Synthesis of Peptide 1 >

Peptide 1 was synthesized by Fmoc solid-phase peptide synthesis. Specifically, using Trityl-OH-ChemMatrix resin (0.41 mmole/g, 25 mg, 10.25 µmol), the reaction was sequentially proceeded each step lasting 9 minutes in a DMF (103 µL) solution consisting of Fmoc amino acid derivative (41.0 µmol, 4 eq.), HBTU (41.0 µmol, 4 eq.), HOBt (41.0 µmol, 4 eq.), and DIEA (61.5 µmol, 6 eq.) under microwave irradiation (50°C). The Fmoc group was deprotected by treating under microwave irradiation (50°C) for 3 minutes in 20% piperidine/DMF. The Peptide 1 of interest was synthesized by repeating these two steps: elongation of various Fmoc amino acids and de-Fmocation.

### < Synthesis of Glycopeptides 2 to 21 >

Fmoc glycoamino acid derivatives having glycans represented by the following structures were prepared. Glycoamino Acid 24 was synthesized with reference to L. Corcilius, R. J. Payne, Org. Lett. 2013, 15, 5794-5797; D. Liu, et al. J. Am. Chem. Soc. 2021, 143, 20216-20223.

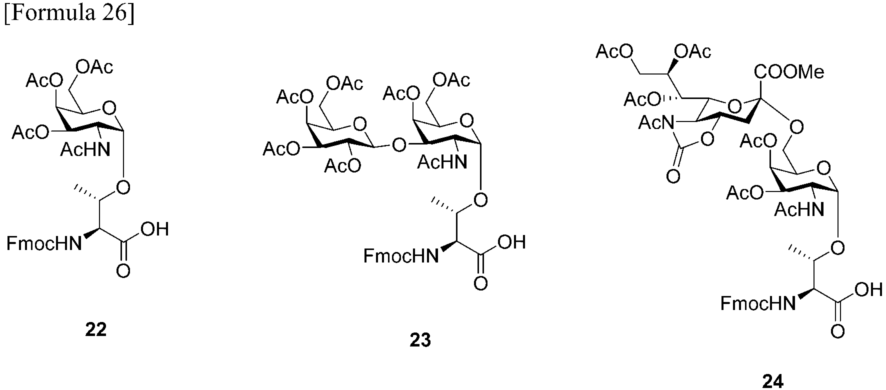

When glycosylation is performed on threonine (Thr2152) at position 2 from the N-terminal side, threonine (Thr2155) at position 5 from the N-terminal side, threonine (Thr2156) at position 6 from the N-terminal side, in Glycopeptides 2 to 21, the Fmoc glycoamino acid derivatives having the above-mentioned glycans were condensed, instead of the usual Fmoc amino acid derivatives, under the following conditions [microwave irradiation at 50°C for 9 minutes in DMF (51 µL) consisting of Fmoc glycoamino acid derivative (41.0 µmol, 2 eq.), HBTU (41.0 µmol, 2 eq.), HOBt (41.0 µmol, 2 eq.), and DIEA (61.5 µmol, 4 eq.)]. After condensation of all amino acid and glycoamino acid residues, TFA : H₂O : TIS (v : v : v = 95 : 2.5 : 2.5) were added to the reaction system, and the obtained mixture was then shaken at room temperature for 2 hours. Thereafter, the reaction solution was filtrated, and a crude product was then precipitated by adding *tert*-butylmethyl ether (20 mL), followed by performing centrifugation (7,000 x g, 4°C, 20 minutes) to remove the supernatant. The precipitate was dissolved in a 50% acetonitrile aqueous solution, and was then freeze-dried to obtain glycopeptide intermediates. Of these freeze-dried products, Glycopeptide Intermediates 2 to 6 and 12 to 16, which did not contain sialic acid residues, were dissolved in methanol (5 mL). Subsequently, the pH of the solution was adjusted to pH 12.5 by addition of a 1 N sodium hydroxide aqueous solution, and the thus obtained solution was then shaken at room temperature for 2 hours to deprotect the acetyl groups in the glycan region. The reaction solution was neutralized with a 30% acetic acid aqueous solution. Thereafter, the solvent was distilled away, and the resulting syrupy residue was then purified by reversed-phase high-performance liquid chromatography to obtain Glycopeptides 2 to 6 and 12 to 16 of interest.

Glycopeptides 7 to 11 each containing the ST antigen (Neu5Acα2,3Galβ1,3GalNAcα1) were induced from Glycopeptides 2 to 6 by enzymatic transglycosylation reaction. Specifically, a reaction was carried out at 37°C for 48 hours (Glycopeptides 2 to 4) or for 96 hours (Glycopeptide 5) in a reaction system, in which Glycopeptides 2 to 5 (5 mM), *Pasteurella multocida*-derived recombinant α-2,3-sialyltransferase (100 mU/mL), and CMP-Neu5Ac (30 mM) were dissolved in 100 mM HEPES (pH 7.0, 20 mM MgCl2, 2% BSA) at the final concentrations shown in the parentheses. Thereafter, the obtained mixture was directly purified by reverse-phase high-performance liquid chromatography to obtain Glycopeptides 7 to 10 of interest. Glycopeptide 11 was obtained by performing a reaction at room temperature for 24 hours in a reaction system, in which Glycopeptide 6 (1 mM), rat-derived recombinant α-2,3-sialyltransferase (20 mU/mL), and CMP-Neu5Ac (5 mM) were dissolved in 50 mM HEPES (pH 7.0, 10 mM MgCl2, 0.1% BSA) at the final concentrations shown in the parentheses, followed by direct purification of the mixture by reversed-phase high-performance liquid chromatography in the same manner as described above (Nishimura, S.-I. et al., Convergent solid-phase synthesis of macromolecular MUC1 models truly mimicking serum glycoprotein biomarkers of interstitial lung diseases. J. Am. Chem. Soc. 2016, 138, 8392-8395).

With regard to Glycopeptides 17 to 21 each containing the STn antigen (Neu5Acα2,6GalNAcα1), glycopeptide intermediates obtained by freeze-drying after termination of solid-phase synthesis were dissolved in methanol (4 mL), and the pH of the solution was then adjusted to pH 11.5 by addition of a 1 N sodium hydroxide solution aqueous solution, and the solution was then shaken at room temperature for 2 hours to deprotect the acetyl groups in the glycan region. Subsequently, the reaction solution was adjusted to pH 12 by addition of a 50 mM NaOH aqueous solution to the reaction system, and the resulting solution was further shaken for additional 5 hours to hydrolyze the methyl groups. The reaction solution was neutralized with a 30% acetic acid aqueous solution. Thereafter, the solvent was distilled away, and the resulting syrupy residue was purified by reversed-phase high-performance liquid chromatography to obtain Glycopeptides 17 to 21 of interest.

Hereafter, the synthetic schemes for Glycopeptides 11 and 21 will be shown as representative schemes.

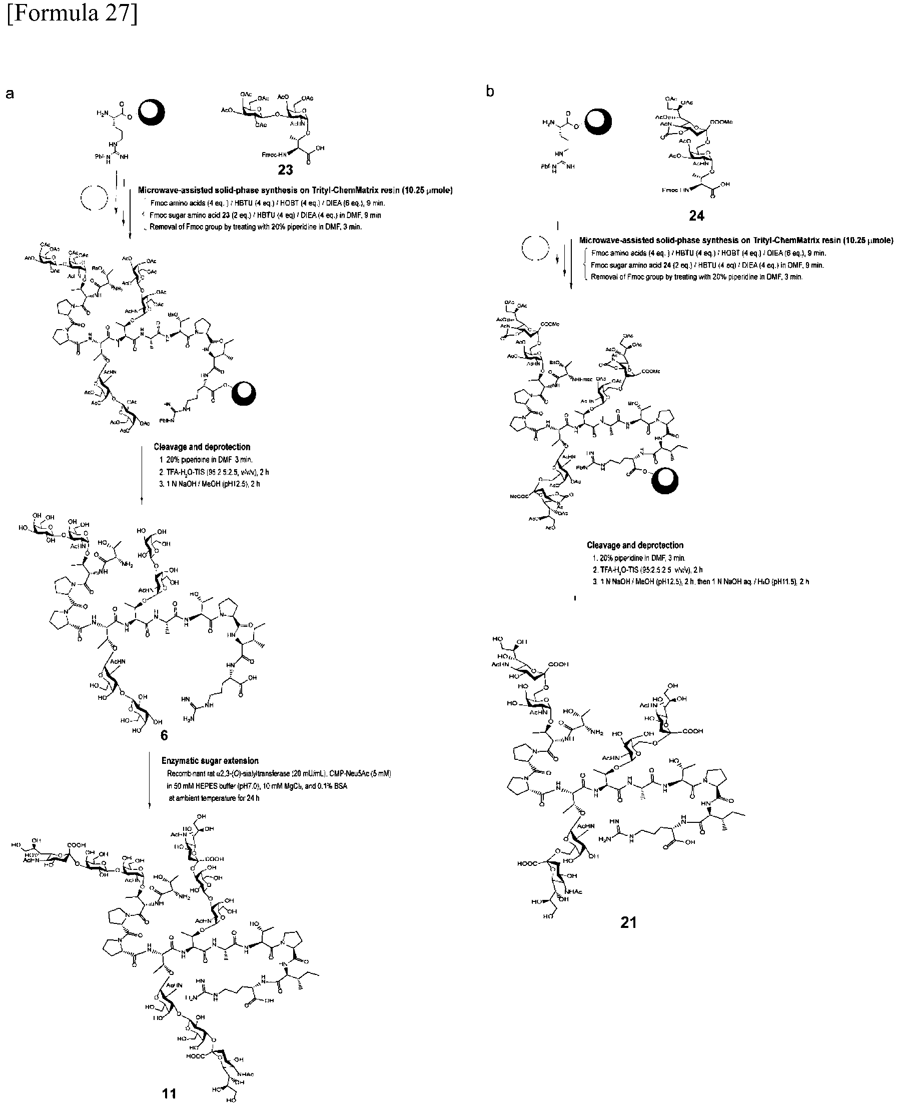

### < Reversed-phase high-performance liquid chromatography (RP-HPLC) and mass spectrometry (MALDI-TOFMS) of Peptide 1 and Glycopeptides 2 to 21 >

Separation and analysis of glycopeptides by RP-HPLC were performed using a system consisting of reversed-phase C18 Inertsil ODS-3 column (250 x 4.6 mm I.D.; GL Sciences Inc., Tokyo, Japan), a Hitachi L-7100 intelligent pump, and an L-7405 UV detector. Specifically, elution was carried out by a gradient program, in which a solution (A/B = 95/5) consisting of eluent A (0.1% trifluoroacetic acid aqueous solution) and eluent B (acetonitrile containing 0.1% trifluoroacetic acid) was run at a flow rate of 1.0 mL/min for 50 minutes, followed by a gradient in which the proportion of eluent B increased from 5% to 30% over 50 min [0 min: A/B = 95/5 → 50 min: A/B = 70/30].

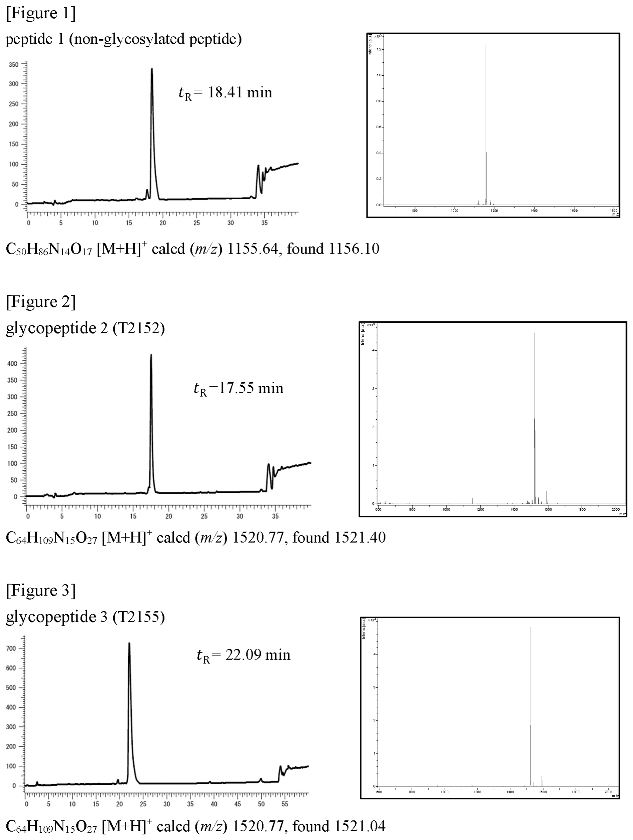

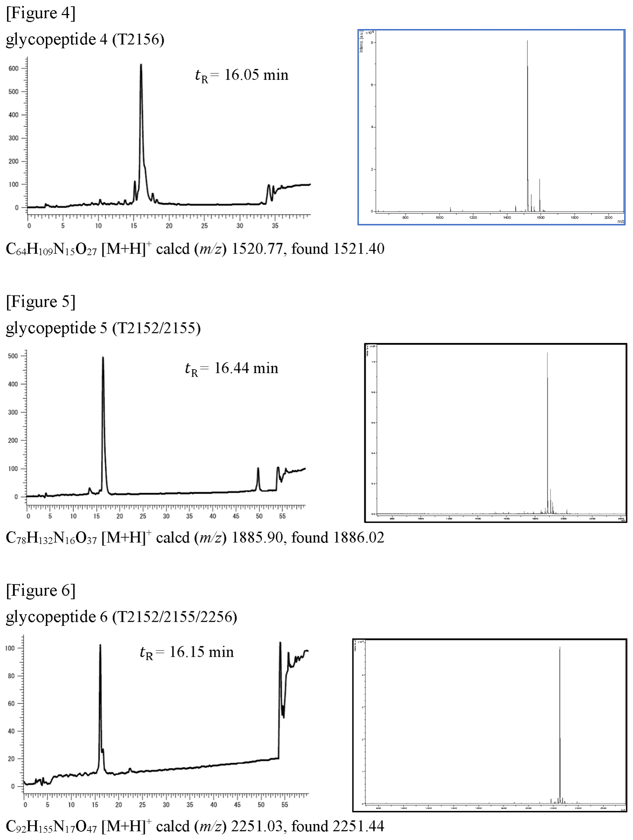

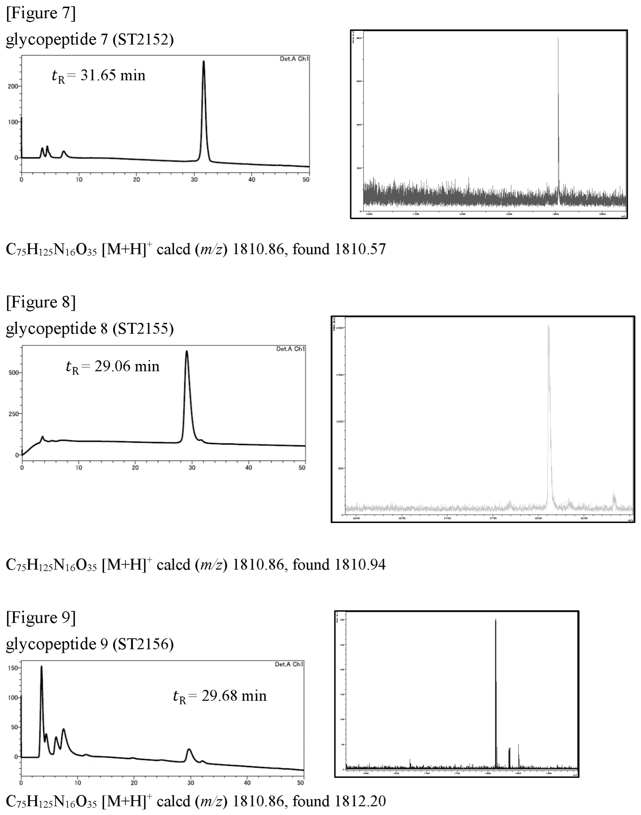

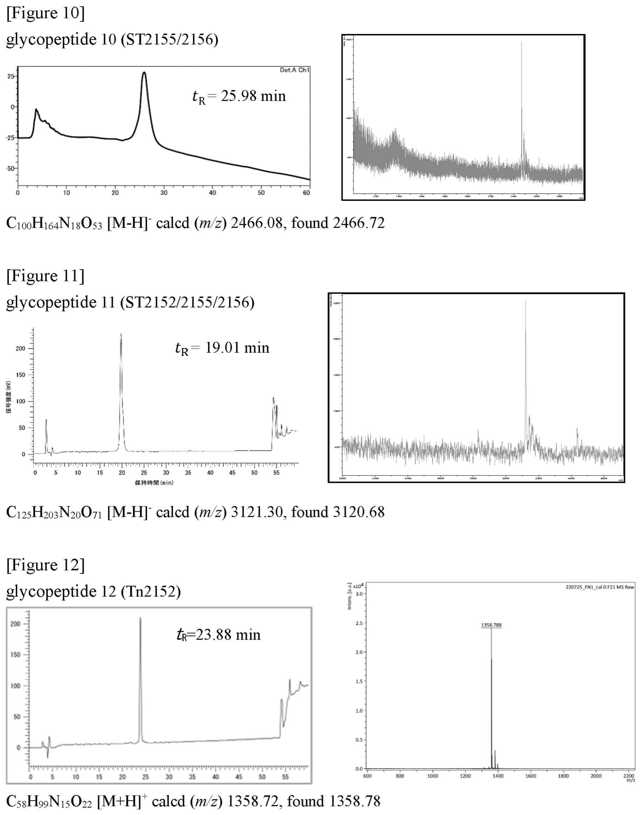

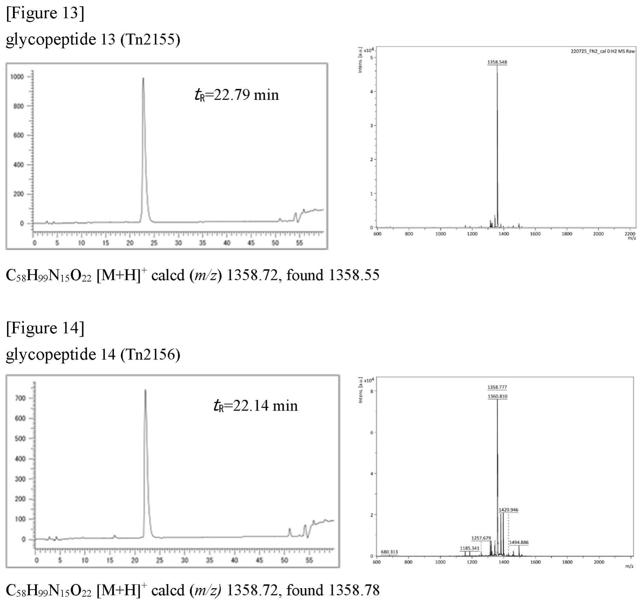

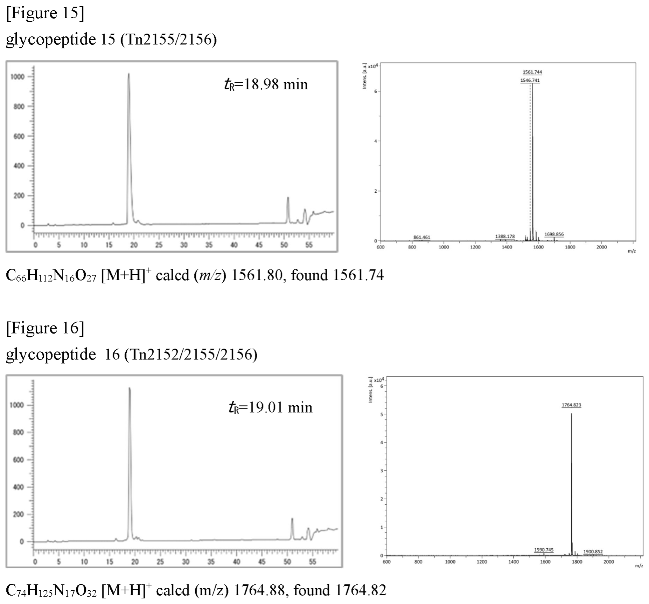

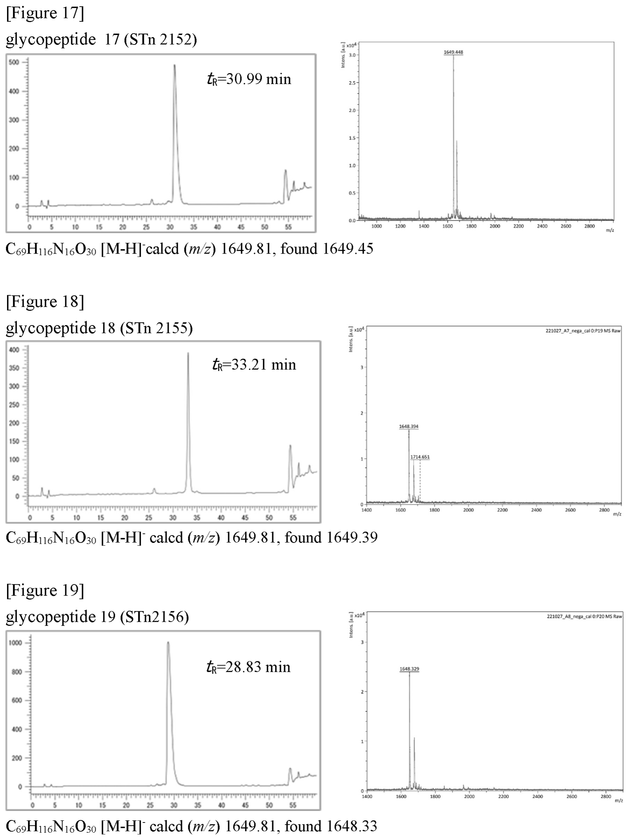

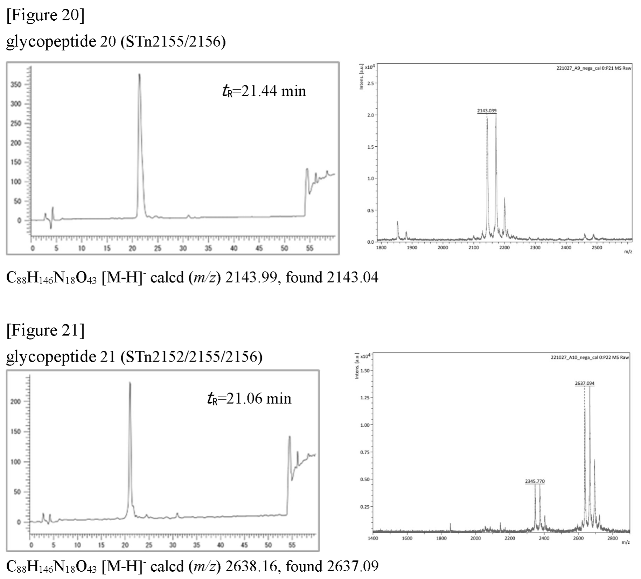

The molecular weights of the glycopeptides were measured by MALDI-TOF/MS using a Bruker Ultraflex III mass spectrometer. The spectrometer was operated in reflector positive and negative mode using 2,5-dihydrobenzoic acid (DHB) as the matrix. The results of the mass spectrometry by MALDI-TOF/MS for Peptide 1 and Glycopeptides 2 to 21 are shown in Figures 1 to 21.

### < Setting of SRM channel for Peptide 1 and Glycopeptides 2 to 21 >

The SRM (selective reaction monitoring) parameters for Peptide 1 and Glycopeptides 2 to 21 were optimized and determined by the following procedures using a system consisting of LC-MS/MS(4000 QTRAP triple quadrupole mass spectrometer(AB Sciex Pte. Ltd.)) and Eksigent NanoLC Ekspart^{™} 400 (AB Sciex Pte. Ltd.).

Specifically,
(a) parent ion selection (Q1) in the first quadrupole,
(b) optimization (Q2) of collision-induced dissociation (CID) in the second quadrupole, and
(c) evaluation and selection (Q3) of fragment ions specific to individual glycopeptides in the third quadrupole,
were carried out, and the SRM parameters for each glycopeptide were determined (e.g., S.-I. Nishimura, et al., Selective reaction monitoring approach using structure-defined synthetic glycopeptides for validating glycopeptide biomarkers pre-determined by bottom-up glycoproteomics. RSC Adv 2022, 12, 21385-008121393).

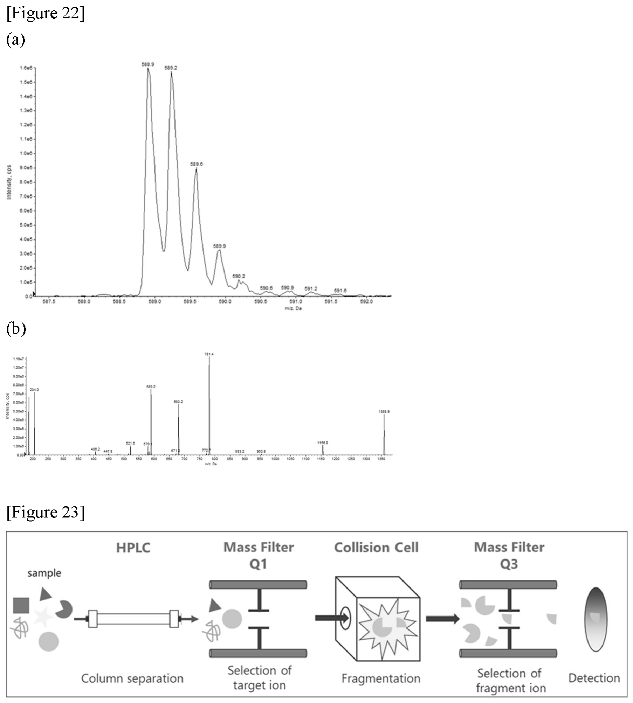

The step of optimizing SRM parameters is shown below, using Glycopeptide 16 as an example. By using Enhanced Mass (EMS) mode in combination with Enhanced Resolution (ER) mode, accurate Q1 and further, the charge number of Q1, were determined. For example, since the parent ion of Glycopeptide 16 (2152/2155/2156Tn) has multiple isotopomers centered at *m*/*z* 589.3 (theoretical value: *m*/*z* 589.30) observed at intervals of 0.3 Da, this ion is demonstrated to be a triply charged ion (Figure 22(a)) shows the actually obtained spectrum of Glycopeptide 16 in ER mode). Furthermore, in Q2, fragment ions generated by CID that gradually changed the collision energy (CE) from 25 volts (0.8 min) to 130 volts (5 min) in Enhanced Product Ion (EPI) mode were observed, and Q3 was optimized (Figure 22(b)). Six fragment ions were identified for the Q3 candidate of Glycopeptide 16: *m*/*z* 186.0 ([dehydrated GalNAc+H]⁺), 204.0 ([GalNAc+H]⁺), 680.2 ([M-2GalNAc+2H]²⁺), 781.4 ([M-GalNAc+2H]²⁺), 1156.0 ([Naked+H]⁺), and 1358.9 ([M-2GalNAc+H]⁺).

Herein, the quadrupole mass spectrometer adjusts the radio frequency (RF) voltage and the direct current (DC) voltage, so that four parallel rod-shaped electrodes function as mass filters that selectively pass only ions in a specific mass region. Therefore, once the target ion Q1 and the fragment ion Q3 are determined, the voltages may be set so that only these ions pass through each filter. At that time, four parameters, namely, delustering potential (DP), entrance potential (EP), collision energy (CE), and collision cell exit potential (CXP), need to be optimized. DP is the voltage used to guide all ions generated by the ion generator to Q0, EP is the voltage used to guide the ions from Q0 to Q1, CE is the voltage used to fragment target ions, and CXP is the voltage used to guide the generated fragment ions into Q3. These voltages are adjusted and optimized to maximize detection sensitivity. Naturally, of the four parameters, CE has the greatest impact on the sensitivity of parent ions and fragment ions (Figure 23).

The SRM parameters of Peptide 1 and Glycopeptides 2 to 21 determined by the above-mentioned method are as follows.

**[Table 1-1]**

| Compound | Q1 | Q3 | Structure | DP | EP | CE | CXP |
|---|---|---|---|---|---|---|---|
| Non-glycosylated (Peptide 1) | 578.3 | 385.3 | *1 | 106 | 10 | 47 | 10 |
| | 578.3 | 477.5 | *2 | 81 | 10 | 25 | 12 |
| | 578.3 | 486.5 | *3 | 81 | 10 | 41 | 6 |
| | 578.3 | 557.5 | *4 | 91 | 10 | 39 | 16 |
| T2152 (Glycopeptide 2) | 760.9 | 204.1 | 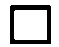 | 111 | 10 | 37 | 17 |
| | 760.9 | 366.4 | 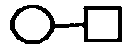 | 111 | 10 | 28 | 10 |
| | 760.9 | 1155.7 | pep | 116 | 10 | 31 | 40 |
| T2155 (Glycopeptide 3) | 761 | 204.1 | 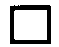 | 91 | 10 | 41 | 10 |
| | 761 | 366.2 | 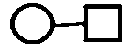 | 96 | 10 | 31 | 28 |
| | 761 | 1155.7 | pep | 106 | 10 | 32 | 30 |
| T2156 (Glycopeptide 4) | 760.9 | 204.2 | 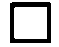 | 76 | 10 | 37 | 10 |
| | 760.9 | 366.2 | 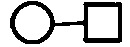 | 81 | 10 | 31 | 21 |
| | 760.9 | 1155.71 | pep | 96 | 10 | 39 | 14 |
| T2155/2156 (Glycopeptide 5) | 943.4 | 204.1 | 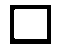 | 136 | 10 | 49 | 12 |
| | 943.4 | 366.1 | 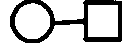 | 141 | 10 | 37 | 12 |
| | 943.4 | 1155.8 | pep | 106 | 10 | 47 | 42 |
| T2152/2155/2156 (Glycopeptide 6) | 1126.1 | 204.1 | 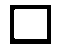 | 136 | 10 | 63 | 16 |
| | 1126.1 | 366.4 | 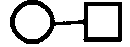 | 131 | 10 | 43 | 12 |
| | 1126.1 | 1156.6 | pep | 141 | 10 | 69 | 19 |
| ST2152 (Glycopeptide 7) | 906.6 | 204 | 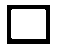 | 131 | 10 | 41 | 11 |
| | 906.6 | 274.2 | 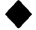 | 121 | 10 | 44 | 6 |
| | 906.6 | 1155.7 | pep | 126 | 10 | 40 | 35 |
| ST2155 (Glycopeptide 8) | 906.5 | 204.1 | 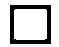 | 121 | 10 | 47 | 11 |
| | 906.5 | 274.1 | 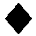 | 131 | 10 | 49 | 17 |
| | 906.5 | 1155.7 | pep | 116 | 10 | 41 | 32 |
| ST2156 (Glycopeptide 9) | 906.5 | 204 | 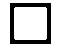 | 96 | 10 | 47 | 38 |
| | 906.5 | 274.11 | 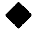 | 96 | 10 | 42 | 16 |
| | 906.5 | 1155.71 | pep | 126 | 10 | 41 | 24 |
| ST2155/2156 (Glycopeptide 10) | 1234.7 | 203.9 | 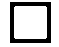 | 141 | 10 | 55 | 15 |
| | 1234.7 | 274.2 | 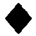 | 166 | 10 | 61 | 14 |
| | 1234.7 | 1155.5 | pep | 146 | 10 | 68 | 31 |
| ST2152/2155/2156 (Glycopeptide 11) | 1042.5 | 204.2 | 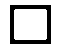 | 126 | 10 | 55 | 19 |
| | 1042.5 | 274.2 | 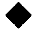 | 136 | 10 | 51 | 6 |
| | 1042.5 | 1155.7 | pep | 131 | 10 | 53 | 30 |

**[Table 1-2]**

| | | | | | | | |
|---|---|---|---|---|---|---|---|
| Tn2152 (Glycopeptide 12) | 679.8 | 185.9 | 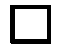 | 71 | 10 | 36 | 10 |
| | 679.8 | 204 | 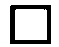 | 91 | 10 | 28 | 10 |
| | 679.8 | 1155.7 | pep | 91 | 10 | 34 | 35 |
| Tn2155 (Glycopeptide 13) | 680 | 186 | 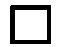 | 86 | 10 | 39 | 10 |
| | 680 | 204 | 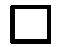 | 66 | 10 | 31 | 18 |
| | 680 | 1155.7 | pep | 66 | 10 | 29 | 32 |
| Tn2156 (Glycopeptide 14) | 679.8 | 186 | 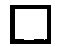 | 46 | 10 | 51 | 10 |
| | 679.8 | 204 | 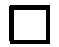 | 66 | 10 | 30 | 11 |
| | 679.8 | 1155.7 | pep | 81 | 10 | 29 | 33 |
| Tn2155/2156 (Glycopeptide 15) | 781.3 | 186 | 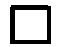 | 96 | 10 | 40 | 10 |
| | 781.3 | 204 | 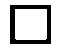 | 101 | 10 | 33 | 11 |
| | 781.3 | 1156 | pep | 91 | 10 | 37 | 32 |
| Tn2152/2155/2156 (Glycopeptide 16) | 589.2 | 186 | 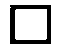 | 51 | 10 | 27 | 14 |
| | 589.2 | 204 | 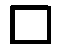 | 51 | 10 | 21 | 18 |
| | 589.2 | 1156 | pep | 51 | 10 | 25 | 18 |
| Tn2152S (Glycopeptide 17) | 825.3 | 204 | 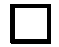 | 76 | 10 | 37 | 11 |
| | 825.3 | 274 | 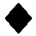 | 81 | 10 | 45 | 22 |
| | 825.3 | 1155.7 | pep | 101 | 10 | 37 | 40 |
| Tn2155S (Glycopeptide 18) | 825.3 | 204 | 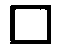 | 91 | 10 | 37 | 10 |
| | 825.3 | 273.8 | 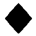 | 61 | 10 | 42 | 6 |
| | 825.3 | 1155.7 | pep | 101 | 10 | 37 | 33 |
| Tn2156S (Glycopeptide 19) | 825.3 | 204 | 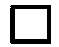 | 76 | 10 | 41 | 11 |
| | 825.3 | 274 | 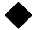 | 71 | 10 | 38 | 14 |
| | 825.3 | 1155.7 | pep | 111 | 10 | 35 | 43 |
| STn2155/2156 (Glycopeptide 20) | 715.7 | 204 | 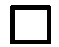 | 61 | 10 | 33 | 12 |
| | 715.7 | 274 | 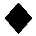 | 46 | 10 | 28 | 16 |
| | 715.7 | 1155.8 | pep | 46 | 10 | 30 | 12 |
| STn2152/2155/2156 (Glycopeptide 21) | 880.3 | 204 | 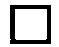 | 71 | 10 | 41 | 12 |
| | 880.3 | 274 | 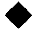 | 66 | 10 | 33 | 16 |
| | 880.3 | 1156.8 | pep | 71 | 10 | 49 | 32 |

| | | | | | | | |
|---|---|---|---|---|---|---|---|
| *1 to *4 are fragment ions generated from Peptide 1: *1: y₃ [M+H]⁺ calcd *m*/*z* 384.25, *2: y₉ [M+2H]²⁺ calcd *m*/*z* 476.77, *3: y₄ [M+H]⁺ calcd *m*/*z* 485.30, *4: y₅ [M+H]⁺ calcd *m*/*z* 556.33 □: GalNAcα1 ○: Galβ1 ◊: Neu5Ac pep: Peptide 1 | | | | | | | |

### < Preparation of calibration curves of Peptide 1 and Glycopeptides 2 to 21 (synthetic (glyco)peptide standard preparations) >

The concentrations of the standard solutions to be prepared using synthetic (glyco)peptide standard preparations (Peptide 1 and Glycopeptides 2 to 21) to be used in preparation of calibration curves were determined by amino acid analysis. Specifically, synthetic glycopeptide standard preparations, namely, Peptide 1 and Glycopeptides 2 to 21, were eluted using a nanoLC (Eksigent NanoLC Expert^{™} 400) under the following conditions. That is, elution was carried out by a gradient program, in which a solution (A/B = 95/5) consisting of eluent A (0.1% trifluoroacetic acid aqueous solution) and eluent B (acetonitrile containing 0.1% trifluoroacetic acid) was run at a flow rate of 5.0 mL/min for 30 minutes from the beginning, followed by a gradient in which the proportion of eluent B increased from 5% to 30% over 30 min [0 min: A/B = 95/5 → 30 min: A/B = 70/30].

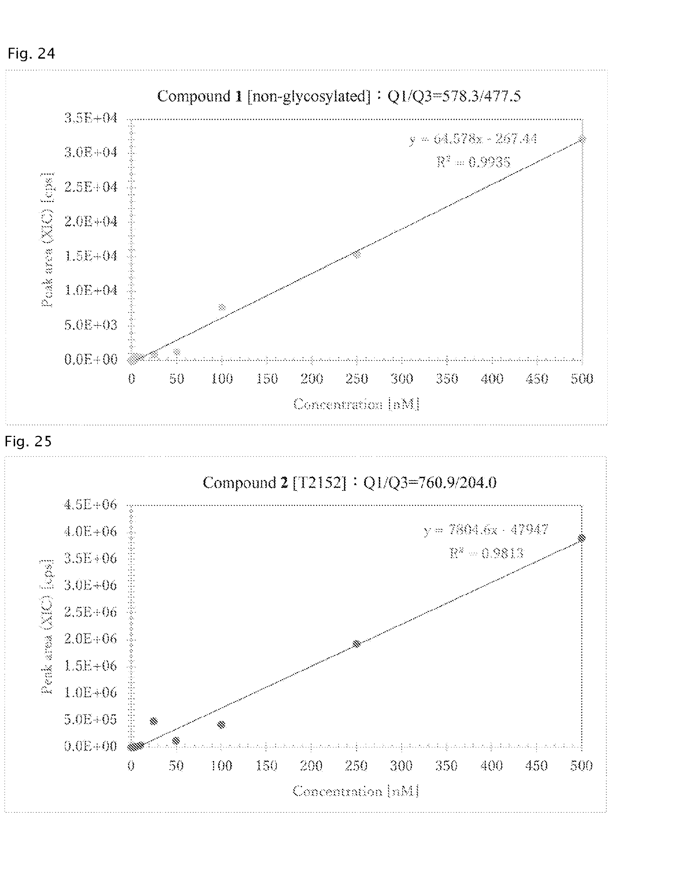

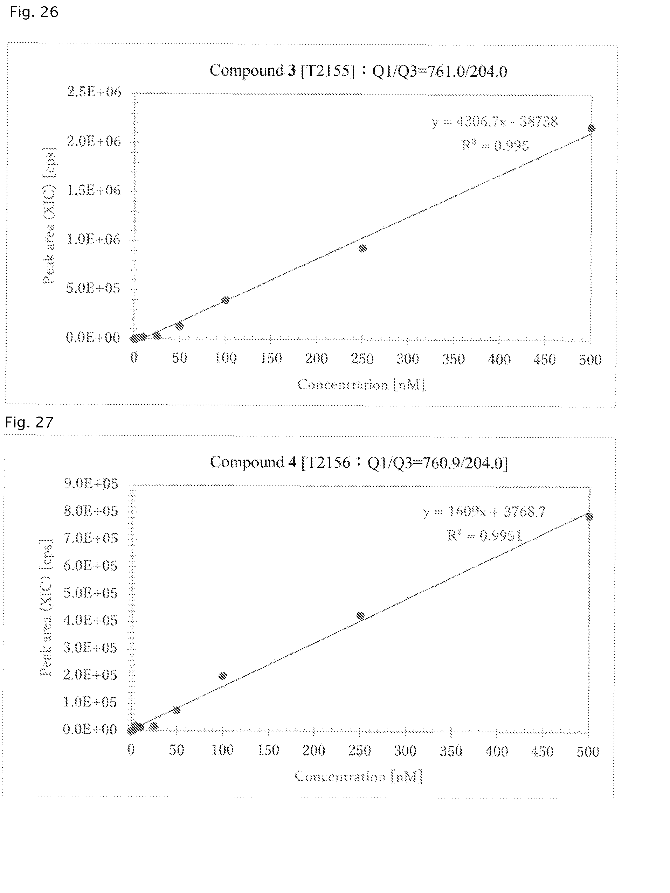

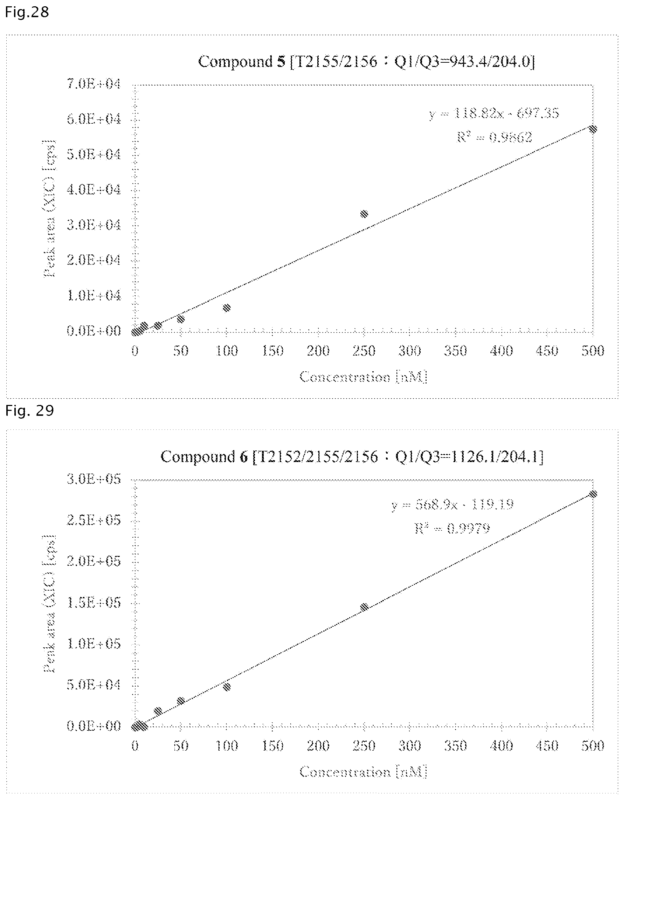

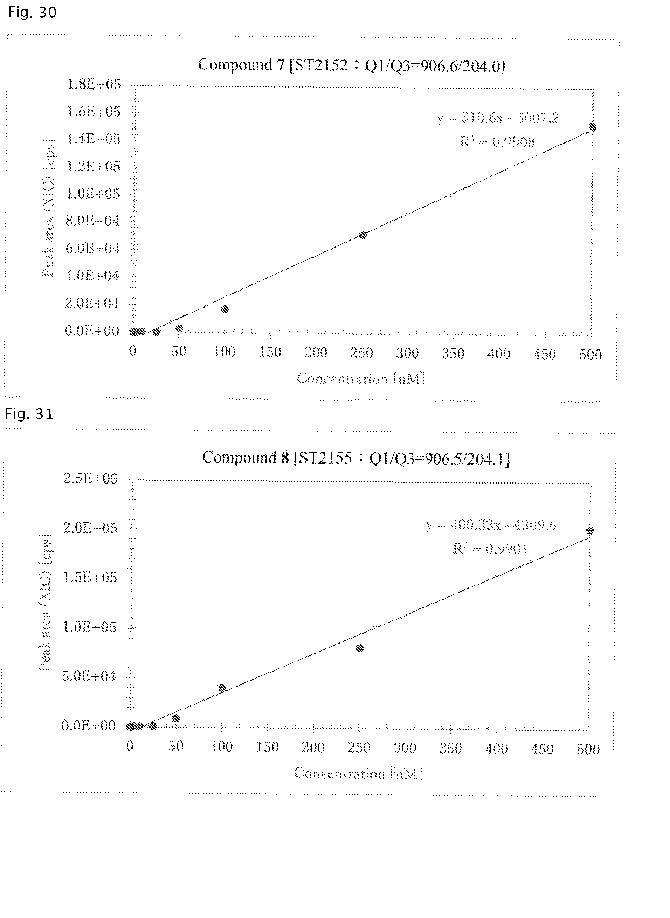

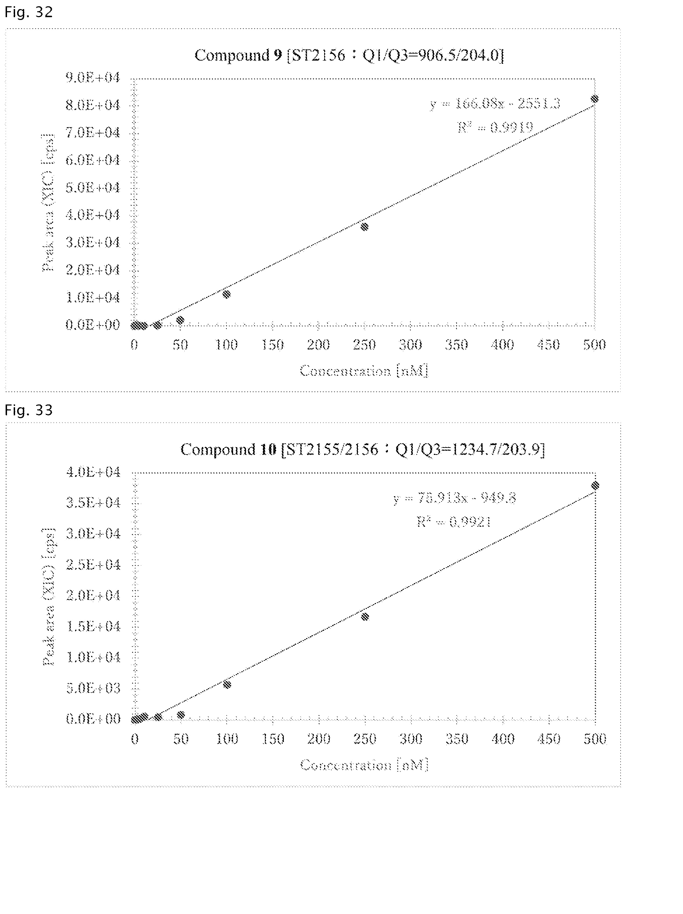

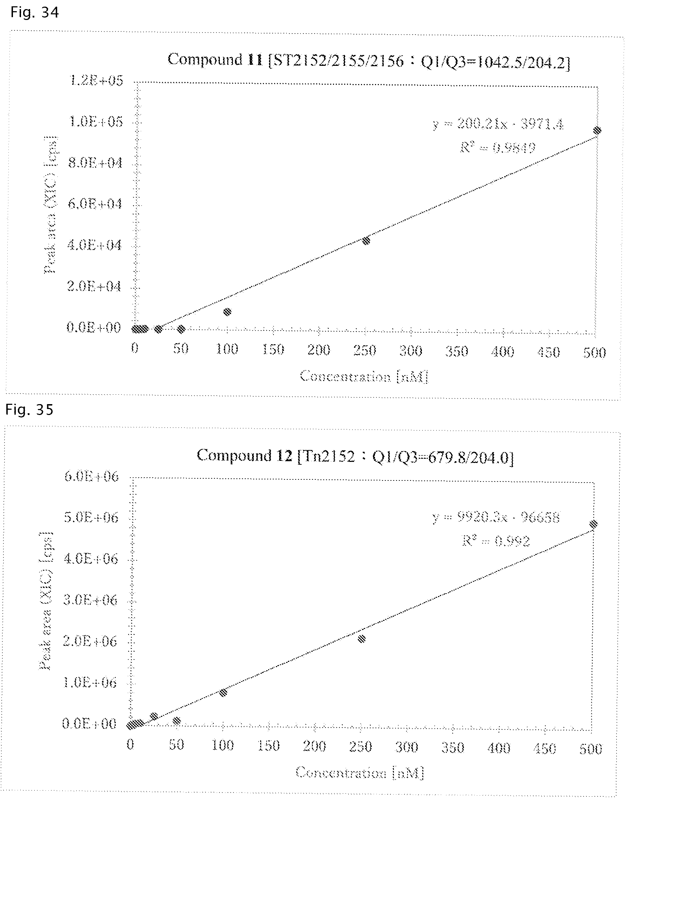

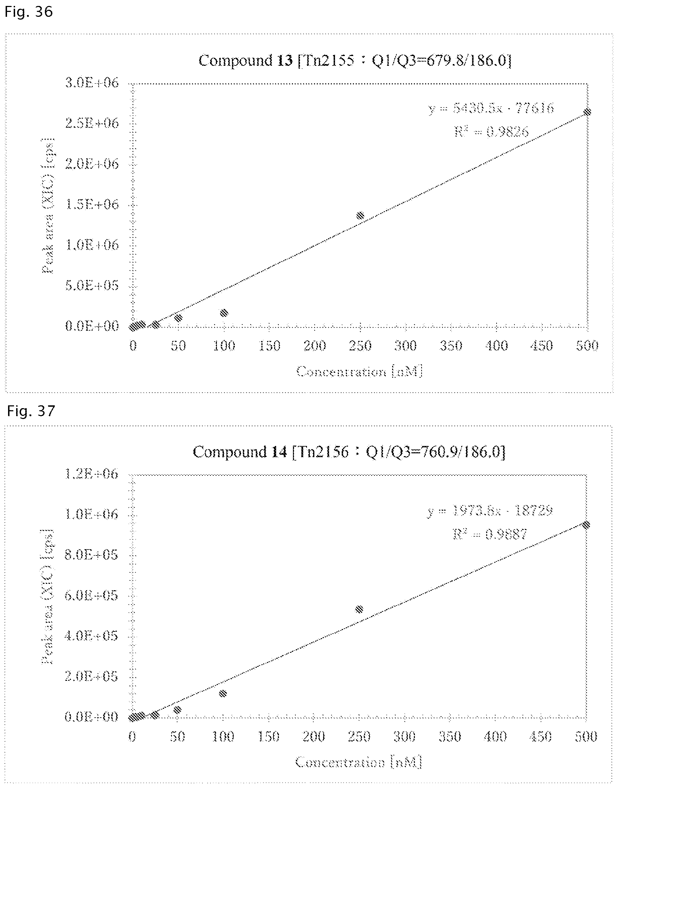

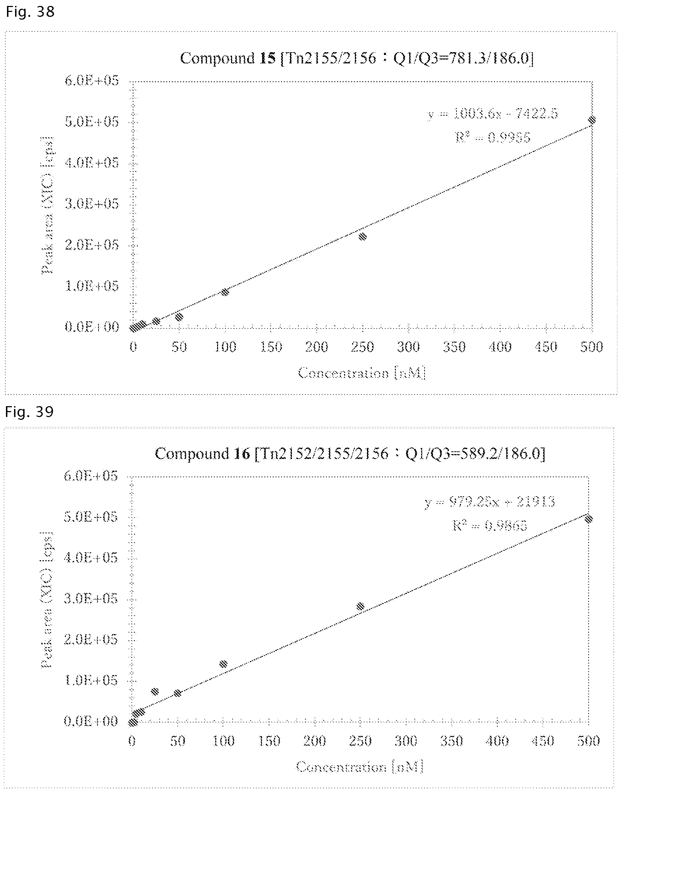

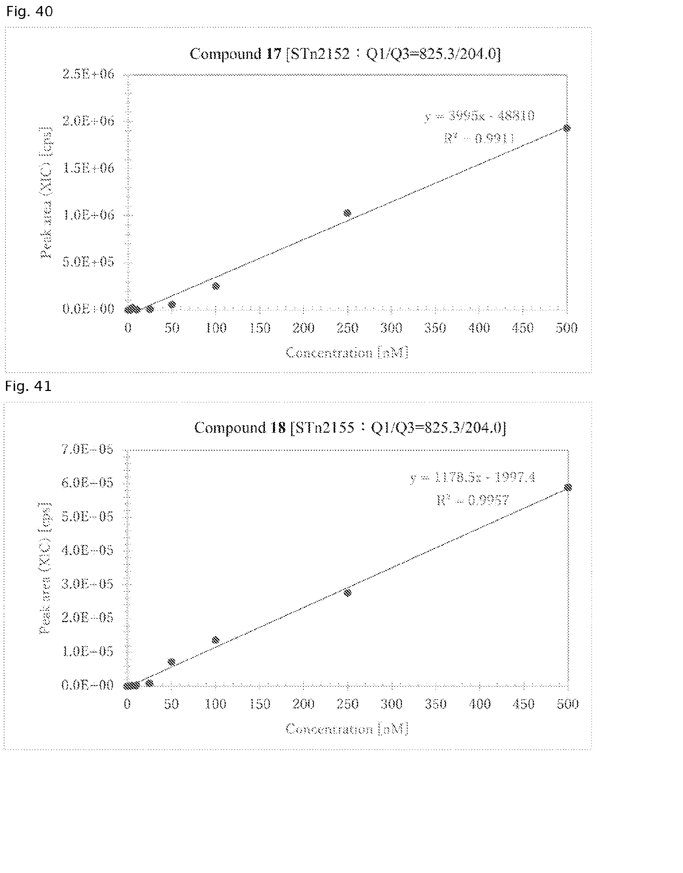

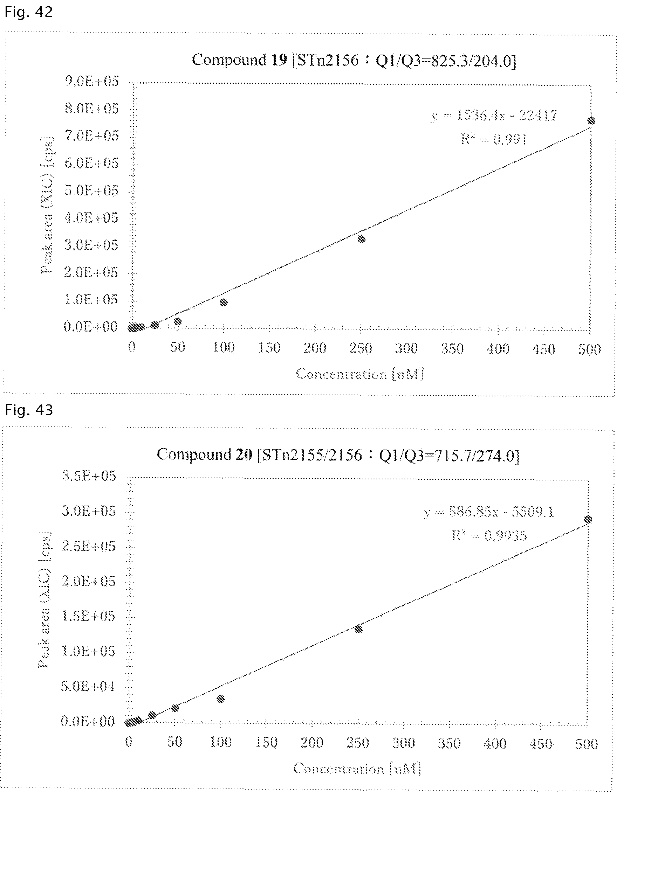

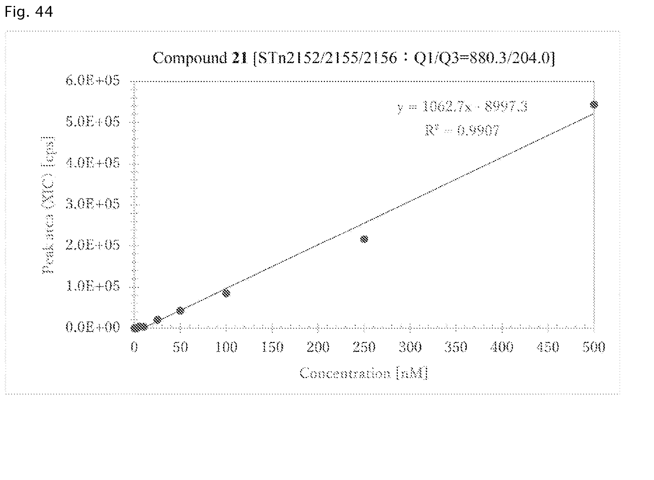

With regard to the above-described nanoLC elution conditions and the SRM (MS/MS) parameter of each glycopeptide, a calibration curve was prepared by plotting the peak areas of extracted ion chromatograms (XICs) for 0, 2.5, 5, 10, 25, 50, 100, 250, and 500 nM solutions of each glycopeptide. Among the obtained results, the SRM (MS/MS) parameter that yielded a calibration curve with the highest detection sensitivity was adopted. For example, regarding Peptide 1, CE (25 V) for generating the fragment ion *m*/*z* 477.5 (Q3) is the optimal channel. Similarly, calibration curves were prepared for all of Peptide 1 and glycopeptides 2 to 21, which were to be used as standard preparations (Figures 24 to 44).

### < Quantitative analysis of glycopeptide decomposition products (Peptide 1 and Glycopeptides 2 to 21) derived from fibronectin (FN) in peripheral blood of liver cancer patients, pancreatic cancer patients, and control healthy subjects >

### << Serum sample information >>

Forty human serum specimens were collected at Okayama University Hospital (healthy subjects' serum, entries 1 to 5; and pancreatic cancer patients' serum, stage IVb, entries 26 to 30) and Hokkaido University Hospital (hepatocellular carcinoma (HCC) patients' serum, entries 6 to 25), after the necessary procedures, including explanation of the study contents and informed consent regarding the significance of the study, were completed, and the collected samples were then stored.

It is to be noted that the implementation of the present example has already been approved by the Ethics Committee of the Graduate School of Advanced Life Science, Hokkaido University.

**[Table 2]**

| Entry | Clinical diagnosis | Sex | Age |
|---|---|---|---|
| 1 | Healthy | male | 50's |
| 2 | Healthy | male | 50's |
| 3 | Healthy | male | 50's |
| 4 | Healthy | male | 60's |
| 5 | Healthy | male | 60's |
| 6 | HCC stage 1 | male | 60's |
| 7 | HCC stage 1 | male | 70's |
| 8 | HCC stage 1 | male | 50's |
| 9 | HCC stage 1 | male | 70's |
| 10 | HCC stage 1 | male | 50's |
| 11 | HCC stage 2 | male | 50's |
| 12 | HCC stage 2 | male | 60's |
| 13 | HCC stage 2 | male | 60's |
| 14 | HCC stage 2 | male | 60's |
| 15 | HCC stage 2 | male | 50's |
| 16 | HCC stage 3 | male | 60's |
| 17 | HCC stage 3 | male | 50's |
| 18 | HCC stage 3 | male | 60's |
| 19 | HCC stage 3 | male | 50's |
| 20 | HCC stage 3 | male | 60's |
| 21 | HCC stage 4 | male | 60's |
| 22 | HCC stage 4 | male | 50's |
| 23 | HCC stage 4 | male | 60's |
| 24 | HCC stage 4 | male | 60's |
| 25 | HCC stage 4 | male | 60's |

**[Table 3]**

| Entry | Clinical diagnosis | Sex | Age |
|---|---|---|---|
| 26 | Pancreatic 4b | male | 60's |
| 27 | Pancreatic 4b | male | 60's |
| 28 | Pancreatic 4b | male | 70's |
| 29 | Pancreatic 4b | male | 60's |
| 30 | Pancreatic 4b | male | 60's |

### << Pre-treatment conditions for serum samples >>

Using the multiple-affinity removal system ("MARS", Agilent Technologies spin columns), the top 14 major serum proteins with high blood concentrations contained in serum (10 µL) were removed (N. Zolotarjova, et al., Combination of affinity depletion of abundant proteins and reversed-phase fractionation in proteomic analysis of human plasma/serum. J. Chromatogr. A 2008, 1189, 332-338; E. Jankovska, et al., Affinity depletion versus relative protein enrichment: A side-by-side comparison of two major strategies for increasing human cerebrospinal fluid proteome coverage. Clin. Proteomics 2019, 16, 9). Subsequently, the resultant was centrifuged at 12,000 g for 5 minutes using an Amicon Ultra 100K (molecular weight cutoff, Merck), and the resulting residue was then dissolved in a 50 mM ammonium carbonate aqueous solution (100 µL). Thereafter, 100 mM dithiothreitol (DTT, 10 µL) was added to the solution, and the mixture was then treated at 37°C for 1 hour. After that, 123 mM iodoacetamide (IAA, 7 µL) was further added to the reaction mixture, and the thus obtained mixture was treated at room temperature for 30 minutes in a cool, dark place. Thereafter, 100 mM DTT (10 µL) was added to the reaction system to inactivate the IAA, and then, trypsin (Promega, sequencing-grade modified trypsin, trypsin/protein = 1/50) was added, and the mixture was then digested with the trypsin at 37°C for 16 hours. Thereafter, the mixture containing glycopeptides and peptide fragments generated by the trypsin digestion was purified using a GL-Tip GC (GL-Science) (Y. Uchida, et al. A study protocol for quantitative targeted absolute proteomics (QTAP) by LC-MS/MS: Application for inter-strain differences in protein expression levels of transporters, receptors, claudin-5, and marker proteins at the blood-brain barrier in ddY, FVB, and C57BL/6J mice. Fluids Barriers CNS 2013, 10, 21). The solvent was completely distilled away, and the residue was then dissolved in a 0.1% formic acid aqueous solution (10 µL) to obtain the original serum concentration, and thereafter, the obtained solution was subjected to LC-MS/MS (SRM) analysis. Furthermore, it was confirmed that the concentration of the trypsin digest of the serum protein remained unchanged during these series of pretreatment steps by previously spiking (adding) a synthetic glycopeptide standard preparation of known concentration into the reaction system.

### << Absolute quantification of FN-derived glycopeptides in trypsin digests >>

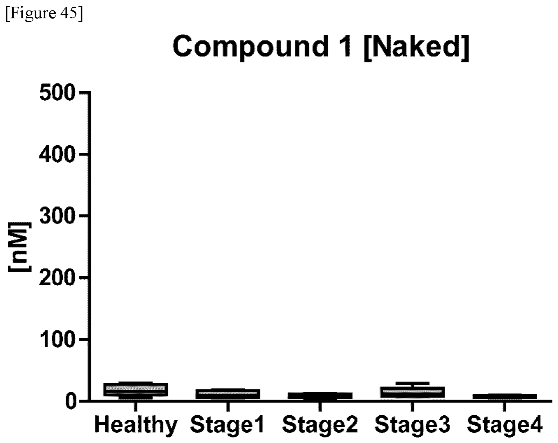

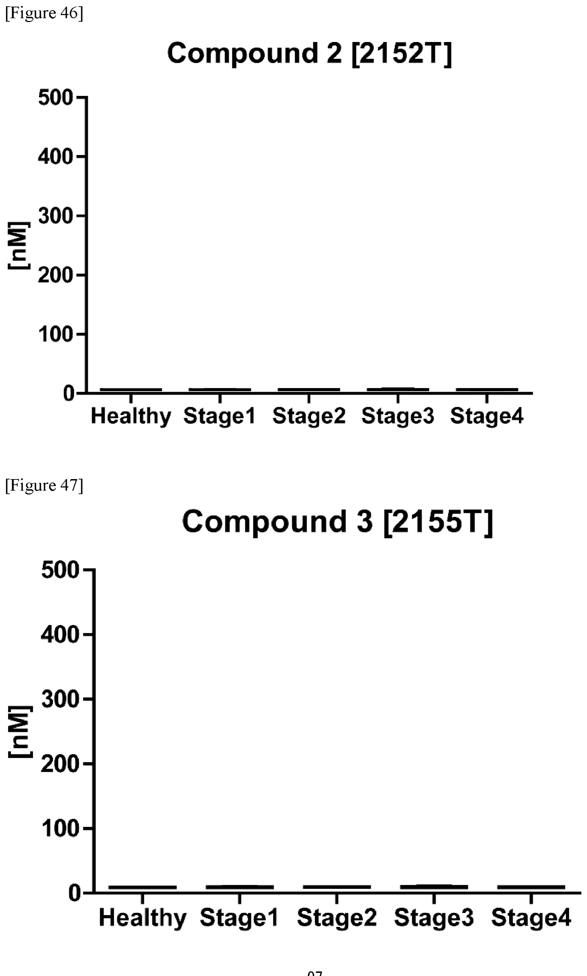

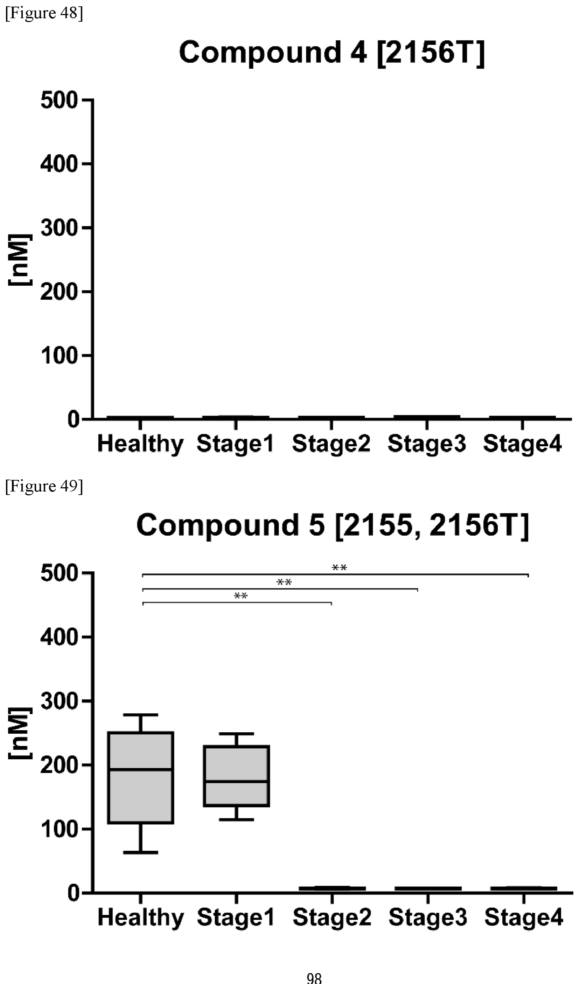

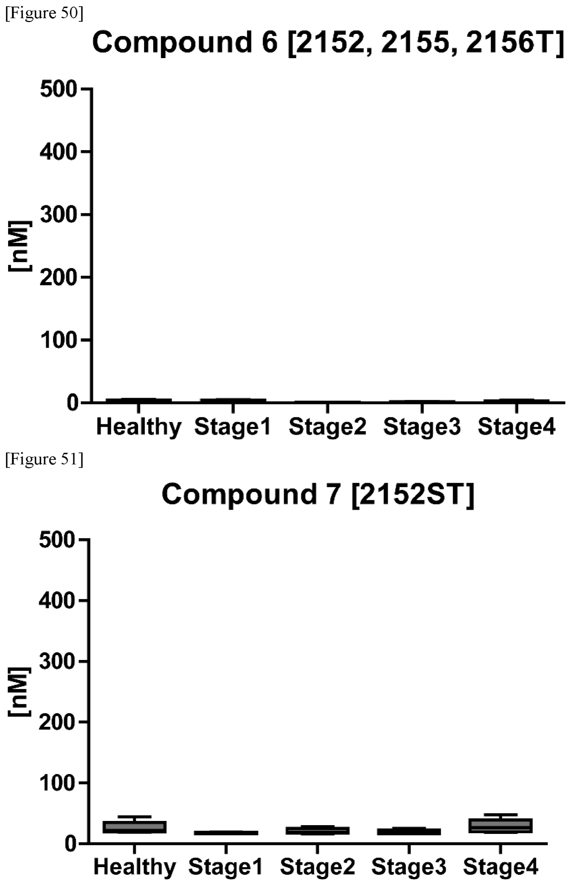

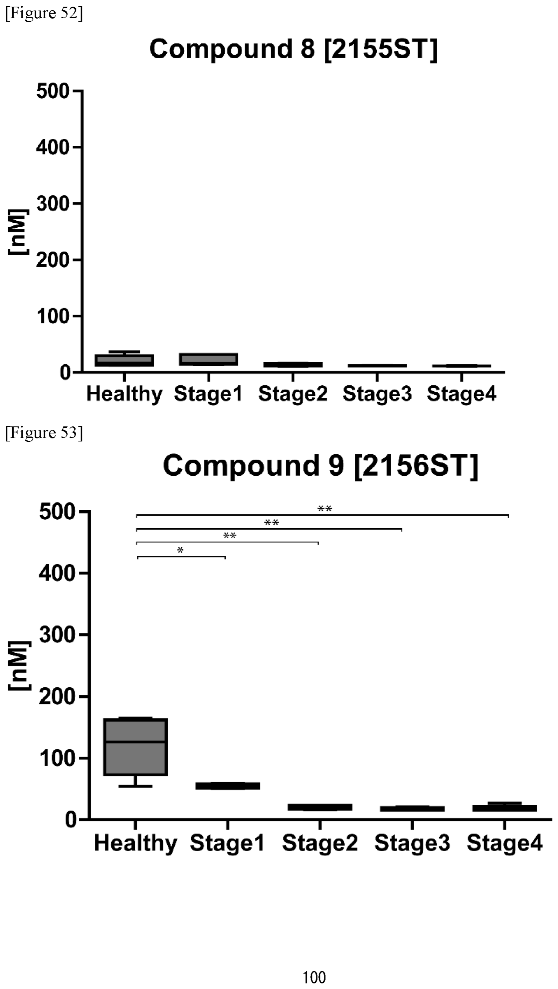

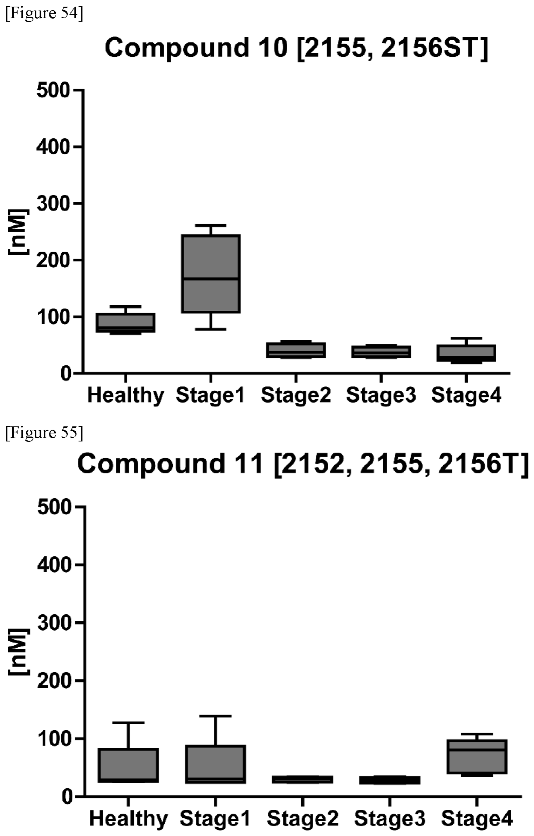

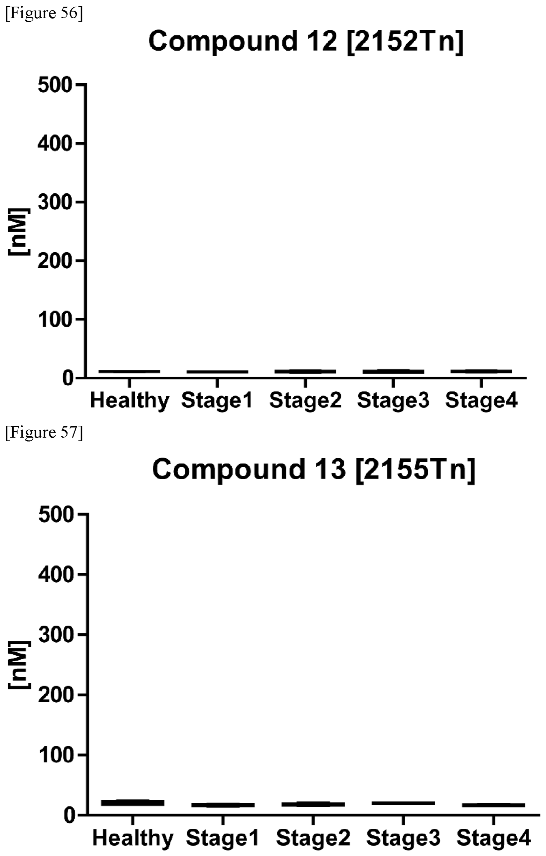

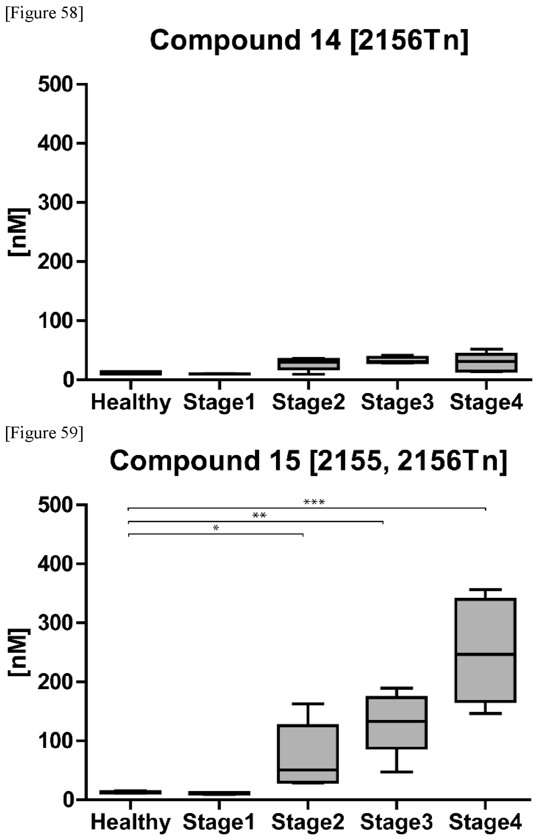

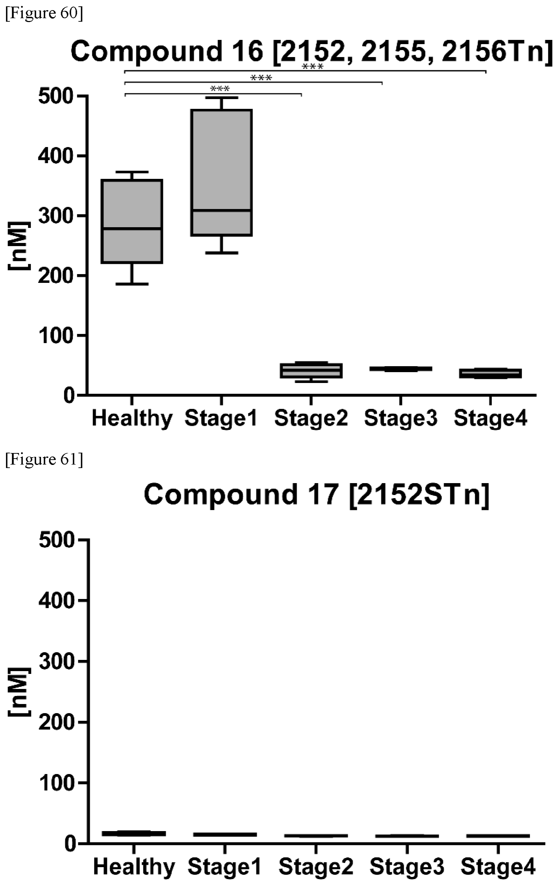

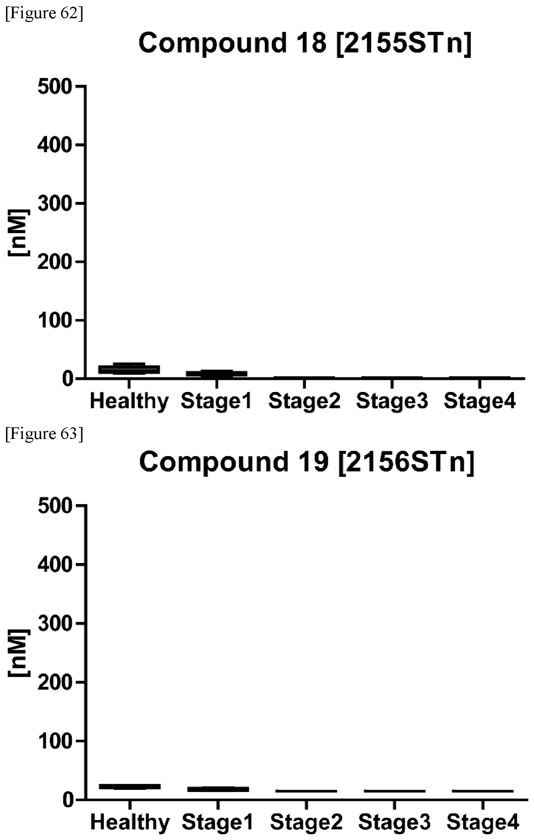

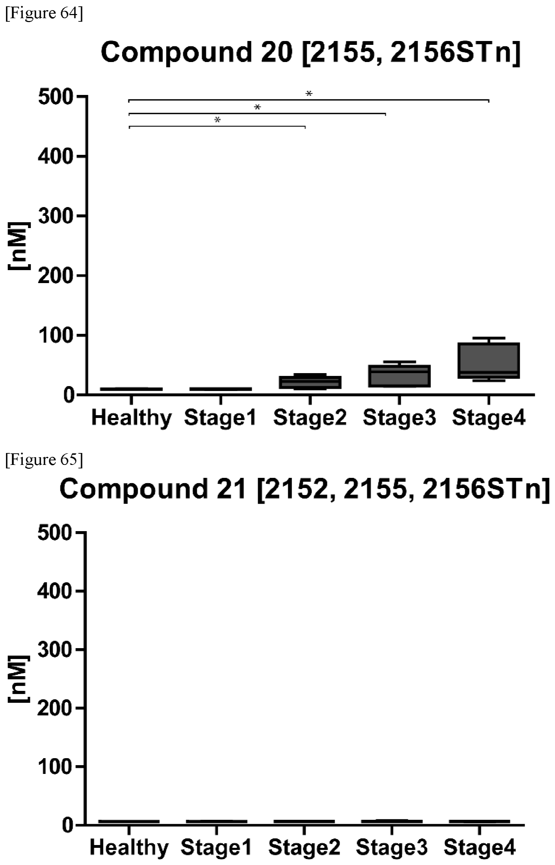

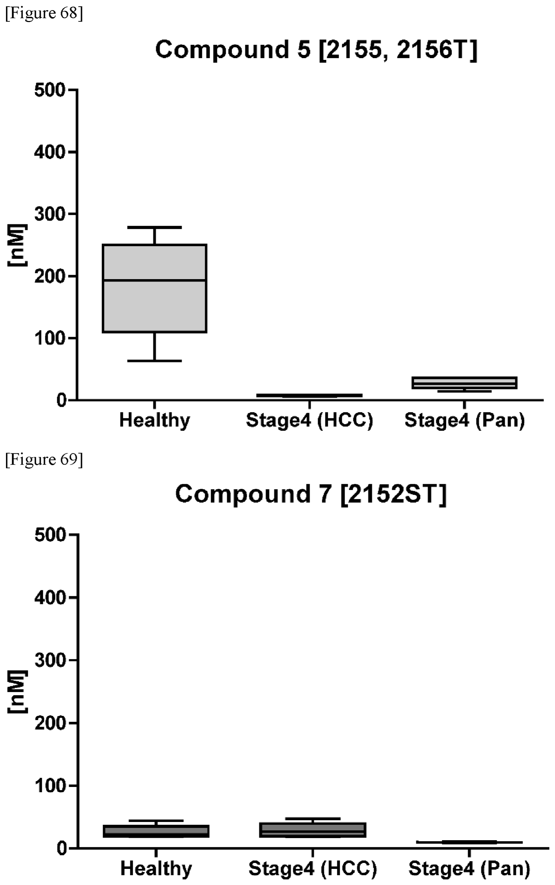

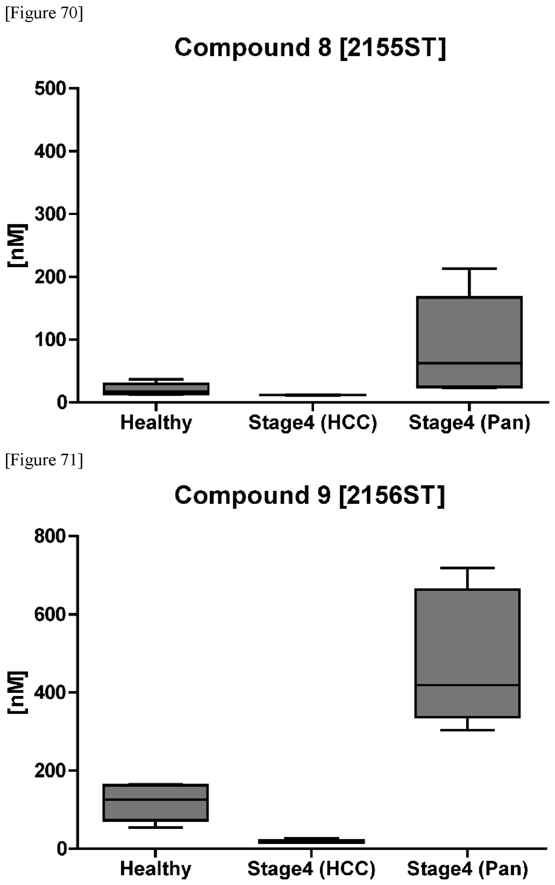

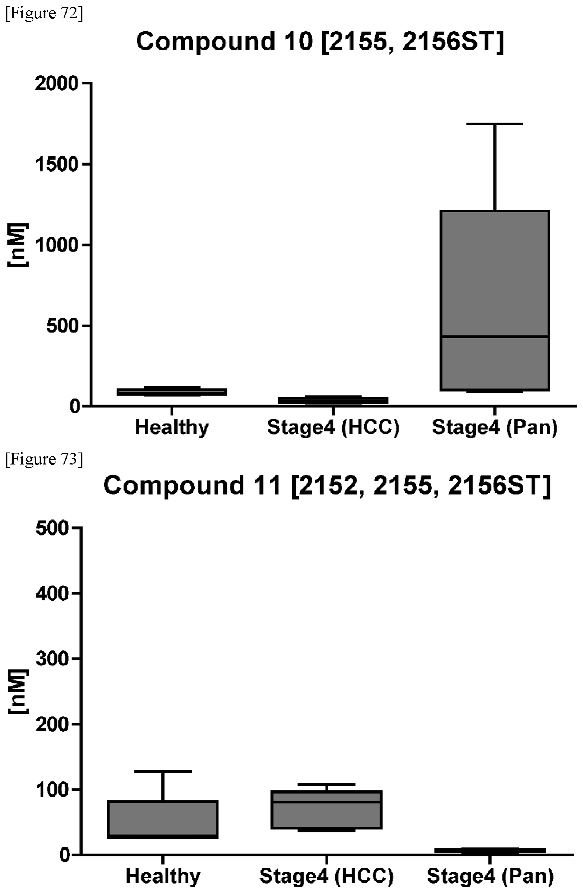

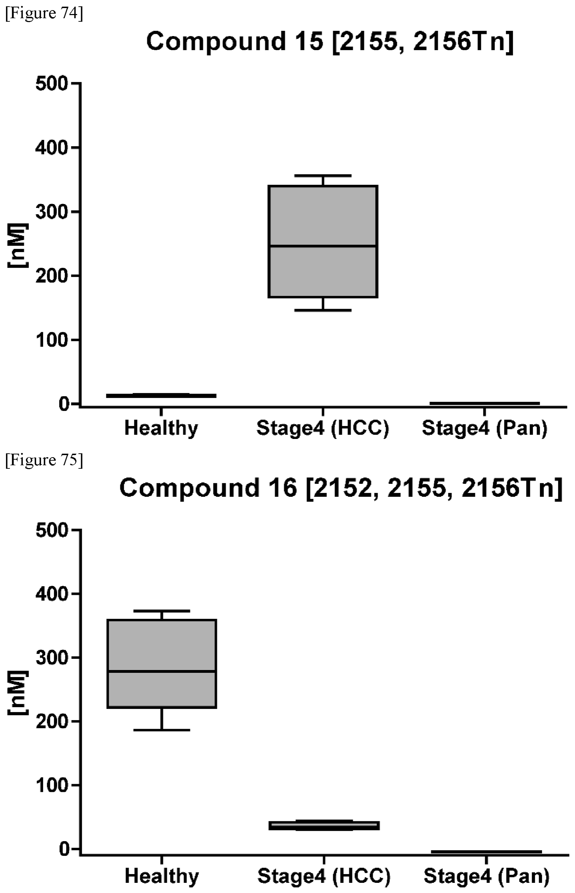

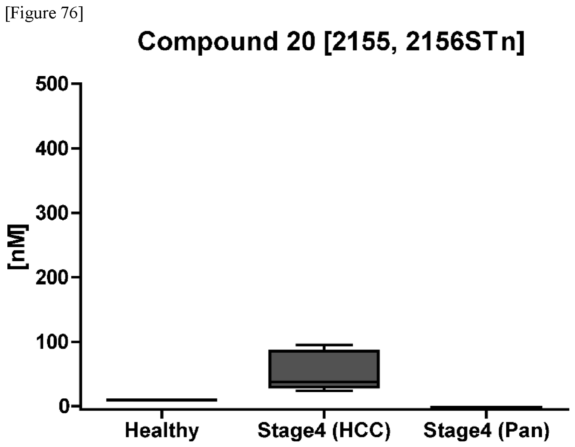

Based on the calibration curves (Figures 24 to 44) prepared using the optimized SRM channels for each of the synthesized Peptide 1 and Glycopeptides 2 to 21, the blood concentrations (nM) of target (Glyco)Peptide Fragments 1 to 21 in the trypsin digests of serum samples from liver cancer patients (Liver), pancreatic cancer patients (Pan, stage 4 only) and healthy subjects (5 specimens per group) were determined (Table 4 below). It is to be noted that these results are displayed as box plots (Figures 45 to 65 and Figures 68 to 76). It is to be noted that, in Figures 68 to 76, a comparison was made in blood glycopeptide concentrations among healthy subjects and stage 4 liver cancer and pancreatic cancer patients.

**[Table 4-1]**

| Compound | Conc.[min - max (nM)/average (nM)] | | | | | |
|---|---|---|---|---|---|---|
| | Healthy | Stage 1 (Liver) | Stage 2 (Liver) | Stage 3 (Liver) | Stage 4 (Liver) | Stage 4 (Pan) |
| Non-glycosylated (Peptide 1) | 5.54-29.91 | 5.49-18.44 | 5.34-12.40 | 7.89-28.90 | 5.52-10.02 | - |
| | 18.16 | 11.29 | 9.30 | 14.03 | 7.40 | - |
| T2152 (**2**) (Glycopeptide 2) | 6.25-6.54 | 6.34-6.74 | 6.40-6.91 | 6.31-7.51 | 6.24-6.69 | - |
| | 6.42 | 6.45 | 6.64 | 6.67 | 6.51 | - |
| T2155 (**3**) (Glycopeptide 3) | 9.09-9.46 | 9.05-10.11 | 9.19-9.76 | 9.14-10.97 | 9.07-9.87 | - |
| | 9.18 | 9.32 | 9.47 | 9.70 | 9.53 | - |
| T2156 (**4**) (Glycopeptide 4) | 2.46-3.03 | 2.44-3.83 | 2.81-3.59 | 3.16-4.53 | 2.44-3.71 | - |
| | 2.84 | 3.08 | 3.19 | 3.76 | 3.02 | - |
| T2155/2156 (**5**) (Glycopeptide 5) | 63.48-278.47 | 114.94-248.76 | 6.19-8.77 | 6.47-8.15 | 6.31-8.51 | 14.38-36.26 |
| | 182.78 | 181.21 | 7.16 | 7.34 | 7.25 | 27.75 |
| T2152/2155/2156 (**6**) (Glycopeptide 6) | 1.45-5.63 | 1.41-5.27 | 0.49-1.22 | 0.85-2.37 | 0.67-4.53 | - |
| | 2.98 | 3.02 | 0.95 | 1.25 | 1.70 | - |
| ST2152 (**7**) (Glycopeptide 7) | 18.78-44.31 | 16.27-19.98 | 16.19-28.50 | 16.31-25.58 | 18.39-47.58 | 8.54-11.25 |
| | 26.31 | 17.73 | 21.02 | 19.68 | 29.09 | 9.8 |
| ST2155 (**8**) (Glycopeptide 8) | 12.90-37.04 | 13.93-32.32 | 11.20-16.89 | 11.27-12.67 | 11.21-12.16 | 23.40-213.36 |
| | 20.58 | 22.17 | 13.47 | 11.91 | 11.63 | 89.07 |
| ST2156 (**9**) (Glycopeptide 9) | 54.56-165.41 | 50.86-59.16 | 16.59-23.59 | 20.88-15.54 | 15.46-26.75 | 303.34-718.39 |
| | 119.44 | 55.28 | 20.96 | 17.43 | 18.21 | 483.89 |
| ST2155/2156 (**10**) (Glycopeptide 10) | 70.53-118.15 | 77.92-261.35 | 28.20-56.72 | 28.03-49.99 | 19.48-62.19 | 93.47-1752.96 |
| | 88.02 | 173.81 | 40.18 | 37.64 | 34.73 | 611.52 |

**[Table 4-2]**

| | | | | | | |
|---|---|---|---|---|---|---|
| ST2152/2155/2156 (**11**) (Glycopeptide 11) | 26.57-127.87 | 24.76-139.01 | 24.92-33.79 | 22.81-34.42 | 36.82-108.24 | 4.16-9.07 |
| | 49.42 | 51.12 | 29.63 | 28.30 | 71.32 | 7.31 |
| Tn2152 (**12**) (Glycopeptide 12) | 10.80-11.52 | 10.00-10.76 | 9.84-12.51 | 9.82-13.04 | 10.15-12.69 | - |
| | 11.09 | 10.47 | 10.94 | 10.88 | 11.34 | - |
| Tn2155 (**13**) (Glycopeptide 13) | 17.97-23.64 | 15.82-18.48 | 16.48-20.10 | 19.66-20.19 | 15.87-18.08 | - |
| | 20.44 | 16.91 | 17.76 | 19.94 | 16.62 | - |
| Tn2156 (**14**) (Glycopeptide 14) | 9.75-14.07 | 9.61-10.48 | 9.50-36.37 | 28.61-41.33 | 13.76-51.89 | - |
| | 11.39 | 9.86 | 27.21 | 33.09 | 29.42 | - |
| Tn2155/2156 (**15**) (Glycopeptide 15) | 11.59-15.09 | 9.09-12.34 | 28.70-162.54 | 47.15-189.34 | 146.30-356.34 | 1.22-1.40 |
| | 12.93 | 10.90 | 72.38 | 131.19 | 251.96 | 1,29 |
| Tn2152/2155/2156 (**16**) (Glycopeptide 16) | 186.18-373.05 | 238.05-497.43 | 23.05-54.74 | 41.37-46.65 | 30.28-44.11 | ~ 0 |
| | 288.13 | 359.47 | 41.31 | 44.73 | 36.24 | ~ 0 |
| STn2152 (**17**) (Glycopeptide 17) | 14.27-19.28 | 13.92-15.76 | 12.44-13.17 | 12.26-12.94 | 12.33-13.08 | - |
| | 16.65 | 14.75 | 12.79 | 12.50 | 12.66 | - |
| STn2155 (**18**) (Glycopeptide 18) | 9.18-25.51 | 5.34-13.10 | 1.74-1.82 | 1.74-2.04 | 1.74-2.04 | - |
| | 15.21 | 8.62 | 1.78 | 1.86 | 1.83 | - |
| STn2156 (**19**) (Glycopeptide 19) | 19.98-24.86 | 15.96-20.49 | 14.61-14.79 | 14.64-14.70 | 14.62-14.89 | - |
| | 22.61 | 18.00 | 14.65 | 14.67 | 14.71 | - |
| STn2155/2156 (**20**) (Glycopeptide 20) | 9.59-10.56 | 9.55-10.56 | 10.49-33.89 | 14.80-55.43 | 24.15-95.47 | ~ 0 |
| | 9.86 | 10.06 | 21.19 | 33.15 | 53.89 | ~ 0 |
| STn2152/2155/2156 (**21**) (Glycopeptide 21) | 8.90-14.68 | 9.50-19.12 | 8.61-8.94 | 8.50-8.76 | 8.54-8.69 | - |
| | 12.67 | 13.12 | 8.72 | 8.62 | 8.61 | - |

All of Glycopeptide Fragments 5, 9, 10, and 16 were found to be at high concentrations (100 to 300 nM) in the serum of healthy subjects and early-stage liver cancer patients (stage 1). It became clear from these results that the peptide region ²¹⁵¹Thr-Thr-Pro-Pro-Thr-Thr-Ala-Thr-Pro-Ile-Arg²¹⁶¹, generated by trypsin digestion within the IIICS domain, is universally expressed in fibronectin, and that the three notable sites (Thr2152, Thr2155, and Thr2156) are modified with 4 types of cancer-specific O-glycans at high frequency. In addition, Glycopeptide Fragments 2, 3, 4, 6, 7, 8, 12, 13, 17, 18, and 19 were rarely detected in liver cancer patients, suggesting that glycosylation within this region occurs at, at least, two (5, 10, 15, and 20) of the three notable sites, or at all of the three positions (11 and 16) (these six glycopeptides accounted for 70% of the total). In particular, Glycopeptide Fragments 5, 10, 15, and 20, in which Thr2155 and Thr2156 were simultaneously modified, accounted for 40% of the total.

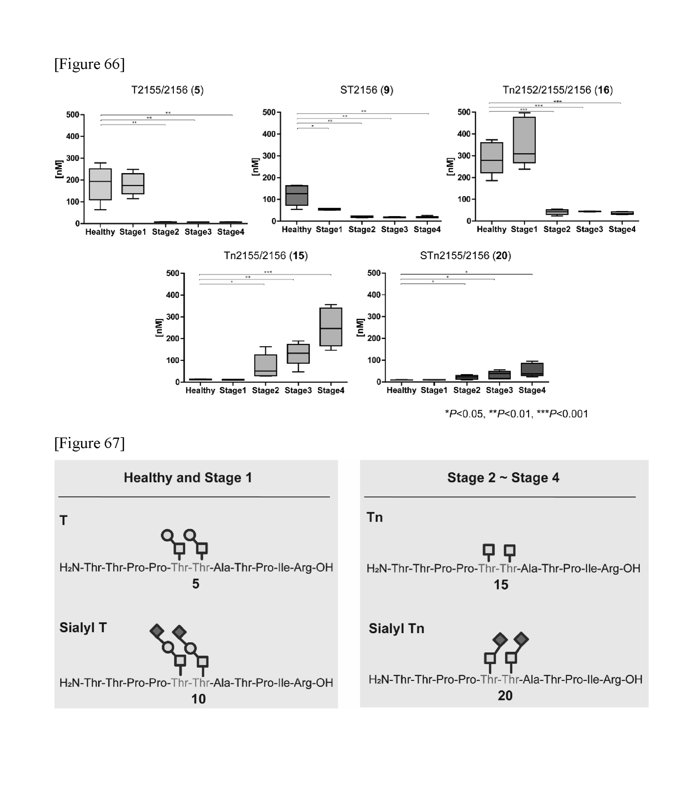

Furthermore, a statistical significance test (unpaired two-tailed t-test) was carried out on Glycopeptides 5, 9, 15, 16, and 20, which show a significant correlation with the progression level (stage) of liver cancer. As shown in Figure 66, position-specific changes in glycan structure were found to occur with cancer progression.

With regard to the fluctuation profile of the concentrations (expression levels) of Glycopeptide Fragments 5, 9, 15, 16, and 20 derived from the IIICS domain of fibronectin in liver cancer, it became clear that the tendency of increase and decrease of Glycopeptide Fragments 5, 9, and 16 was linked to that of Glycopeptide Fragments 15 and 20, and that a sharp change occurred at the boundary between the group of healthy subjects and stage 1 cancer patients, and the group of stage 2 or later cancer patients. Focusing on Glycopeptide Fragments 5, 10, 15, and 20, in which Thr2155 and Thr2156 are simultaneously modified among the three sites (Thr2152, Thr2155, and Thr2156) reported to be glycosylated within this region, it is considered that a significant structural changes, as shown in Figure 67, is generated by post-translational modification in this peptide region, as cancer progresses from stage 1 to stage 2 or later.

As described above, in the present embodiment, there has been realized a novel technique of diagnosing liver cancer, and in particular, of rapidly, quantitatively and precisely determining the progression level of the cancer from early stage (stage 1) to stage 2 or later, according to tandem mass spectrometry (SRM method) using human serum. The fibronectin-derived glycopeptides produced by precision synthesis are novel substances that enable the present technique and also serve as cancer-specific "dynamic epitopes." Antibodies that specifically recognize these glycopeptides can also serve as new important target molecules in the development of new pharmaceutical products that prevent cancer spread and metastasis. Therefore, the significance and impact of the present invention in creation of new techniques and/or new industries is extremely significant.

Furthermore, significant differences were found in the concentrations of Glycopeptide 8, Glycopeptide 9 and Glycopeptide 10 between stage 4 pancreatic cancer patients and healthy subjects. It has been demonstrated that the measurement of the concentrations of these glycopeptides may enable the diagnosis of pancreatic cancer. Thus, the present technique is also effective in diagnosing cancers other than liver cancer.

The present invention is the world's first rapid and precise diagnostic technique capable of determining the stage of cancer progression through the quantitative analysis of specific glycopeptide fragments in the trypsin digest of a blood protein, using LC-MS/MS mass spectrometry (selective reaction monitoring method, SRM). It is expected that the present technique will be put into practical use in society in the early stage.
SEQ ID NO: 1
TTPPTTATPIR

## Claims

1. A glycopeptide, in which one glycan consisting of 6 or less sugars binds to each of one or two or more threonines in a peptide consisting of the amino acid sequence TTPPTTATPIR, and the glycan is an O-linked glycan.

2. The glycopeptide according to claim 1, wherein the glycan is selected from the group consisting of the following (1) to (4):
(1) GalNAcα1,
(2) Galβ1,3GalNAcα1,
(3) Neu5Acα2,6GalNAcα1, and
(4) Neu5Acα2,3Galβ1,3GalNAcα1.

3. The glycopeptide according to claim 1, wherein the binding position(s) of the glycan is one, two or three sites selected from the group consisting of the threonine at position 2, the threonine at position 5, and the threonine at position 6 from the N-terminal side.

4. The glycopeptide according to claim 1, wherein the binding position(s) of the glycan is the threonine at position 2, the threonine at position 5, the threonine at position 6, the threonines at positions 2 and 5, the threonines at positions 2 and 6, the threonines at positions 5 and 6, or the threonines at positions 2, 5 and 6, from the N-terminal side.

5. The glycopeptide according to claim 1, wherein the glycans binding to each of the two or more threonines are homologous or heterologous.

6. The glycopeptide according to claim 2, wherein the glycopeptide having the glycan (2) is a glycopeptide represented by any of the following structural formulae:

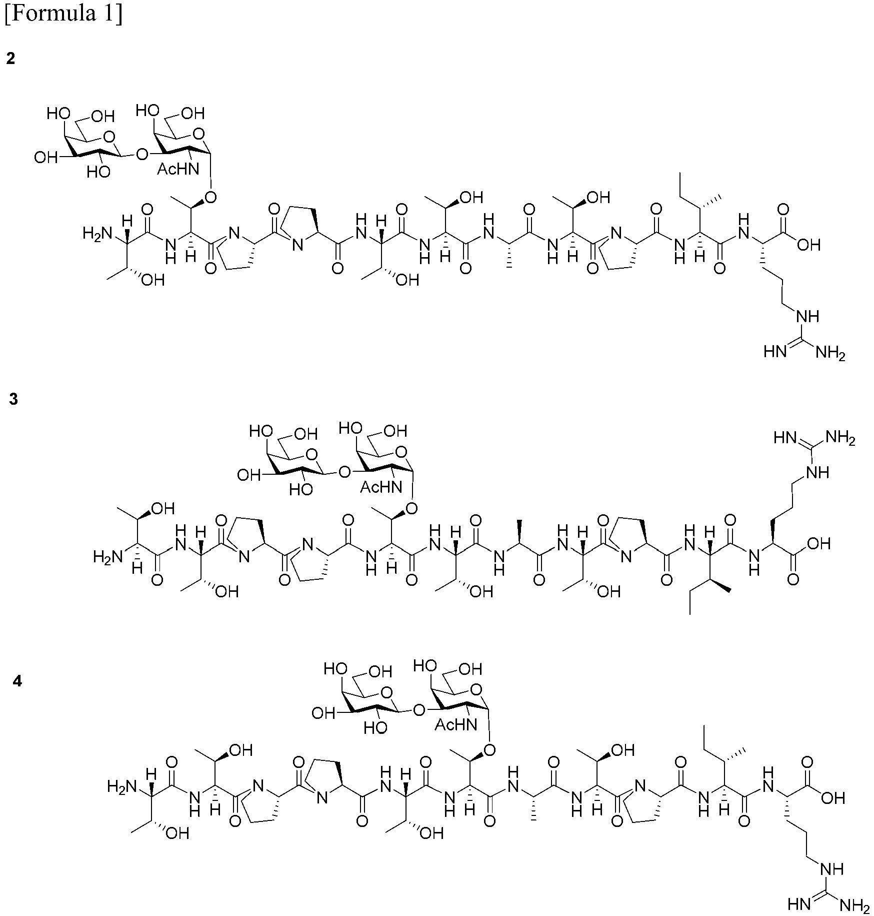

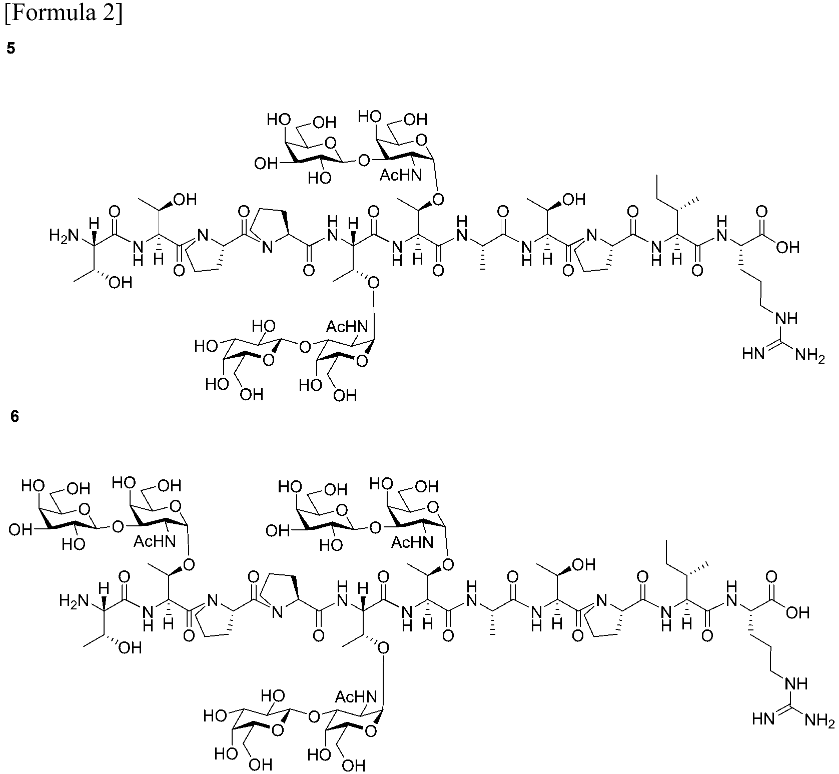

7. The glycopeptide according to claim 2, wherein the glycopeptide having the glycan (4) is a glycopeptide represented by any of the following structural formulae:

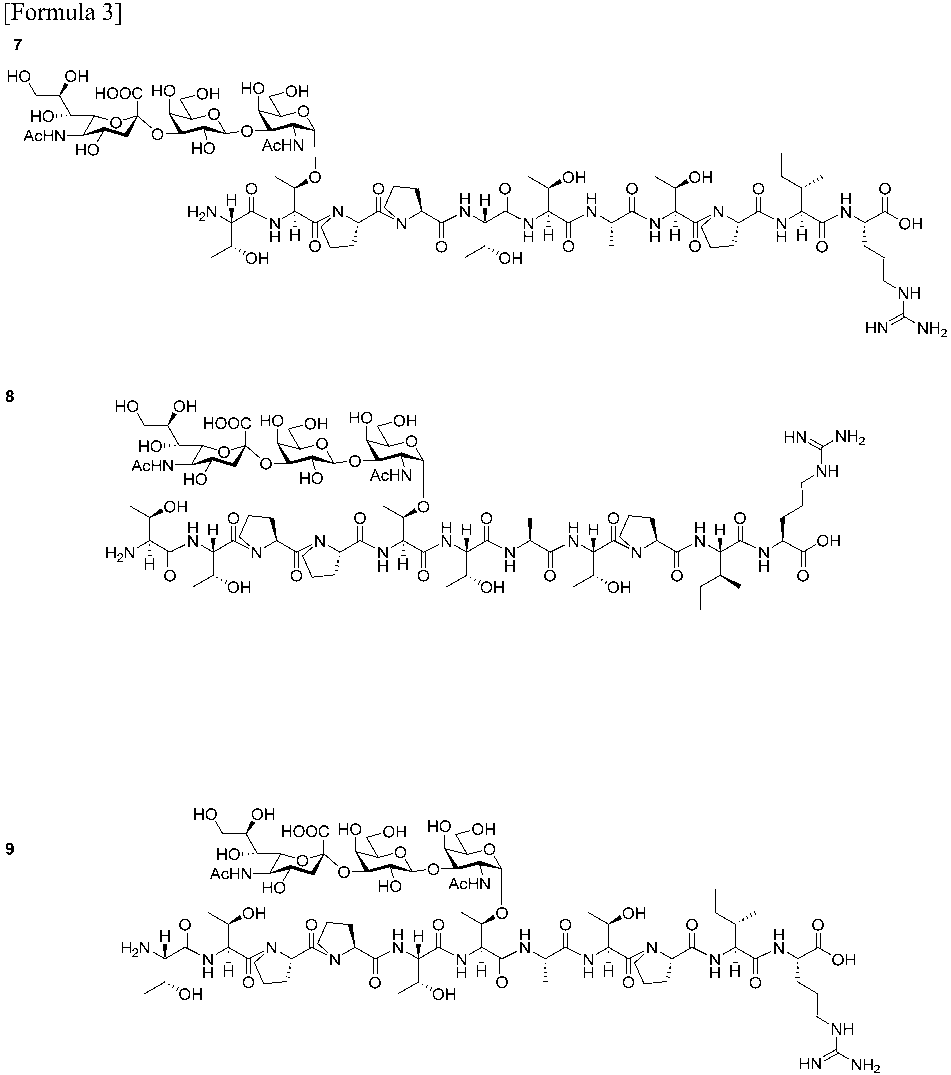

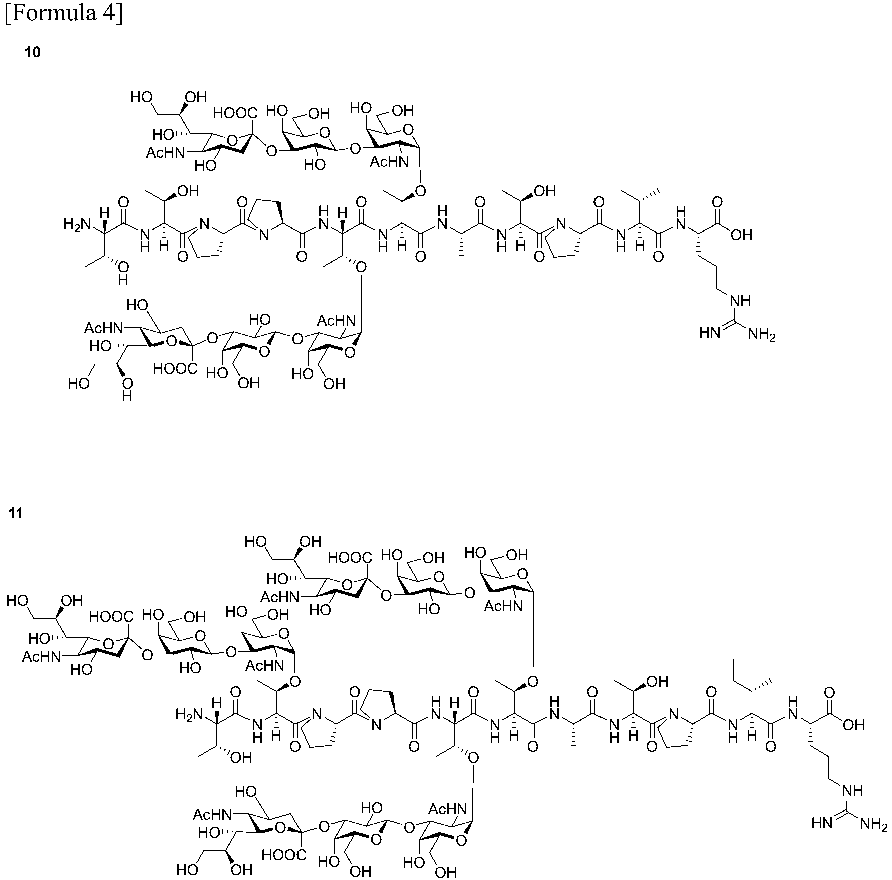

8. The glycopeptide according to claim 2, wherein the glycopeptide having the glycan (1) is a glycopeptide represented by any of the following structural formulae:

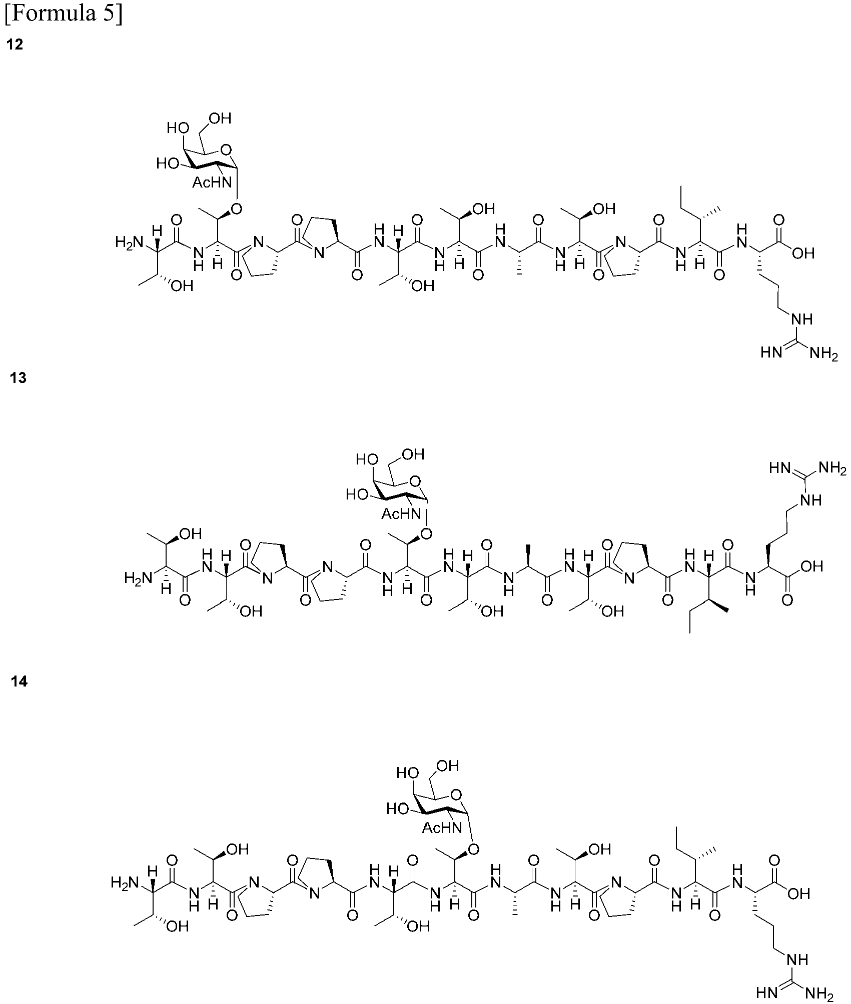

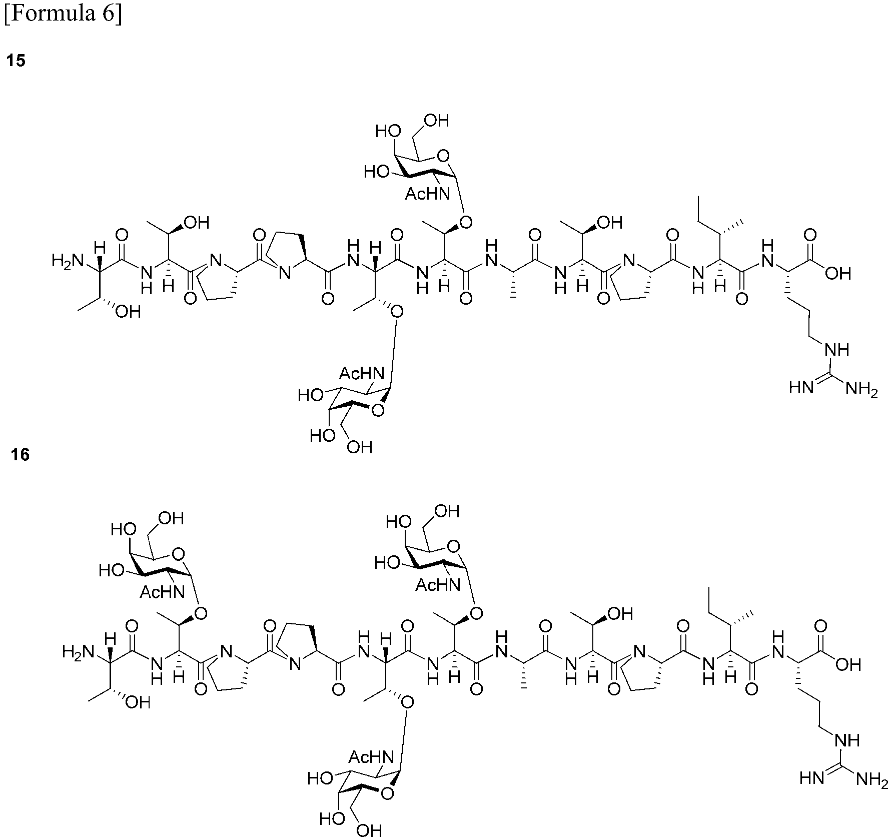

9. The glycopeptide according to claim 2, wherein the glycopeptide having the glycan (3) is a glycopeptide represented by any of the following structural formulae:

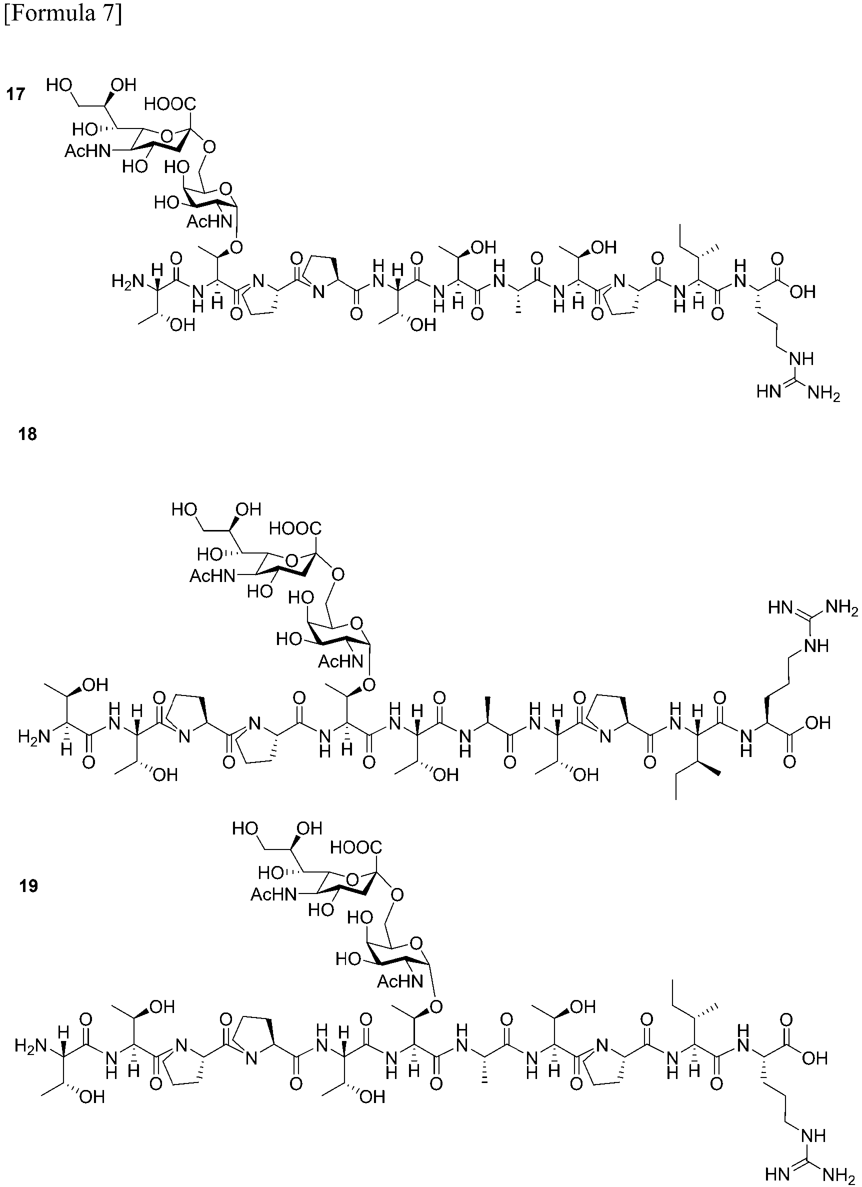

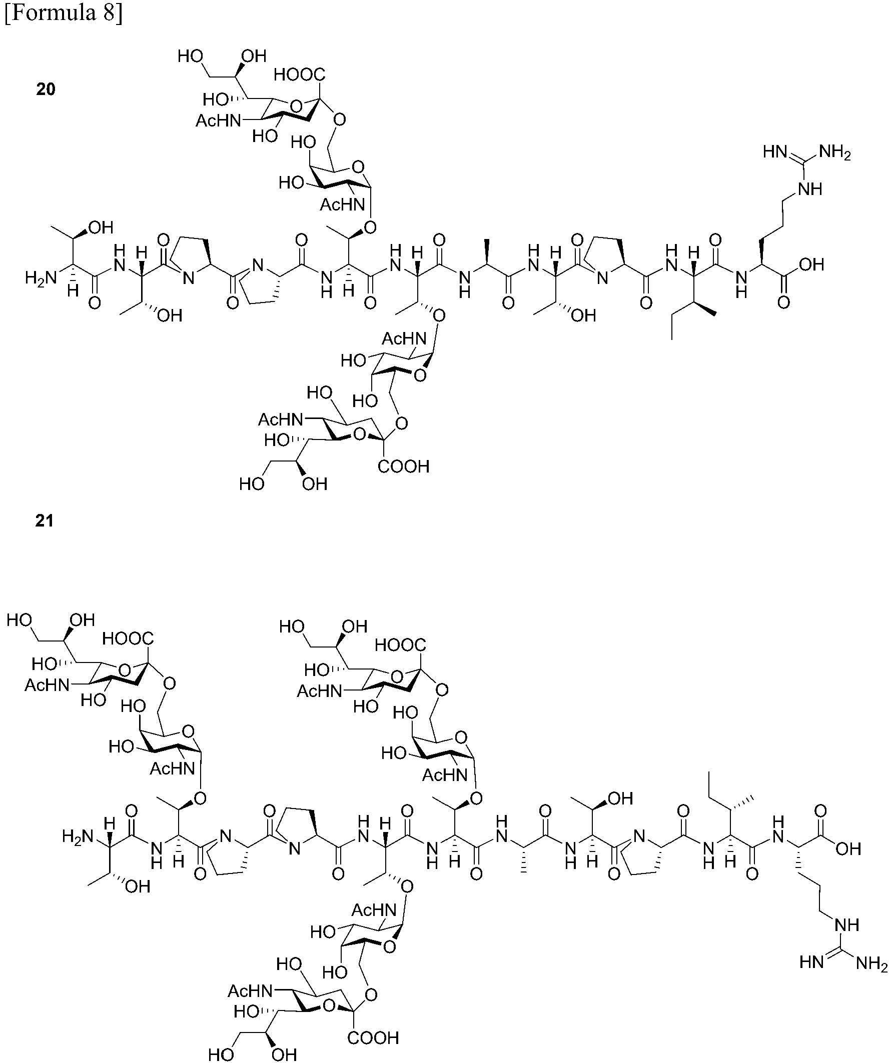

10. The glycopeptide according to any one of claims 1 to 9, which is used as a tumor marker.

11. The glycopeptide according to any one of claims 1 to 9, which is used as an immunogen for antibody production.

12. A method for analyzing a glycopeptide, comprising:
analyzing whether or not a glycopeptide is contained in a sample containing a glycopeptide obtained by protease digestion of the serum or the plasma, wherein
the glycopeptide is a peptide consisting of the amino acid sequence TTPPTTATPIR, in which one glycan consisting of 6 or less sugars binds to each of one or two or more threonines, and the glycan is an O-linked glycan.

13. The method for analyzing a glycopeptide according to claim 12, comprising analyzing whether or not Glycopeptide 15 and/or Glycopeptide 20 represented by the following structural formulae are contained in the sample:

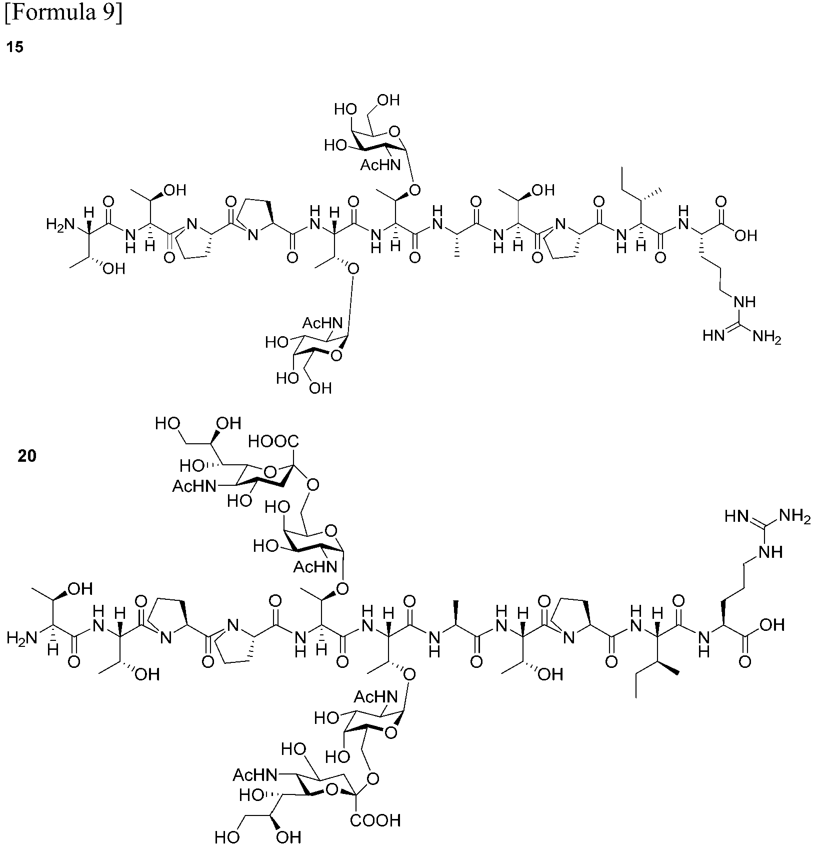

14. The method for analyzing a glycopeptide according to claim 13, comprising further analyzing whether or not one type, or two or more types selected from the group consisting of Glycopeptide 5, Glycopeptide 9, Glycopeptide 10 and Glycopeptide 16 represented by the following structural formulae are contained in the sample:

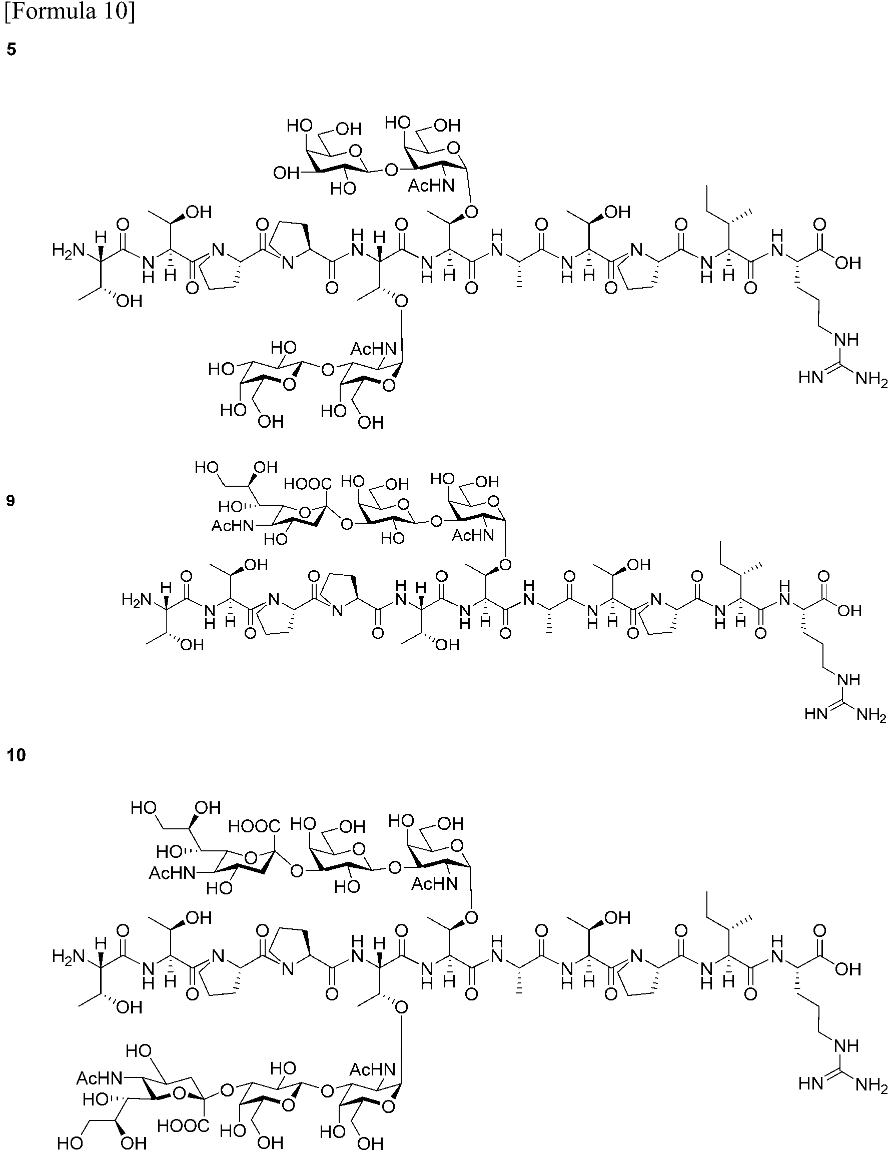

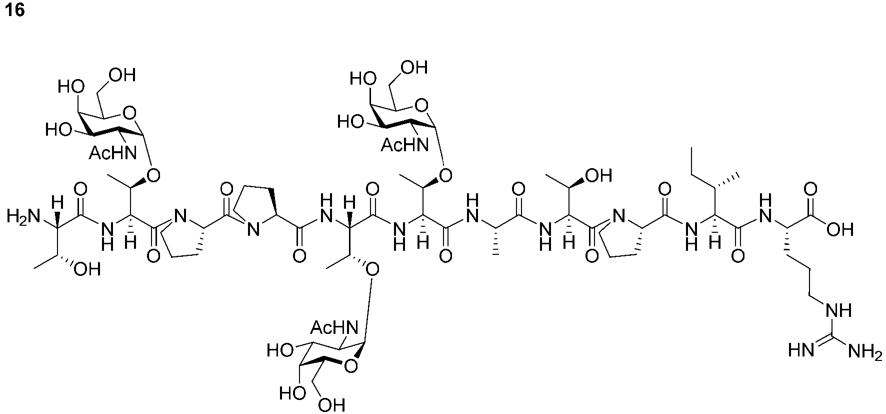

15. A method for analyzing a glycopeptide, comprising analyzing whether or not one type, or two or more types selected from the group consisting of Glycopeptide 5, Glycopeptide 9, Glycopeptide 10 and Glycopeptide 16 represented by the following structural formulae are contained in a sample containing a glycopeptide obtained by protease digestion of the serum or the plasma:

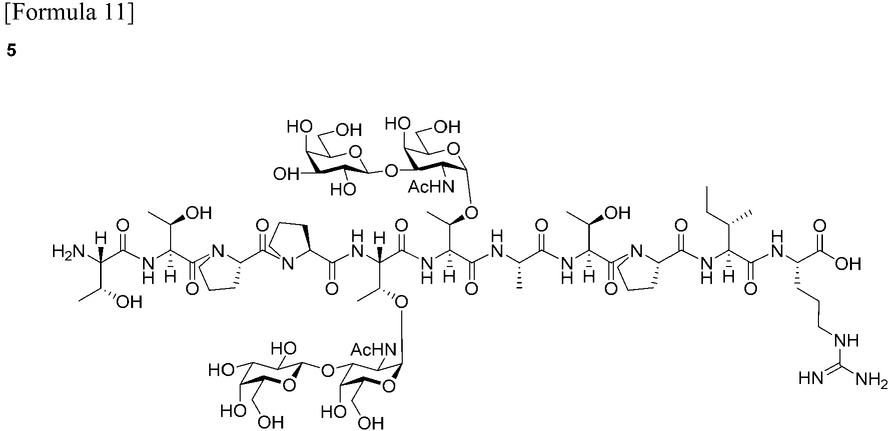

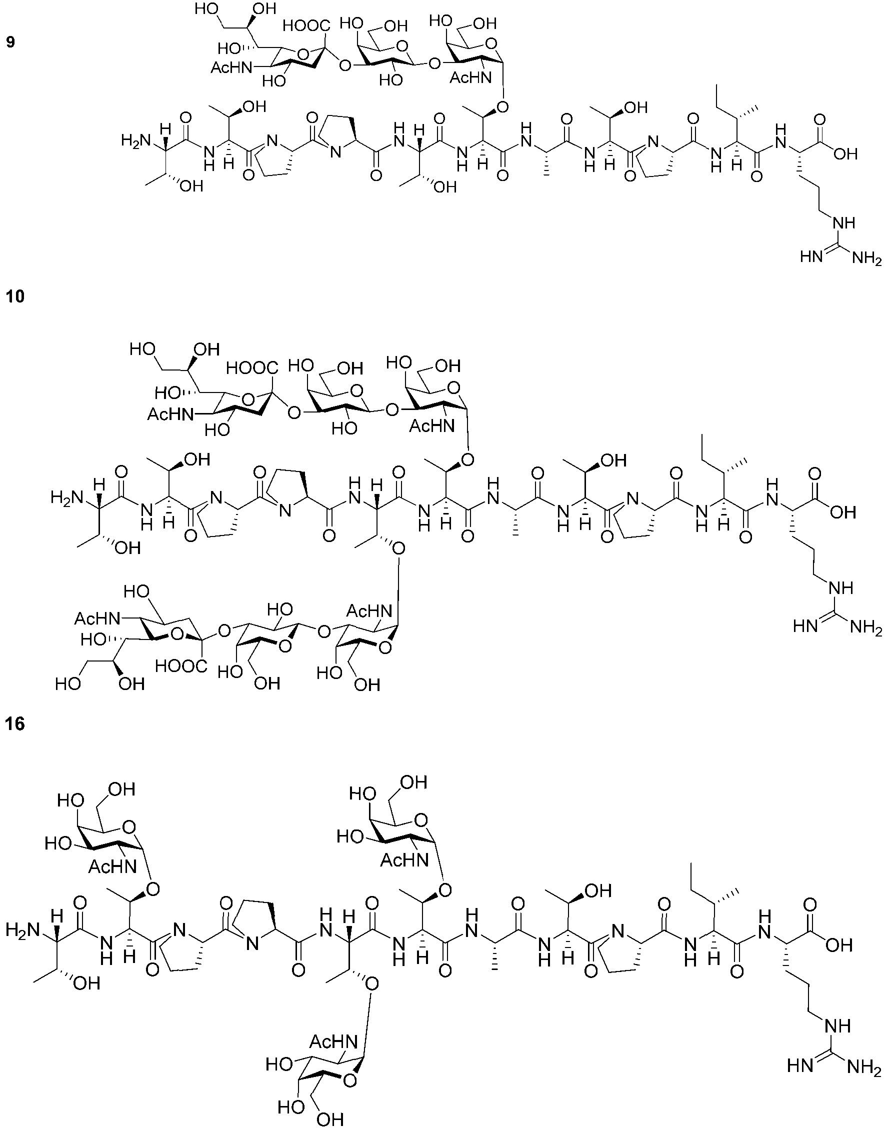

16. The method for analyzing a glycopeptide according to any one of claims 12 to 15, wherein the analyzing comprises performing the mass spectrometry of the glycopeptide.

17. The method for analyzing a glycopeptide according to claim 16, wherein the analyzing comprises performing the mass spectrometry of the glycopeptide according to tandem mass spectrometry.

18. The method for analyzing a glycopeptide according to any one of claims 12 to 15, which is used in detection of digestive system cancer and/or detection of the stage of digestive system cancer progression.

19. The method for analyzing a glycopeptide according to claim 18, wherein the digestive system cancer is liver cancer or pancreatic cancer.

20. The method for analyzing a glycopeptide according to any one of claims 12 to 15, wherein the sample is obtained by removing a portion of a serum protein or a plasma protein.

21. A method for analyzing cancer, including the method for analyzing a glycopeptide according to any one of claims 12 to 15.
